# FASCICULE DE BREVET EUROPEEN

(11) **EP 2 951 297 B1**
(45) Date de publication et mention de la délivrance du brevet: **03.05.2017**
(21) Numéro de dépôt: 14708609.4
(22) Date de dépôt: 03.02.2014
(51) Int. Cl.: C12N 9/64, A61K 38/48, C12N 15/12

(54) **MUTANTS DU FACTEUR X**
FAKTOR X MUTANTE
FACTOR X MUTANTS

(30) Priorité: 04.02.2013 FR 1350930
(43) Date de publication de la demande: 09.12.2015
(73) Titulaire: Laboratoire Français du Fractionnement et des Biotechnologies, 91940 Les Ulis (FR)
(72) Inventeur: PLANTIER, Jean-Luc, 59170 Croix (FR)
(74) Mandataire: Cabinet Plasseraud
(86) Numéro de dépôt international: PCT/FR2014/050191
(87) Numéro de publication internationale: WO 2014/118481

(56) Documents cités:
- WO-A1-2006/018204
- WO-A2-01/10896
- WO-A2-2006/128668

### SEQUENCE LISTING

<110> LFB SA
<120> Mutants du facteur X
<130> BCT140034 QT
<160> 158
<170> PatentIn version 3.5
<210> 1
   <211> 306
   <212> PRT
   <213> Homo sapiens
<400> 1
   Ser Val Ala Gln Ala Thr Ser Ser Ser Gly Glu Ala Pro Asp Ser Ile
   1               5                   10                  15
   Thr Trp Lys Pro Tyr Asp Ala Ala Asp Leu Asp Pro Thr Glu Asn Pro
               20                  25                  30
   Phe Asp Leu Leu Asp Phe Asn Gln Thr Gln Pro Glu Arg Gly Asp Asn
           35                  40                  45
   Asn Leu Thr Arg Ile Val Gly Gly Gln Glu Cys Lys Asp Gly Glu Cys
       50                  55                  60
   Pro Trp Gln Ala Leu Leu Ile Asn Glu Glu Asn Glu Gly Phe Cys Gly
   65                  70                  75                  80
   Gly Thr Ile Leu Ser Glu Phe Tyr Ile Leu Thr Ala Ala His Cys Leu
                   85                  90                  95
   Tyr Gln Ala Lys Arg Phe Lys Val Arg Val Gly Asp Arg Asn Thr Glu
               100                 105                 110
   Gln Glu Glu Gly Gly Glu Ala Val His Glu Val Glu Val Val Ile Lys
           115                 120                 125
   His Asn Arg Phe Thr Lys Glu Thr Tyr Asp Phe Asp Ile Ala Val Leu
       130                 135                 140
   Arg Leu Lys Thr Pro Ile Thr Phe Arg Met Asn Val Ala Pro Ala Cys
   145                 150                 155                 160
   Leu Pro Glu Arg Asp Trp Ala Glu Ser Thr Leu Met Thr Gln Lys Thr
                   165                 170                 175
   Gly Ile Val Ser Gly Phe Gly Arg Thr His Glu Lys Gly Arg Gln Ser
   180 185 190
   Thr Arg Leu Lys Met Leu Glu Val Pro Tyr Val Asp Arg Asn Ser Cys
   195 200 205
   Lys Leu Ser Ser Ser Phe Ile Ile Thr Gln Asn Met Phe Cys Ala Gly
   210 215 220
   Tyr Asp Thr Lys Gln Glu Asp Ala Cys Gln Gly Asp Ser Gly Gly Pro
   225 230 235 240
   His Val Thr Arg Phe Lys Asp Thr Tyr Phe Val Thr Gly Ile Val Ser
   245 250 255
   Trp Gly Glu Gly Cys Ala Arg Lys Gly Lys Tyr Gly Ile Tyr Thr Lys
   260 265 270
   Val Thr Ala Phe Leu Lys Trp Ile Asp Arg Ser Met Lys Thr Arg Gly
   275 280 285
   Leu Pro Lys Ala Lys Ser His Ala Pro Glu Val Ile Thr Ser Ser Pro
   290 295 300
   Leu Lys
   305
<210> 2
   <211> 182
   <212> PRT
   <213> Homo sapiens
<400> 2
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Thr Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg
               180
<210> 3
   <211> 52
   <212> PRT
   <213> Homo sapiens
<400> 3
   Ser Val Ala Gln Ala Thr Ser Ser Ser Gly Glu Ala Pro Asp Ser Ile
   1               5                   10                  15
   Thr Trp Lys Pro Tyr Asp Ala Ala Asp Leu Asp Pro Thr Glu Asn Pro
               20                  25                  30
   Phe Asp Leu Leu Asp Phe Asn Gln Thr Gln Pro Glu Arg Gly Asp Asn
           35                  40                  45
   Asn Leu Thr Arg
       50
<210> 4
   <211> 488
   <212> PRT
   <213> Homo sapiens
<400> 4
<210> 5
   <211> 142
   <212> PRT
   <213> Homo sapiens
<400> 5
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg
       130                 135                 140
<210> 6
   <211> 254
   <212> PRT
   <213> Homo sapiens
<400> 6
<210> 7
   <211> 500
   <212> PRT
   <213> Homo sapiens
<400> 7
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 8
   <211> 500
   <212> PRT
   <213> Artificial Sequence
<220>
   <223> FX-control+
<400> 8
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Asp Phe Leu Ala Glu Gly Gly Gly Val Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 9
   <211> 500
   <212> PRT
   <213> Artificial Sequence
<220>
   <223> FX-IIa selon mutation A
<400> 9
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Asp Phe Leu Ala Glu Gly Leu Thr Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 10
   <211> 500
   <212> PRT
   <213> Artificial Sequence
<220>
   <223> FX-PAR1 selon mutation A
<400> 10
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Lys Ala Thr Asn Ala Thr Leu Ser Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 11
   <211> 500
   <212> PRT
   <213> Artificial Sequence
<220>
   <223> FX-PAR1M selon mutation A
<400> 11
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Lys Ala Thr Gln Ala Thr Leu Ser Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 12
   <211> 500
   <212> PRT
   <213> Artificial Sequence
<220>
   <223> FX-FXIa1 selon mutation A'
<400> 12
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Thr Ser Lys Leu Thr Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 13
   <211> 500
   <212> PRT
   <213> Artificial Sequence
<220>
   <223> FX-FXIa2 selon mutation A'
<400> 13
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Ser Ser Gly
   180 185 190
   Glu Ala Pro Asp Ser Ile Thr Trp Lys Pro Tyr Asp Ala Ala Asp Leu
   195 200 205
   Asp Pro Thr Glu Asn Pro Phe Asp Leu Leu Asp Phe Asn Gln Thr Gln
   210 215 220
   Pro Glu Arg Gly Phe Asn Asp Phe Thr Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
             275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 14
   <211> 500
   <212> PRT
   <213> Artificial Sequence
<220>
   <223> FX-Kal1 selon mutation A'
<400> 14
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Leu Ser Ser Met Thr Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 15
   <211> 500
   <212> PRT
   <213> Artificial Sequence
<220>
   <223> FX-Kal2 selon mutation A'
<400> 15
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Pro Pro Ser Leu Thr Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 16
   <211> 500
   <212> PRT
   <213> Artificial Sequence
<220>
   <223> FX-Kal3 selon mutation A'
<400> 16
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Ser Ser Gly
   180 185 190
   Glu Ala Pro Asp Ser Ile Thr Trp Lys Pro Tyr Asp Ala Ala Asp Leu
   195 200 205
   Asp Pro Thr Glu Asn Pro Phe Asp Leu Leu Asp Phe Asn Gln Thr Gln
   210 215 220
   Pro Glu Arg Gly Leu Ser Cys Gly Gln Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 17
   <211> 510
   <212> PRT
   <213> Artificial Sequence
<220>
   <223> FX-control+
<400> 17
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Asp Phe Leu Ala Glu Gly
   225                 230                 235                 240
   Gly Gly Val Arg Ile Val Gly Gly Gln Glu Cys Lys Asp Gly Glu Cys
                   245                 250                 255
   Pro Trp Gln Ala Leu Leu Ile Asn Glu Glu Asn Glu Gly Phe Cys Gly
               260                 265                 270
   Gly Thr Ile Leu Ser Glu Phe Tyr Ile Leu Thr Ala Ala His Cys Leu
           275                 280                 285
   Tyr Gln Ala Lys Arg Phe Lys Val Arg Val Gly Asp Arg Asn Thr Glu
       290                 295                 300
   Gln Glu Glu Gly Gly Glu Ala Val His Glu Val Glu Val Val Ile Lys
   305                 310                 315                 320
   His Asn Arg Phe Thr Lys Glu Thr Tyr Asp Phe Asp Ile Ala Val Leu
   325 330 335
   Arg Leu Lys Thr Pro Ile Thr Phe Arg Met Asn Val Ala Pro Ala Cys
   340 345 350
   Leu Pro Glu Arg Asp Trp Ala Glu Ser Thr Leu Met Thr Gln Lys Thr
   355 360 365
   Gly Ile Val Ser Gly Phe Gly Arg Thr His Glu Lys Gly Arg Gln Ser
   370 375 380
   Thr Arg Leu Lys Met Leu Glu Val Pro Tyr Val Asp Arg Asn Ser Cys
   385 390 395 400
   Lys Leu Ser Ser Ser Phe Ile Ile Thr Gln Asn Met Phe Cys Ala Gly
   405 410 415
   Tyr Asp Thr Lys Gln Glu Asp Ala Cys Gln Gly Asp Ser Gly Gly Pro
   420 425 430
   His Val Thr Arg Phe Lys Asp Thr Tyr Phe Val Thr Gly Ile Val Ser
   435 440 445
   Trp Gly Glu Gly Cys Ala Arg Lys Gly Lys Tyr Gly Ile Tyr Thr Lys
   450 455 460
   Val Thr Ala Phe Leu Lys Trp Ile Asp Arg Ser Met Lys Thr Arg Gly
   465 470 475 480
   Leu Pro Lys Ala Lys Ser His Ala Pro Glu Val Ile Thr Ser Ser Pro
   485 490 495
   Leu Lys Glu Asp Gln Val Asp Pro Arg Leu Ile Asp Gly Lys
   500 505 510
<210> 18
   <211> 510
   <212> PRT
   <213> Artificial Sequence
<220>
   <223> FX-IIa selon mutation B
<400> 18
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Asp Phe Leu Ala Glu Gly
   225                 230                 235                 240
   Leu Thr Pro Arg Ile Val Gly Gly Gln Glu Cys Lys Asp Gly Glu Cys
                   245                 250                 255
   Pro Trp Gln Ala Leu Leu Ile Asn Glu Glu Asn Glu Gly Phe Cys Gly
               260                 265                 270
   Gly Thr Ile Leu Ser Glu Phe Tyr Ile Leu Thr Ala Ala His Cys Leu
               275                 280                 285
   Tyr Gln Ala Lys Arg Phe Lys Val Arg Val Gly Asp Arg Asn Thr Glu
       290                 295                 300
   Gln Glu Glu Gly Gly Glu Ala Val His Glu Val Glu Val Val Ile Lys
   305                 310                 315                 320
   His Asn Arg Phe Thr Lys Glu Thr Tyr Asp Phe Asp Ile Ala Val Leu
                   325                 330                 335
   Arg Leu Lys Thr Pro Ile Thr Phe Arg Met Asn Val Ala Pro Ala Cys
               340                 345                 350
   Leu Pro Glu Arg Asp Trp Ala Glu Ser Thr Leu Met Thr Gln Lys Thr
           355                 360                 365
   Gly Ile Val Ser Gly Phe Gly Arg Thr His Glu Lys Gly Arg Gln Ser
       370                 375                 380
   Thr Arg Leu Lys Met Leu Glu Val Pro Tyr Val Asp Arg Asn Ser Cys
   385                 390                 395                 400
   Lys Leu Ser Ser Ser Phe Ile Ile Thr Gln Asn Met Phe Cys Ala Gly
                   405                 410                 415
   Tyr Asp Thr Lys Gln Glu Asp Ala Cys Gln Gly Asp Ser Gly Gly Pro
               420                 425                 430
   His Val Thr Arg Phe Lys Asp Thr Tyr Phe Val Thr Gly Ile Val Ser
           435                 440                 445
   Trp Gly Glu Gly Cys Ala Arg Lys Gly Lys Tyr Gly Ile Tyr Thr Lys
       450                 455                 460
   Val Thr Ala Phe Leu Lys Trp Ile Asp Arg Ser Met Lys Thr Arg Gly
   465                 470                 475                 480
   Leu Pro Lys Ala Lys Ser His Ala Pro Glu Val Ile Thr Ser Ser Pro
                   485                 490                 495
   Leu Lys Glu Asp Gln Val Asp Pro Arg Leu Ile Asp Gly Lys
               500                 505                 510
<210> 19
   <211> 510
   <212> PRT
   <213> Artificial Sequence
<220>
   <223> FX-PAR1 selon mutation B
<400> 19
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Lys Ala Thr Asn Ala Thr
   225                 230                 235                 240
   Leu Ser Pro Arg Ile Val Gly Gly Gln Glu Cys Lys Asp Gly Glu Cys
                   245                 250                 255
   Pro Trp Gln Ala Leu Leu Ile Asn Glu Glu Asn Glu Gly Phe Cys Gly
               260                 265                 270
   Gly Thr Ile Leu Ser Glu Phe Tyr Ile Leu Thr Ala Ala His Cys Leu
           275                 280                 285
   Tyr Gln Ala Lys Arg Phe Lys Val Arg Val Gly Asp Arg Asn Thr Glu
       290                 295                 300
   Gln Glu Glu Gly Gly Glu Ala Val His Glu Val Glu Val Val Ile Lys
   305                 310                 315                 320
   His Asn Arg Phe Thr Lys Glu Thr Tyr Asp Phe Asp Ile Ala Val Leu
                   325                 330                 335
   Arg Leu Lys Thr Pro Ile Thr Phe Arg Met Asn Val Ala Pro Ala Cys
               340                 345                 350
   Leu Pro Glu Arg Asp Trp Ala Glu Ser Thr Leu Met Thr Gln Lys Thr
           355                 360                 365
   Gly Ile Val Ser Gly Phe Gly Arg Thr His Glu Lys Gly Arg Gln Ser
       370                 375                 380
   Thr Arg Leu Lys Met Leu Glu Val Pro Tyr Val Asp Arg Asn Ser Cys
   385                 390                 395                 400
   Lys Leu Ser Ser Ser Phe Ile Ile Thr Gln Asn Met Phe Cys Ala Gly
                   405                 410                 415
   Tyr Asp Thr Lys Gln Glu Asp Ala Cys Gln Gly Asp Ser Gly Gly Pro
               420                 425                 430
   His Val Thr Arg Phe Lys Asp Thr Tyr Phe Val Thr Gly Ile Val Ser
           435                 440                 445
   Trp Gly Glu Gly Cys Ala Arg Lys Gly Lys Tyr Gly Ile Tyr Thr Lys
       450                 455                 460
   Val Thr Ala Phe Leu Lys Trp Ile Asp Arg Ser Met Lys Thr Arg Gly
   465                 470                 475                 480
   Leu Pro Lys Ala Lys Ser His Ala Pro Glu Val Ile Thr Ser Ser Pro
   485 490 495
   Leu Lys Glu Asp Gln Val Asp Pro Arg Leu Ile Asp Gly Lys
   500 505 510
<210> 20
   <211> 510
   <212> PRT
   <213> Artificial Sequence
<220>
   <223> FX-PAR1M selon mutation B
<400> 20
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
   180 185 190
   Glu Ala Pro Asp Ser Ile Thr Trp Lys Pro Tyr Asp Ala Ala Asp Leu
   195 200 205
   Asp Pro Thr Glu Asn Pro Phe Asp Leu Leu Asp Phe Asn Gln Thr Gln
   210 215 220
   Pro Glu Arg Gly Asp Asn Asn Leu Thr Arg Lys Ala Thr Gln Ala Thr
   225 230 235 240
   Leu Ser Pro Arg Ile Val Gly Gly Gln Glu Cys Lys Asp Gly Glu Cys
   245 250 255
   Pro Trp Gln Ala Leu Leu Ile Asn Glu Glu Asn Glu Gly Phe Cys Gly
   260 265 270
   Gly Thr Ile Leu Ser Glu Phe Tyr Ile Leu Thr Ala Ala His Cys Leu
   275 280 285
   Tyr Gln Ala Lys Arg Phe Lys Val Arg Val Gly Asp Arg Asn Thr Glu
   290 295 300
   Gln Glu Glu Gly Gly Glu Ala Val His Glu Val Glu Val Val Ile Lys
   305 310 315 320
   His Asn Arg Phe Thr Lys Glu Thr Tyr Asp Phe Asp Ile Ala Val Leu
   325 330 335
   Arg Leu Lys Thr Pro Ile Thr Phe Arg Met Asn Val Ala Pro Ala Cys
   340 345 350
   Leu Pro Glu Arg Asp Trp Ala Glu Ser Thr Leu Met Thr Gln Lys Thr
   355 360 365
   Gly Ile Val Ser Gly Phe Gly Arg Thr His Glu Lys Gly Arg Gln Ser
   370 375 380
   Thr Arg Leu Lys Met Leu Glu Val Pro Tyr Val Asp Arg Asn Ser Cys
   385 390 395 400
   Lys Leu Ser Ser Ser Phe Ile Ile Thr Gln Asn Met Phe Cys Ala Gly
   405 410 415
   Tyr Asp Thr Lys Gln Glu Asp Ala Cys Gln Gly Asp Ser Gly Gly Pro
   420 425 430
   His Val Thr Arg Phe Lys Asp Thr Tyr Phe Val Thr Gly Ile Val Ser
   435 440 445
   Trp Gly Glu Gly Cys Ala Arg Lys Gly Lys Tyr Gly Ile Tyr Thr Lys
   450 455 460
   Val Thr Ala Phe Leu Lys Trp Ile Asp Arg Ser Met Lys Thr Arg Gly
   465 470 475 480
   Leu Pro Lys Ala Lys Ser His Ala Pro Glu Val Ile Thr Ser Ser Pro
   485 490 495
   Leu Lys Glu Asp Gln Val Asp Pro Arg Leu Ile Asp Gly Lys
   500 505 510
<210> 21
   <211> 506
   <212> PRT
   <213> Artificial Sequence
<220>
   <223> FX-FXIa1 selon mutation B
<400> 21
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Thr Ser Lys Leu Thr Arg
   225                 230                 235                 240
   Ile Val Gly Gly Gln Glu Cys Lys Asp Gly Glu Cys Pro Trp Gln Ala
                   245                 250                 255
   Leu Leu Ile Asn Glu Glu Asn Glu Gly Phe Cys Gly Gly Thr Ile Leu
               260                 265                 270
   Ser Glu Phe Tyr Ile Leu Thr Ala Ala His Cys Leu Tyr Gln Ala Lys
           275                 280                 285
   Arg Phe Lys Val Arg Val Gly Asp Arg Asn Thr Glu Gln Glu Glu Gly
       290                 295                 300
   Gly Glu Ala Val His Glu Val Glu Val Val Ile Lys His Asn Arg Phe
   305                 310                 315                 320
   Thr Lys Glu Thr Tyr Asp Phe Asp Ile Ala Val Leu Arg Leu Lys Thr
                   325                 330                 335
   Pro Ile Thr Phe Arg Met Asn Val Ala Pro Ala Cys Leu Pro Glu Arg
               340                 345                 350
   Asp Trp Ala Glu Ser Thr Leu Met Thr Gln Lys Thr Gly Ile Val Ser
           355                 360                 365
   Gly Phe Gly Arg Thr His Glu Lys Gly Arg Gln Ser Thr Arg Leu Lys
       370                 375                 380
   Met Leu Glu Val Pro Tyr Val Asp Arg Asn Ser Cys Lys Leu Ser Ser
   385                 390                 395                 400
   Ser Phe Ile Ile Thr Gln Asn Met Phe Cys Ala Gly Tyr Asp Thr Lys
                   405                 410                 415
   Gln Glu Asp Ala Cys Gln Gly Asp Ser Gly Gly Pro His Val Thr Arg
               420                 425                 430
   Phe Lys Asp Thr Tyr Phe Val Thr Gly Ile Val Ser Trp Gly Glu Gly
           435                 440                 445
   Cys Ala Arg Lys Gly Lys Tyr Gly Ile Tyr Thr Lys Val Thr Ala Phe
       450                 455                 460
   Leu Lys Trp Ile Asp Arg Ser Met Lys Thr Arg Gly Leu Pro Lys Ala
   465                 470                 475                 480
   Lys Ser His Ala Pro Glu Val Ile Thr Ser Ser Pro Leu Lys Glu Asp
                   485                 490                 495
   Gln Val Asp Pro Arg Leu Ile Asp Gly Lys
               500                 505
<210> 22
   <211> 506
   <212> PRT
   <213> Artificial Sequence
<220>
   <223> FX-FXIa2 selon mutation B
<400> 22
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Ser Ser Gly
   180 185 190
   Glu Ala Pro Asp Ser Ile Thr Trp Lys Pro Tyr Asp Ala Ala Asp Leu
   195 200 205
   Asp Pro Thr Glu Asn Pro Phe Asp Leu Leu Asp Phe Asn Gln Thr Gln
   210 215 220
   Pro Glu Arg Gly Asp Asn Asn Leu Thr Arg Phe Asn Asp Phe Thr Arg
   225 230 235 240
   Ile Val Gly Gly Gln Glu Cys Lys Asp Gly Glu Cys Pro Trp Gln Ala
   245 250 255
   Leu Leu Ile Asn Glu Glu Asn Glu Gly Phe Cys Gly Gly Thr Ile Leu
   260 265 270
   Ser Glu Phe Tyr Ile Leu Thr Ala Ala His Cys Leu Tyr Gln Ala Lys
   275 280 285
   Arg Phe Lys Val Arg Val Gly Asp Arg Asn Thr Glu Gln Glu Glu Gly
   290 295 300
   Gly Glu Ala Val His Glu Val Glu Val Val Ile Lys His Asn Arg Phe
   305 310 315 320
   Thr Lys Glu Thr Tyr Asp Phe Asp Ile Ala Val Leu Arg Leu Lys Thr
   325 330 335
   Pro Ile Thr Phe Arg Met Asn Val Ala Pro Ala Cys Leu Pro Glu Arg
               340                 345                 350
   Asp Trp Ala Glu Ser Thr Leu Met Thr Gln Lys Thr Gly Ile Val Ser
           355                 360                 365
   Gly Phe Gly Arg Thr His Glu Lys Gly Arg Gln Ser Thr Arg Leu Lys
       370                 375                 380
   Met Leu Glu Val Pro Tyr Val Asp Arg Asn Ser Cys Lys Leu Ser Ser
   385                 390                 395                 400
   Ser Phe Ile Ile Thr Gln Asn Met Phe Cys Ala Gly Tyr Asp Thr Lys
                   405                 410                 415
   Gln Glu Asp Ala Cys Gln Gly Asp Ser Gly Gly Pro His Val Thr Arg
               420                 425                 430
   Phe Lys Asp Thr Tyr Phe Val Thr Gly Ile Val Ser Trp Gly Glu Gly
           435                 440                 445
   Cys Ala Arg Lys Gly Lys Tyr Gly Ile Tyr Thr Lys Val Thr Ala Phe
       450                 455                 460
   Leu Lys Trp Ile Asp Arg Ser Met Lys Thr Arg Gly Leu Pro Lys Ala
   465                 470                 475                 480
   Lys Ser His Ala Pro Glu Val Ile Thr Ser Ser Pro Leu Lys Glu Asp
                   485                 490                 495
   Gln Val Asp Pro Arg Leu Ile Asp Gly Lys
               500                 505
<210> 23
   <211> 506
   <212> PRT
   <213> Artificial Sequence
<220>
   <223> FX-Kal1 selon mutation B
<400> 23
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Leu Ser Ser Met Thr Arg
   225                 230                 235                 240
   Ile Val Gly Gly Gln Glu Cys Lys Asp Gly Glu Cys Pro Trp Gln Ala
                   245                 250                 255
   Leu Leu Ile Asn Glu Glu Asn Glu Gly Phe Cys Gly Gly Thr Ile Leu
               260                 265                 270
   Ser Glu Phe Tyr Ile Leu Thr Ala Ala His Cys Leu Tyr Gln Ala Lys
           275                 280                 285
   Arg Phe Lys Val Arg Val Gly Asp Arg Asn Thr Glu Gln Glu Glu Gly
       290                 295                 300
   Gly Glu Ala Val His Glu Val Glu Val Val Ile Lys His Asn Arg Phe
   305                 310                 315                 320
   Thr Lys Glu Thr Tyr Asp Phe Asp Ile Ala Val Leu Arg Leu Lys Thr
                   325                 330                 335
   Pro Ile Thr Phe Arg Met Asn Val Ala Pro Ala Cys Leu Pro Glu Arg
               340                 345                 350
   Asp Trp Ala Glu Ser Thr Leu Met Thr Gln Lys Thr Gly Ile Val Ser
           355                 360                 365
   Gly Phe Gly Arg Thr His Glu Lys Gly Arg Gln Ser Thr Arg Leu Lys
       370                 375                 380
   Met Leu Glu Val Pro Tyr Val Asp Arg Asn Ser Cys Lys Leu Ser Ser
   385                 390                 395                 400
   Ser Phe Ile Ile Thr Gln Asn Met Phe Cys Ala Gly Tyr Asp Thr Lys
                   405                 410                 415
   Gln Glu Asp Ala Cys Gln Gly Asp Ser Gly Gly Pro His Val Thr Arg
               420                 425                 430
   Phe Lys Asp Thr Tyr Phe Val Thr Gly Ile Val Ser Trp Gly Glu Gly
           435                 440                 445
   Cys Ala Arg Lys Gly Lys Tyr Gly Ile Tyr Thr Lys Val Thr Ala Phe
       450                 455                 460
   Leu Lys Trp Ile Asp Arg Ser Met Lys Thr Arg Gly Leu Pro Lys Ala
   465                 470                 475                 480
   Lys Ser His Ala Pro Glu Val Ile Thr Ser Ser Pro Leu Lys Glu Asp
                   485                 490                 495
   Gln Val Asp Pro Arg Leu Ile Asp Gly Lys
               500                 505
<210> 24
   <211> 506
   <212> PRT
   <213> Artificial Sequence
<220>
   <223> FX-Kal2 selon mutation B
<400> 24
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Pro Pro Ser Leu Thr Arg
   225                 230                 235                 240
   Ile Val Gly Gly Gln Glu Cys Lys Asp Gly Glu Cys Pro Trp Gln Ala
                   245                 250                 255
   Leu Leu Ile Asn Glu Glu Asn Glu Gly Phe Cys Gly Gly Thr Ile Leu
               260                 265                 270
   Ser Glu Phe Tyr Ile Leu Thr Ala Ala His Cys Leu Tyr Gln Ala Lys
           275                 280                 285
   Arg Phe Lys Val Arg Val Gly Asp Arg Asn Thr Glu Gln Glu Glu Gly
       290                 295                 300
   Gly Glu Ala Val His Glu Val Glu Val Val Ile Lys His Asn Arg Phe
   305                 310                 315                 320
   Thr Lys Glu Thr Tyr Asp Phe Asp Ile Ala Val Leu Arg Leu Lys Thr
                   325                 330                 335
   Pro Ile Thr Phe Arg Met Asn Val Ala Pro Ala Cys Leu Pro Glu Arg
               340                 345                 350
   Asp Trp Ala Glu Ser Thr Leu Met Thr Gln Lys Thr Gly Ile Val Ser
           355                 360                 365
   Gly Phe Gly Arg Thr His Glu Lys Gly Arg Gln Ser Thr Arg Leu Lys
       370                 375                 380
   Met Leu Glu Val Pro Tyr Val Asp Arg Asn Ser Cys Lys Leu Ser Ser
   385                 390                 395                 400
   Ser Phe Ile Ile Thr Gln Asn Met Phe Cys Ala Gly Tyr Asp Thr Lys
                   405                 410                 415
   Gln Glu Asp Ala Cys Gln Gly Asp Ser Gly Gly Pro His Val Thr Arg
               420                 425                 430
   Phe Lys Asp Thr Tyr Phe Val Thr Gly Ile Val Ser Trp Gly Glu Gly
           435                 440                 445
   Cys Ala Arg Lys Gly Lys Tyr Gly Ile Tyr Thr Lys Val Thr Ala Phe
       450                 455                 460
   Leu Lys Trp Ile Asp Arg Ser Met Lys Thr Arg Gly Leu Pro Lys Ala
   465                 470                 475                 480
   Lys Ser His Ala Pro Glu Val Ile Thr Ser Ser Pro Leu Lys Glu Asp
                   485                 490                 495
   Gln Val Asp Pro Arg Leu Ile Asp Gly Lys
   500 505
<210> 25
   <211> 506
   <212> PRT
   <213> Artificial Sequence
<220>
   <223> FX-Kal3 selon mutation B
<400> 25
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Ser Ser Gly
               180                 185                 190
   Glu Ala Pro Asp Ser Ile Thr Trp Lys Pro Tyr Asp Ala Ala Asp Leu
           195                 200                 205
   Asp Pro Thr Glu Asn Pro Phe Asp Leu Leu Asp Phe Asn Gln Thr Gln
       210                 215                 220
   Pro Glu Arg Gly Asp Asn Asn Leu Thr Arg Leu Ser Cys Gly Gln Arg
   225                 230                 235                 240
   Ile Val Gly Gly Gln Glu Cys Lys Asp Gly Glu Cys Pro Trp Gln Ala
                   245                 250                 255
   Leu Leu Ile Asn Glu Glu Asn Glu Gly Phe Cys Gly Gly Thr Ile Leu
               260                 265                 270
   Ser Glu Phe Tyr Ile Leu Thr Ala Ala His Cys Leu Tyr Gln Ala Lys
           275                 280                 285
   Arg Phe Lys Val Arg Val Gly Asp Arg Asn Thr Glu Gln Glu Glu Gly
       290                 295                 300
   Gly Glu Ala Val His Glu Val Glu Val Val Ile Lys His Asn Arg Phe
   305                 310                 315                 320
   Thr Lys Glu Thr Tyr Asp Phe Asp Ile Ala Val Leu Arg Leu Lys Thr
                   325                 330                 335
   Pro Ile Thr Phe Arg Met Asn Val Ala Pro Ala Cys Leu Pro Glu Arg
               340                 345                 350
   Asp Trp Ala Glu Ser Thr Leu Met Thr Gln Lys Thr Gly Ile Val Ser
           355                 360                 365
   Gly Phe Gly Arg Thr His Glu Lys Gly Arg Gln Ser Thr Arg Leu Lys
       370                 375                 380
   Met Leu Glu Val Pro Tyr Val Asp Arg Asn Ser Cys Lys Leu Ser Ser
   385                 390                 395                 400
   Ser Phe Ile Ile Thr Gln Asn Met Phe Cys Ala Gly Tyr Asp Thr Lys
                   405                 410                 415
   Gln Glu Asp Ala Cys Gln Gly Asp Ser Gly Gly Pro His Val Thr Arg
               420                 425                 430
   Phe Lys Asp Thr Tyr Phe Val Thr Gly Ile Val Ser Trp Gly Glu Gly
           435                 440                 445
   Cys Ala Arg Lys Gly Lys Tyr Gly Ile Tyr Thr Lys Val Thr Ala Phe
   450 455 460
   Leu Lys Trp Ile Asp Arg Ser Met Lys Thr Arg Gly Leu Pro Lys Ala
   465 470 475 480
   Lys Ser His Ala Pro Glu Val Ile Thr Ser Ser Pro Leu Lys Glu Asp
   485 490 495
   Gln Val Asp Pro Arg Leu Ile Asp Gly Lys
   500 505
<210> 26
   <211> 500
   <212> PRT
   <213> Artificial Sequence
<220>
   <223> FX-control+
<400> 26
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Asp Ser Ile Thr Trp Lys Pro
   180 185 190
   Tyr Asp Ala Ala Asp Leu Asp Pro Thr Glu Asn Pro Phe Asp Leu Leu
   195 200 205
   Asp Phe Asn Gln Thr Gln Pro Glu Arg Gly Asp Asn Asn Leu Thr Arg
   210 215 220
   Asp Phe Leu Ala Glu Gly Gly Gly Val Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 27
   <211> 500
   <212> PRT
   <213> Artificial Sequence
<220>
   <223> FX-IIa selon mutation C
<400> 27
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Asp Ser Ile Thr Trp Lys Pro
               180                 185                 190
   Tyr Asp Ala Ala Asp Leu Asp Pro Thr Glu Asn Pro Phe Asp Leu Leu
           195                 200                 205
   Asp Phe Asn Gln Thr Gln Pro Glu Arg Gly Asp Asn Asn Leu Thr Arg
       210                 215                 220
   Asp Phe Leu Ala Glu Gly Leu Thr Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
   370 375 380
   Val Asp Arg Asn Ser Cys Lys Leu Ser Ser Ser Phe Ile Ile Thr Gln
   385 390 395 400
   Asn Met Phe Cys Ala Gly Tyr Asp Thr Lys Gln Glu Asp Ala Cys Gln
   405 410 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 28
   <211> 500
   <212> PRT
   <213> Artificial Sequence
<220>
   <223> FX-PAR1 selon mutation C
<400> 28
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Asp Ser Ile Thr Trp Lys Pro
               180                 185                 190
   Tyr Asp Ala Ala Asp Leu Asp Pro Thr Glu Asn Pro Phe Asp Leu Leu
           195                 200                 205
   Asp Phe Asn Gln Thr Gln Pro Glu Arg Gly Asp Asn Asn Leu Thr Arg
       210                 215                 220
   Lys Ala Thr Asn Ala Thr Leu Ser Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 29
   <211> 500
   <212> PRT
   <213> Artificial Sequence
<220>
   <223> FX-PAR1M selon mutation C
<400> 29
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Asp Ser Ile Thr Trp Lys Pro
               180                 185                 190
   Tyr Asp Ala Ala Asp Leu Asp Pro Thr Glu Asn Pro Phe Asp Leu Leu
           195                 200                 205
   Asp Phe Asn Gln Thr Gln Pro Glu Arg Gly Asp Asn Asn Leu Thr Arg
       210                 215                 220
   Lys Ala Thr Gln Ala Thr Leu Ser Pro Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 30
   <211> 500
   <212> PRT
   <213> Artificial Sequence
<220>
   <223> FX-FXIa1 selon mutation C'
<400> 30
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Asp Ser Ile
               180                 185                 190
   Thr Trp Lys Pro Tyr Asp Ala Ala Asp Leu Asp Pro Thr Glu Asn Pro
           195                 200                 205
   Phe Asp Leu Leu Asp Phe Asn Gln Thr Gln Pro Glu Arg Gly Asp Asn
       210                 215                 220
   Asn Leu Thr Arg Thr Ser Lys Leu Thr Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 31
   <211> 500
   <212> PRT
   <213> Artificial Sequence
<220>
   <223> FX-FXIa2 selon mutation C'
<400> 31
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Asp Ser Ile
               180                 185                 190
   Thr Trp Lys Pro Tyr Asp Ala Ala Asp Leu Asp Pro Thr Glu Asn Pro
           195                 200                 205
   Phe Asp Leu Leu Asp Phe Asn Gln Thr Gln Pro Glu Arg Gly Asp Asn
       210                 215                 220
   Asn Leu Thr Arg Phe Asn Asp Phe Thr Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
           275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
   420 425 430
   Val Thr Gly Ile Val Ser Trp Gly Glu Gly Cys Ala Arg Lys Gly Lys
   435 440 445
   Tyr Gly Ile Tyr Thr Lys Val Thr Ala Phe Leu Lys Trp Ile Asp Arg
   450 455 460
   Ser Met Lys Thr Arg Gly Leu Pro Lys Ala Lys Ser His Ala Pro Glu
   465 470 475 480
   Val Ile Thr Ser Ser Pro Leu Lys Glu Asp Gln Val Asp Pro Arg Leu
   485 490 495
   Ile Asp Gly Lys
   500
<210> 32
   <211> 500
   <212> PRT
   <213> Artificial Sequence
<220>
   <223> FX-Kal1 selon mutation C'
<400> 32
   Met Gly Arg Pro Leu His Leu Val Leu Leu Ser Ala Ser Leu Ala Gly
   1 5 10 15
   Leu Leu Leu Leu Gly Glu Ser Leu Phe Ile Arg Arg Glu Gln Ala Asn
   20 25 30
   Asn Ile Leu Ala Arg Val Arg Arg Ala Asn Ser Phe Leu Glu Glu Met
   35 40 45
   Lys Lys Gly His Leu Glu Arg Glu Cys Met Glu Glu Thr Cys Ser Tyr
   50 55 60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Asp Ser Ile
   180 185 190
   Thr Trp Lys Pro Tyr Asp Ala Ala Asp Leu Asp Pro Thr Glu Asn Pro
   195 200 205
   Phe Asp Leu Leu Asp Phe Asn Gln Thr Gln Pro Glu Arg Gly Asp Asn
   210 215 220
   Asn Leu Thr Arg Leu Ser Ser Met Thr Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
   325 330 335
   Asn Val Ala Pro Ala Cys Leu Pro Glu Arg Asp Trp Ala Glu Ser Thr
   340 345 350
   Leu Met Thr Gln Lys Thr Gly Ile Val Ser Gly Phe Gly Arg Thr His
   355 360 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 33
   <211> 500
   <212> PRT
   <213> Artificial Sequence
<220>
   <223> FX-Kal2 selon mutation C'
<400> 33
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65 70 75 80
   Trp Asn Lys Tyr Lys Asp Gly Asp Gln Cys Glu Thr Ser Pro Cys Gln
   85 90 95
   Asn Gln Gly Lys Cys Lys Asp Gly Leu Gly Glu Tyr Thr Cys Thr Cys
   100 105 110
   Leu Glu Gly Phe Glu Gly Lys Asn Cys Glu Leu Phe Thr Arg Lys Leu
   115 120 125
   Cys Ser Leu Asp Asn Gly Asp Cys Asp Gln Phe Cys His Glu Glu Gln
   130 135 140
   Asn Ser Val Val Cys Ser Cys Ala Arg Gly Tyr Thr Leu Ala Asp Asn
   145 150 155 160
   Gly Lys Ala Cys Ile Pro Thr Gly Pro Tyr Pro Cys Gly Lys Gln Thr
   165 170 175
   Leu Glu Arg Arg Lys Arg Ser Val Ala Gln Ala Thr Ser Asp Ser Ile
   180 185 190
   Thr Trp Lys Pro Tyr Asp Ala Ala Asp Leu Asp Pro Thr Glu Asn Pro
   195 200 205
   Phe Asp Leu Leu Asp Phe Asn Gln Thr Gln Pro Glu Arg Gly Asp Asn
   210 215 220
   Asn Leu Thr Arg Pro Pro Ser Leu Thr Arg Ile Val Gly Gly Gln Glu
   225 230 235 240
   Cys Lys Asp Gly Glu Cys Pro Trp Gln Ala Leu Leu Ile Asn Glu Glu
   245 250 255
   Asn Glu Gly Phe Cys Gly Gly Thr Ile Leu Ser Glu Phe Tyr Ile Leu
   260 265 270
   Thr Ala Ala His Cys Leu Tyr Gln Ala Lys Arg Phe Lys Val Arg Val
   275 280 285
   Gly Asp Arg Asn Thr Glu Gln Glu Glu Gly Gly Glu Ala Val His Glu
   290 295 300
   Val Glu Val Val Ile Lys His Asn Arg Phe Thr Lys Glu Thr Tyr Asp
   305 310 315 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 34
   <211> 500
   <212> PRT
   <213> Artificial Sequence
<220>
   <223> FX-Kal3 selon mutation C'
<400> 34
   Met Gly Arg Pro Leu His Leu Val Leu Leu Ser Ala Ser Leu Ala Gly
   1               5                   10                  15
   Leu Leu Leu Leu Gly Glu Ser Leu Phe Ile Arg Arg Glu Gln Ala Asn
               20                  25                  30
   Asn Ile Leu Ala Arg Val Arg Arg Ala Asn Ser Phe Leu Glu Glu Met
           35                  40                  45
   Lys Lys Gly His Leu Glu Arg Glu Cys Met Glu Glu Thr Cys Ser Tyr
       50                  55                  60
   Glu Glu Ala Arg Glu Val Phe Glu Asp Ser Asp Lys Thr Asn Glu Phe
   65                  70                  75                  80
   Trp Asn Lys Tyr Lys Asp Gly Asp Gln Cys Glu Thr Ser Pro Cys Gln
                   85                  90                  95
   Asn Gln Gly Lys Cys Lys Asp Gly Leu Gly Glu Tyr Thr Cys Thr Cys
               100                 105                 110
   Leu Glu Gly Phe Glu Gly Lys Asn Cys Glu Leu Phe Thr Arg Lys Leu
           115                 120                 125
   Cys Ser Leu Asp Asn Gly Asp Cys Asp Gln Phe Cys His Glu Glu Gln
       130                 135                 140
   Asn Ser Val Val Cys Ser Cys Ala Arg Gly Tyr Thr Leu Ala Asp Asn
   145                 150                 155                 160
   Gly Lys Ala Cys Ile Pro Thr Gly Pro Tyr Pro Cys Gly Lys Gln Thr
                   165                 170                 175
   Leu Glu Arg Arg Lys Arg Ser Val Ala Gln Ala Thr Ser Asp Ser Ile
               180                 185                 190
   Thr Trp Lys Pro Tyr Asp Ala Ala Asp Leu Asp Pro Thr Glu Asn Pro
           195                 200                 205
   Phe Asp Leu Leu Asp Phe Asn Gln Thr Gln Pro Glu Arg Gly Asp Asn
       210                 215                 220
   Asn Leu Thr Arg Leu Ser Cys Gly Gln Arg Ile Val Gly Gly Gln Glu
   225                 230                 235                 240
   Cys Lys Asp Gly Glu Cys Pro Trp Gln Ala Leu Leu Ile Asn Glu Glu
                   245                 250                 255
   Asn Glu Gly Phe Cys Gly Gly Thr Ile Leu Ser Glu Phe Tyr Ile Leu
               260                 265                 270
   Thr Ala Ala His Cys Leu Tyr Gln Ala Lys Arg Phe Lys Val Arg Val
             275                 280                 285
   Gly Asp Arg Asn Thr Glu Gln Glu Glu Gly Gly Glu Ala Val His Glu
       290                 295                 300
   Val Glu Val Val Ile Lys His Asn Arg Phe Thr Lys Glu Thr Tyr Asp
   305                 310                 315                 320
   Phe Asp Ile Ala Val Leu Arg Leu Lys Thr Pro Ile Thr Phe Arg Met
                   325                 330                 335
   Asn Val Ala Pro Ala Cys Leu Pro Glu Arg Asp Trp Ala Glu Ser Thr
               340                 345                 350
   Leu Met Thr Gln Lys Thr Gly Ile Val Ser Gly Phe Gly Arg Thr His
           355                 360                 365
   Glu Lys Gly Arg Gln Ser Thr Arg Leu Lys Met Leu Glu Val Pro Tyr
       370                 375                 380
   Val Asp Arg Asn Ser Cys Lys Leu Ser Ser Ser Phe Ile Ile Thr Gln
   385                 390                 395                 400
   Asn Met Phe Cys Ala Gly Tyr Asp Thr Lys Gln Glu Asp Ala Cys Gln
                   405                 410                 415
   Gly Asp Ser Gly Gly Pro His Val Thr Arg Phe Lys Asp Thr Tyr Phe
               420                 425                 430
   Val Thr Gly Ile Val Ser Trp Gly Glu Gly Cys Ala Arg Lys Gly Lys
           435                 440                 445
   Tyr Gly Ile Tyr Thr Lys Val Thr Ala Phe Leu Lys Trp Ile Asp Arg
       450                 455                 460
   Ser Met Lys Thr Arg Gly Leu Pro Lys Ala Lys Ser His Ala Pro Glu
   465                 470                 475                 480
   Val Ile Thr Ser Ser Pro Leu Lys Glu Asp Gln Val Asp Pro Arg Leu
                   485                 490                 495
   Ile Asp Gly Lys
               500
<210> 35
   <211> 1500
   <212> DNA
   <213> Artificial Sequence
<220>
   <223> séquence optimisée codant pour FX-WT
<400> 35
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 36
   <211> 21
   <212> DNA
   <213> Artificial Sequence
<220>
   <223> amorce CMVs1
<400> 36
   gggactttcc tacttggcag t 21
<210> 37
   <211> 22
   <212> DNA
   <213> Artificial Sequence
<220>
   <223> amorce SV40-3' UTR
<400> 37
   ttcactgcat tctagttgtg gt 22
<210> 38
   <211> 22
   <212> DNA
   <213> Artificial Sequence
<220>
   <223> amorce 5'efla
<400> 38
   gtggagactg aagttaggcc ag 22
<210> 39
   <211> 27
   <212> DNA
   <213> Artificial Sequence
<220>
   <223> amorce 3FX
<400> 39
   cttcatttcc tccaggaaag agttggc 27
<210> 40
   <211> 20
   <212> DNA
   <213> Artificial Sequence
<220>
   <223> 2BGHPA
<400> 40
   cagatggctg gcaactagaa 20
<210> 41
   <211> 46
   <212> DNA
   <213> Artificial Sequence
<220>
   <223> amorce 3'FX1a
<400> 41
   tccttctgcc aggaagtcct gtgtctggtt gaagtccagc aggtca 46
<210> 42
   <211> 50
   <212> DNA
   <213> Artificial Sequence
<220>
   <223> amorce 3'FX1b
<400> 42
   tcaggccctc ggccaggaag tcctgtgtct ggttgaagtc cagcaggtca 50
<210> 43
   <211> 45
   <212> DNA
   <213> Artificial Sequence
<220>
   <223> amorce 3'FX1c
<400> 43
   tggcgttggt ggccttctgt gtctggttga agtccagcag gtcaa 45
<210> 44
   <211> 48
   <212> DNA
   <213> Artificial Sequence
<220>
   <223> amorce 3'FX1d
<400> 44
   agggtggcct gggtggcctt ctgtgtctgg ttgaagtcca gcaggtca 48
<210> 45
   <211> 42
   <212> DNA
   <213> Artificial Sequence
<220>
   <223> amorce 3'FX1e
<400> 45
   tggtcagctt gctggtgcct ctttcaggct gtgtctggtt ga 42
<210> 46
   <211> 44
   <212> DNA
   <213> Artificial Sequence
<220>
   <223> amorce 3'FX1f
<400> 46
   tggtgaagtc gttgaagcct ctttcaggct gtgtctggtt gaag 44
<210> 47
   <211> 45
   <212> DNA
   <213> Artificial Sequence
<220>
   <223> amorce 3'FX1g
<400> 47
   tggtcatgct gctcaggcct ctttcaggct gtgtctggtt gaagt 45
<210> 48
   <211> 44
   <212> DNA
   <213> Artificial Sequence
<220>
   <223> amorce 3'FX1h
<400> 48
   tggtcaggct gggagggcct ctttcaggct gtgtctggtt gaag 44
<210> 49
   <211> 44
   <212> DNA
   <213> Artificial Sequence
<220>
   <223> amorce 3'FX1i
<400> 49
   tctggccgca ggacaggcct ctttcaggct gtgtctggtt gaag 44
<210> 50
   <211> 50
   <212> DNA
   <213> Artificial Sequence
<220>
   <223> amorce 3FX-2a
<400> 50
   ctccttctgc caggaagtcc ctagtcagat tgttatcgcc tctttcaggc 50
<210> 51
   <211> 54
   <212> DNA
   <213> Artificial Sequence
<220>
   <223> amorce 3FX-2b
<400> 51
   gtcaggccct cggccaggaa gtccctagtc agattgttat cgcctctttc aggc 54
<210> 52
   <211> 51
   <212> DNA
   <213> Artificial Sequence
<220>
   <223> amorce 3FX-2c
<400> 52
   agggtggcgt tggtggcctt cctagtcaga ttgttatcgc ctctttcagg c 51
<210> 53
   <211> 51
   <212> DNA
   <213> Artificial Sequence
<220>
   <223> amorce 3FX-2d
<400> 53
   agggtggcct gggtggcctt cctagtcaga ttgttatcgc ctctttcagg c 51
<210> 54
   <211> 44
   <212> DNA
   <213> Artificial Sequence
<220>
   <223> amorce 3FX-2e
<400> 54
   tcagcttgct ggtcctagtc agattgttat cgcctctttc aggc 44
<210> 55
   <211> 46
   <212> DNA
   <213> Artificial Sequence
<220>
   <223> amorce 3FX-2f
<400> 55
   ggtgaagtcg ttgaacctag tcagattgtt atcgcctctt tcaggc 46
<210> 56
   <211> 42
   <212> DNA
   <213> Artificial Sequence
<220>
   <223> amorce 3FX-2g
<400> 56
   atgctgctca gcctagtcag attgttatcg cctctttcag gc 42
<210> 57
   <211> 45
   <212> DNA
   <213> Artificial Sequence
<220>
   <223> amorce 3FX-2h
<400> 57
   gtcaggctgg gaggcctagt cagattgtta tcgcctcttt caggc 45
<210> 58
   <211> 47
   <212> DNA
   <213> Artificial Sequence
<220>
   <223> amorce 3FX-2i
<400> 58
   tctggccgca ggacagccta gtcagattgt tatcgcctct ttcaggc 47
<210> 59
   <211> 43
   <212> DNA
   <213> Artificial Sequence
<220>
   <223> amorce 5'FX1a
<400> 59
   gcagaaggag gaggagtgag gatcgtggga ggacaggagt gca 43
<210> 60
   <211> 43
   <212> DNA
   <213> Artificial Sequence
<220>
   <223> amorce 5'FX1b
<400> 60
   agggcctgac ccctaggatc gtgggaggac aggagtgcaa gga 43
<210> 61
   <211> 55
   <212> DNA
   <213> Artificial Sequence
<220>
   <223> amorce 5'FX1cbis
<400> 61
   aggccaccaa cgccaccctg tcccctagga tcgtgggagg acaggagtgc aagga 55
<210> 62
   <211> 45
   <212> DNA
   <213> Artificial Sequence
<220>
   <223> amorce 5'FX1d
<400> 62
   caggccaccc tgagccctag gatcgtggga ggacaggagt gcaag 45
<210> 63
   <211> 44
   <212> DNA
   <213> Artificial Sequence
<220>
   <223> amorce 5'FX1e
<400> 63
   accagcaagc tgaccaggat cgtgggagga caggagtgca agga 44
<210> 64
   <211> 42
   <212> DNA
   <213> Artificial Sequence
<220>
   <223> amorce 5'FX1f
<400> 64
   caacgacttc accaggatcg tgggaggaca ggagtgcaag ga 42
<210> 65
   <211> 43
   <212> DNA
   <213> Artificial Sequence
<220>
   <223> amorce 5'FX1g
<400> 65
   tgagcagcat gaccaggatc gtgggaggac aggagtgcaa gga 43
<210> 66
   <211> 44
   <212> DNA
   <213> Artificial Sequence
<220>
   <223> amorce 5'FX1h
<400> 66
   cctcccagcc tgaccaggat cgtgggagga caggagtgca agga 44
<210> 67
   <211> 43
   <212> DNA
   <213> Artificial Sequence
<220>
   <223> amorce 5'FX1i
<400> 67
   tgtcctgcgg ccagaggatc gtgggaggac aggagtgcaa gga 43
<210> 68
   <211> 32
   <212> DNA
   <213> Artificial Sequence
<220>
   <223> amorce 5'fusion FX
<400> 68
   tcttccattt cagctagcaa gcttgccgcc ac 32
<210> 69
   <211> 41
   <212> DNA
   <213> Artificial Sequence
<220>
   <223> amorce 3 fusion FX
<400> 69
   agctctagac aattgattta aatggatcct cacttgccgt c 41
<210> 70
   <211> 23
   <212> DNA
   <213> Artificial Sequence
<220>
   <223> amorce 5'FXWT
<400> 70
   accagctgct agcaagcttg ccg 23
<210> 71
   <211> 39
   <212> DNA
   <213> Artificial Sequence
<220>
   <223> amorce 3'FX-SwaI
<400> 71
   gaaactattt aaatggatcc tcacttgccg tcaatcagc 39
<210> 72
   <211> 39
   <212> DNA
   <213> Artificial Sequence
<220>
   <223> amorce 3 FXF3
<400> 72
   ccaggtaatg ctatcagcca ctgacctttt gcgcctctc 39
<210> 73
   <211> 38
   <212> DNA
   <213> Artificial Sequence
<220>
   <223> amorce 5 FXF3
<400> 73
   tcagtggctg atagcattac ctggaaacct tatgacgc 38
<210> 74
   <211> 28
   <212> DNA
   <213> Artificial Sequence
<220>
   <223> amorce 3 FXF3 bis
<400> 74
   gctagttgcc tgagccactg accttttg 28
<210> 75
   <211> 44
   <212> DNA
   <213> Artificial Sequence
<220>
   <223> amorce 5 FXF3 bis
<400> 75
   gctcaggcaa ctagcgatag cattacctgg aaaccttatg acgc 44
<210> 76
   <211> 1500
   <212> DNA
   <213> Artificial Sequence
<220>
   <223> séquence nucléique codant SEQ ID NO:8
<400> 76
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac aggacttcct ggcagaagga ggaggagtga ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 77
   <211> 1500
   <212> DNA
   <213> Artificial Sequence
<220>
   <223> séquence nucléique codant SEQ ID NO:9
<400> 77
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac aggacttcct ggccgagggc ctgaccccta ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 78
   <211> 1500
   <212> DNA
   <213> Artificial Sequence
<220>
   <223> séquence nucléique codant SEQ ID NO:10
<400> 78
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagctcc tctggcgagg cccccgatag cattacctgg     600
   aaaccttatg acgccgctga cctggacccc acagagaacc cctttgacct gctggacttc     660
   aaccagacac agaaggccac caacgccacc ctgtccccta ggatcgtggg aggacaggag     720
   tgcaaggacg gagaatgtcc atggcaggcc ctgctgatta acgaggaaaa tgagggattc     780
   tgcggaggca ctatcctgag cgagttctac attctgaccg cagcccactg tctgtatcag     840
   gctaagcgat tcaaagtgcg ggtcggcgac agaaacaccg agcaggagga agggggagaa     900
   gcagtgcacg aggtcgaagt ggtcatcaag cataatcgct tcactaaaga gacctacgac     960
   tttgatatcg ctgtgctgcg cctgaagaca cctattactt tccgaatgaa cgtcgcccct    1020
   gcttgcctgc cagagcgaga ttgggccgaa agcaccctga tgacacagaa aactggcatc    1080
   gtgagcgggt ttggacggac acatgagaag ggcaggcagt ccactcgcct gaaaatgctg    1140
   gaagtgccct acgtcgaccg gaactcttgt aagctgagta gcagcttcat cattacccag    1200
   aatatgtttt gcgccgggta tgacacaaag caggaggatg cttgtcaggg agacagtggc    1260
   gggcctcacg tgactaggtt caaagatact tattttgtga ccggcatcgt cagctgggga    1320
   gagggatgcg cacgcaaggg gaaatacgga atctatacca aggtgacagc ctttctgaaa    1380
   tggattgacc gatctatgaa gacccggggg ctgccaaagg caaaaagtca tgcccccgag    1440
   gtcattacca gttcccctct gaaagaagac caggtggacc caaggctgat tgacggcaag    1500
<210> 79
   <211> 1500
   <212> DNA
   <213> Artificial Sequence
<220>
   <223> séquence nucléique codant SEQ ID NO:11
<400> 79
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagctcc tctggcgagg cccccgatag cattacctgg     600
   aaaccttatg acgccgctga cctggacccc acagagaacc cctttgacct gctggacttc     660
   aaccagacac agaaggccac ccaggccacc ctgagcccta ggatcgtggg aggacaggag     720
   tgcaaggacg gagaatgtcc atggcaggcc ctgctgatta acgaggaaaa tgagggattc     780
   tgcggaggca ctatcctgag cgagttctac attctgaccg cagcccactg tctgtatcag     840
   gctaagcgat tcaaagtgcg ggtcggcgac agaaacaccg agcaggagga agggggagaa      900
   gcagtgcacg aggtcgaagt ggtcatcaag cataatcgct tcactaaaga gacctacgac     960
   tttgatatcg ctgtgctgcg cctgaagaca cctattactt tccgaatgaa cgtcgcccct    1020
   gcttgcctgc cagagcgaga ttgggccgaa agcaccctga tgacacagaa aactggcatc    1080
   gtgagcgggt ttggacggac acatgagaag ggcaggcagt ccactcgcct gaaaatgctg    1140
   gaagtgccct acgtcgaccg gaactcttgt aagctgagta gcagcttcat cattacccag    1200
   aatatgtttt gcgccgggta tgacacaaag caggaggatg cttgtcaggg agacagtggc    1260
   gggcctcacg tgactaggtt caaagatact tattttgtga ccggcatcgt cagctgggga    1320
   gagggatgcg cacgcaaggg gaaatacgga atctatacca aggtgacagc ctttctgaaa    1380
   tggattgacc gatctatgaa gacccggggg ctgccaaagg caaaaagtca tgcccccgag    1440
   gtcattacca gttcccctct gaaagaagac caggtggacc caaggctgat tgacggcaag    1500
<210> 80
   <211> 1500
   <212> DNA
   <213> Artificial Sequence
<220>
   <223> séquence nucléique codant SEQ ID NO:12
<400> 80
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcaccagc aagctgacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 81
   <211> 1500
   <212> DNA
   <213> Artificial Sequence
<220>
   <223> séquence nucléique codant SEQ ID NO:13
<400> 81
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcttcaac gacttcacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 82
   <211> 1500
   <212> DNA
   <213> Artificial Sequence
<220>
   <223> séquence nucléique codant SEQ ID NO:14
<400> 82
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcctgagc agcatgacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 83
   <211> 1500
   <212> DNA
   <213> Artificial Sequence
<220>
   <223> séquence nucléique codant SEQ ID NO:15
<400> 83
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggccctccc agcctgacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 84
   <211> 1500
   <212> DNA
   <213> Artificial Sequence
<220>
   <223> séquence nucléique codant SEQ ID NO:16
<400> 84
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcctgtcc tgcggccaga ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 85
   <211> 1530
   <212> DNA
   <213> Artificial Sequence
<220>
   <223> séquence nucléique codant SEQ ID NO:17
<400> 85
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagctcc tctggcgagg cccccgatag cattacctgg     600
   aaaccttatg acgccgctga cctggacccc acagagaacc cctttgacct gctggacttc     660
   aaccagacac agcctgaaag aggcgataac aatctgacta gggacttcct ggcagaagga     720
   ggaggagtga ggatcgtggg aggacaggag tgcaaggacg gagaatgtcc atggcaggcc     780
   ctgctgatta acgaggaaaa tgagggattc tgcggaggca ctatcctgag cgagttctac     840
   attctgaccg cagcccactg tctgtatcag gctaagcgat tcaaagtgcg ggtcggcgac     900
   agaaacaccg agcaggagga agggggagaa gcagtgcacg aggtcgaagt ggtcatcaag     960
   cataatcgct tcactaaaga gacctacgac tttgatatcg ctgtgctgcg cctgaagaca    1020
   cctattactt tccgaatgaa cgtcgcccct gcttgcctgc cagagcgaga ttgggccgaa    1080
   agcaccctga tgacacagaa aactggcatc gtgagcgggt ttggacggac acatgagaag    1140
   ggcaggcagt ccactcgcct gaaaatgctg gaagtgccct acgtcgaccg gaactcttgt    1200
   aagctgagta gcagcttcat cattacccag aatatgtttt gcgccgggta tgacacaaag    1260
   caggaggatg cttgtcaggg agacagtggc gggcctcacg tgactaggtt caaagatact    1320
   tattttgtga ccggcatcgt cagctgggga gagggatgcg cacgcaaggg gaaatacgga    1380
   atctatacca aggtgacagc ctttctgaaa tggattgacc gatctatgaa gacccggggg    1440
   ctgccaaagg caaaaagtca tgcccccgag gtcattacca gttcccctct gaaagaagac    1500
   caggtggacc caaggctgat tgacggcaag                                     1530
<210> 86
   <211> 1530
   <212> DNA
   <213> Artificial Sequence
<220>
   <223> séquence nucléique codant SEQ ID NO:18
<400> 86
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta gggacttcct ggccgagggc 720
   ctgaccccta ggatcgtggg aggacaggag tgcaaggacg gagaatgtcc atggcaggcc 780
   ctgctgatta acgaggaaaa tgagggattc tgcggaggca ctatcctgag cgagttctac 840
   attctgaccg cagcccactg tctgtatcag gctaagcgat tcaaagtgcg ggtcggcgac 900
   agaaacaccg agcaggagga agggggagaa gcagtgcacg aggtcgaagt ggtcatcaag 960
   cataatcgct tcactaaaga gacctacgac tttgatatcg ctgtgctgcg cctgaagaca 1020
   cctattactt tccgaatgaa cgtcgcccct gcttgcctgc cagagcgaga ttgggccgaa 1080
   agcaccctga tgacacagaa aactggcatc gtgagcgggt ttggacggac acatgagaag 1140
   ggcaggcagt ccactcgcct gaaaatgctg gaagtgccct acgtcgaccg gaactcttgt 1200
   aagctgagta gcagcttcat cattacccag aatatgtttt gcgccgggta tgacacaaag 1260
   caggaggatg cttgtcaggg agacagtggc gggcctcacg tgactaggtt caaagatact 1320
   tattttgtga ccggcatcgt cagctgggga gagggatgcg cacgcaaggg gaaatacgga 1380
   atctatacca aggtgacagc ctttctgaaa tggattgacc gatctatgaa gacccggggg 1440
   ctgccaaagg caaaaagtca tgcccccgag gtcattacca gttcccctct gaaagaagac 1500
   caggtggacc caaggctgat tgacggcaag 1530
<210> 87
   <211> 1530
   <212> DNA
   <213> Artificial Sequence
<220>
   <223> séquence nucléique codant SEQ ID NO:19
<400> 87
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggaaggccac caacgccacc 720
   ctgtccccta ggatcgtggg aggacaggag tgcaaggacg gagaatgtcc atggcaggcc 780
   ctgctgatta acgaggaaaa tgagggattc tgcggaggca ctatcctgag cgagttctac 840
   attctgaccg cagcccactg tctgtatcag gctaagcgat tcaaagtgcg ggtcggcgac 900
   agaaacaccg agcaggagga agggggagaa gcagtgcacg aggtcgaagt ggtcatcaag 960
   cataatcgct tcactaaaga gacctacgac tttgatatcg ctgtgctgcg cctgaagaca 1020
   cctattactt tccgaatgaa cgtcgcccct gcttgcctgc cagagcgaga ttgggccgaa 1080
   agcaccctga tgacacagaa aactggcatc gtgagcgggt ttggacggac acatgagaag 1140
   ggcaggcagt ccactcgcct gaaaatgctg gaagtgccct acgtcgaccg gaactcttgt 1200
   aagctgagta gcagcttcat cattacccag aatatgtttt gcgccgggta tgacacaaag 1260
   caggaggatg cttgtcaggg agacagtggc gggcctcacg tgactaggtt caaagatact 1320
   tattttgtga ccggcatcgt cagctgggga gagggatgcg cacgcaaggg gaaatacgga 1380
   atctatacca aggtgacagc ctttctgaaa tggattgacc gatctatgaa gacccggggg 1440
   ctgccaaagg caaaaagtca tgcccccgag gtcattacca gttcccctct gaaagaagac 1500
   caggtggacc caaggctgat tgacggcaag 1530
<210> 88
   <211> 1530
   <212> DNA
   <213> Artificial Sequence
<220>
   <223> séquence nucléique codant SEQ ID NO:20
<400> 88
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagctcc tctggcgagg cccccgatag cattacctgg     600
   aaaccttatg acgccgctga cctggacccc acagagaacc cctttgacct gctggacttc     660
   aaccagacac agcctgaaag aggcgataac aatctgacta ggaaggccac ccaggccacc     720
   ctgagcccta ggatcgtggg aggacaggag tgcaaggacg gagaatgtcc atggcaggcc     780
   ctgctgatta acgaggaaaa tgagggattc tgcggaggca ctatcctgag cgagttctac     840
   attctgaccg cagcccactg tctgtatcag gctaagcgat tcaaagtgcg ggtcggcgac     900
   agaaacaccg agcaggagga agggggagaa gcagtgcacg aggtcgaagt ggtcatcaag     960
   cataatcgct tcactaaaga gacctacgac tttgatatcg ctgtgctgcg cctgaagaca    1020
   cctattactt tccgaatgaa cgtcgcccct gcttgcctgc cagagcgaga ttgggccgaa    1080
   agcaccctga tgacacagaa aactggcatc gtgagcgggt ttggacggac acatgagaag    1140
   ggcaggcagt ccactcgcct gaaaatgctg gaagtgccct acgtcgaccg gaactcttgt    1200
   aagctgagta gcagcttcat cattacccag aatatgtttt gcgccgggta tgacacaaag    1260
   caggaggatg cttgtcaggg agacagtggc gggcctcacg tgactaggtt caaagatact    1320
   tattttgtga ccggcatcgt cagctgggga gagggatgcg cacgcaaggg gaaatacgga    1380
   atctatacca aggtgacagc ctttctgaaa tggattgacc gatctatgaa gacccggggg    1440
   ctgccaaagg caaaaagtca tgcccccgag gtcattacca gttcccctct gaaagaagac    1500
   caggtggacc caaggctgat tgacggcaag                                     1530
<210> 89
   <211> 1518
   <212> DNA
   <213> Artificial Sequence
<220>
   <223> séquence nucléique codant SEQ ID NO:21
<400> 89
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagctcc tctggcgagg cccccgatag cattacctgg     600
   aaaccttatg acgccgctga cctggacccc acagagaacc cctttgacct gctggacttc     660
   aaccagacac agcctgaaag aggcgataac aatctgacta ggaccagcaa gctgaccagg     720
   atcgtgggag gacaggagtg caaggacgga gaatgtccat ggcaggccct gctgattaac     780
   gaggaaaatg agggattctg cggaggcact atcctgagcg agttctacat tctgaccgca     840
   gcccactgtc tgtatcaggc taagcgattc aaagtgcggg tcggcgacag aaacaccgag     900
   caggaggaag ggggagaagc agtgcacgag gtcgaagtgg tcatcaagca taatcgcttc      960
   actaaagaga cctacgactt tgatatcgct gtgctgcgcc tgaagacacc tattactttc    1020
   cgaatgaacg tcgcccctgc ttgcctgcca gagcgagatt gggccgaaag caccctgatg    1080
   acacagaaaa ctggcatcgt gagcgggttt ggacggacac atgagaaggg caggcagtcc    1140
   actcgcctga aaatgctgga agtgccctac gtcgaccgga actcttgtaa gctgagtagc    1200
   agcttcatca ttacccagaa tatgttttgc gccgggtatg acacaaagca ggaggatgct    1260
   tgtcagggag acagtggcgg gcctcacgtg actaggttca aagatactta ttttgtgacc    1320
   ggcatcgtca gctggggaga gggatgcgca cgcaagggga aatacggaat ctataccaag    1380
   gtgacagcct ttctgaaatg gattgaccga tctatgaaga cccgggggct gccaaaggca    1440
   aaaagtcatg cccccgaggt cattaccagt tcccctctga aagaagacca ggtggaccca    1500
   aggctgattg acggcaag 1518
<210> 90
   <211> 1518
   <212> DNA
   <213> Artificial Sequence
<220>
   <223> séquence nucléique codant SEQ ID NO:22
<400> 90
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggttcaacga cttcaccagg 720
   atcgtgggag gacaggagtg caaggacgga gaatgtccat ggcaggccct gctgattaac 780
   gaggaaaatg agggattctg cggaggcact atcctgagcg agttctacat tctgaccgca 840
   gcccactgtc tgtatcaggc taagcgattc aaagtgcggg tcggcgacag aaacaccgag 900
   caggaggaag ggggagaagc agtgcacgag gtcgaagtgg tcatcaagca taatcgcttc 960
   actaaagaga cctacgactt tgatatcgct gtgctgcgcc tgaagacacc tattactttc 1020
   cgaatgaacg tcgcccctgc ttgcctgcca gagcgagatt gggccgaaag caccctgatg 1080
   acacagaaaa ctggcatcgt gagcgggttt ggacggacac atgagaaggg caggcagtcc 1140
   actcgcctga aaatgctgga agtgccctac gtcgaccgga actcttgtaa gctgagtagc 1200
   agcttcatca ttacccagaa tatgttttgc gccgggtatg acacaaagca ggaggatgct 1260
   tgtcagggag acagtggcgg gcctcacgtg actaggttca aagatactta ttttgtgacc 1320
   ggcatcgtca gctggggaga gggatgcgca cgcaagggga aatacggaat ctataccaag 1380
   gtgacagcct ttctgaaatg gattgaccga tctatgaaga cccgggggct gccaaaggca 1440
   aaaagtcatg cccccgaggt cattaccagt tcccctctga aagaagacca ggtggaccca 1500
   aggctgattg acggcaag 1518
<210> 91
   <211> 1518
   <212> DNA
   <213> Artificial Sequence
<220>
   <223> séquence nucléique codant SEQ ID NO:23
<400> 91
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggctgagcag catgaccagg 720
   atcgtgggag gacaggagtg caaggacgga gaatgtccat ggcaggccct gctgattaac 780
   gaggaaaatg agggattctg cggaggcact atcctgagcg agttctacat tctgaccgca 840
   gcccactgtc tgtatcaggc taagcgattc aaagtgcggg tcggcgacag aaacaccgag 900
   caggaggaag ggggagaagc agtgcacgag gtcgaagtgg tcatcaagca taatcgcttc 960
   actaaagaga cctacgactt tgatatcgct gtgctgcgcc tgaagacacc tattactttc 1020
   cgaatgaacg tcgcccctgc ttgcctgcca gagcgagatt gggccgaaag caccctgatg 1080
   acacagaaaa ctggcatcgt gagcgggttt ggacggacac atgagaaggg caggcagtcc 1140
   actcgcctga aaatgctgga agtgccctac gtcgaccgga actcttgtaa gctgagtagc 1200
   agcttcatca ttacccagaa tatgttttgc gccgggtatg acacaaagca ggaggatgct 1260
   tgtcagggag acagtggcgg gcctcacgtg actaggttca aagatactta ttttgtgacc 1320
   ggcatcgtca gctggggaga gggatgcgca cgcaagggga aatacggaat ctataccaag 1380
   gtgacagcct ttctgaaatg gattgaccga tctatgaaga cccgggggct gccaaaggca 1440
   aaaagtcatg cccccgaggt cattaccagt tcccctctga aagaagacca ggtggaccca 1500
   aggctgattg acggcaag 1518
<210> 92
   <211> 1518
   <212> DNA
   <213> Artificial Sequence
<220>
   <223> séquence nucléique codant SEQ ID NO:24
<400> 92
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggcctcccag cctgaccagg 720
   atcgtgggag gacaggagtg caaggacgga gaatgtccat ggcaggccct gctgattaac 780
   gaggaaaatg agggattctg cggaggcact atcctgagcg agttctacat tctgaccgca 840
   gcccactgtc tgtatcaggc taagcgattc aaagtgcggg tcggcgacag aaacaccgag 900
   caggaggaag ggggagaagc agtgcacgag gtcgaagtgg tcatcaagca taatcgcttc 960
   actaaagaga cctacgactt tgatatcgct gtgctgcgcc tgaagacacc tattactttc 1020
   cgaatgaacg tcgcccctgc ttgcctgcca gagcgagatt gggccgaaag caccctgatg 1080
   acacagaaaa ctggcatcgt gagcgggttt ggacggacac atgagaaggg caggcagtcc 1140
   actcgcctga aaatgctgga agtgccctac gtcgaccgga actcttgtaa gctgagtagc 1200
   agcttcatca ttacccagaa tatgttttgc gccgggtatg acacaaagca ggaggatgct 1260
   tgtcagggag acagtggcgg gcctcacgtg actaggttca aagatactta ttttgtgacc 1320
   ggcatcgtca gctggggaga gggatgcgca cgcaagggga aatacggaat ctataccaag 1380
   gtgacagcct ttctgaaatg gattgaccga tctatgaaga cccgggggct gccaaaggca 1440
   aaaagtcatg cccccgaggt cattaccagt tcccctctga aagaagacca ggtggaccca 1500
   aggctgattg acggcaag 1518
<210> 93
   <211> 1518
   <212> DNA
   <213> Artificial Sequence
<220>
   <223> séquence nucléique codant SEQ ID NO:25
<400> 93
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagctcc tctggcgagg cccccgatag cattacctgg 600
   aaaccttatg acgccgctga cctggacccc acagagaacc cctttgacct gctggacttc 660
   aaccagacac agcctgaaag aggcgataac aatctgacta ggctgtcctg cggccagagg 720
   atcgtgggag gacaggagtg caaggacgga gaatgtccat ggcaggccct gctgattaac 780
   gaggaaaatg agggattctg cggaggcact atcctgagcg agttctacat tctgaccgca 840
   gcccactgtc tgtatcaggc taagcgattc aaagtgcggg tcggcgacag aaacaccgag 900
   caggaggaag ggggagaagc agtgcacgag gtcgaagtgg tcatcaagca taatcgcttc 960
   actaaagaga cctacgactt tgatatcgct gtgctgcgcc tgaagacacc tattactttc 1020
   cgaatgaacg tcgcccctgc ttgcctgcca gagcgagatt gggccgaaag caccctgatg 1080
   acacagaaaa ctggcatcgt gagcgggttt ggacggacac atgagaaggg caggcagtcc 1140
   actcgcctga aaatgctgga agtgccctac gtcgaccgga actcttgtaa gctgagtagc 1200
   agcttcatca ttacccagaa tatgttttgc gccgggtatg acacaaagca ggaggatgct 1260
   tgtcagggag acagtggcgg gcctcacgtg actaggttca aagatactta ttttgtgacc 1320
   ggcatcgtca gctggggaga gggatgcgca cgcaagggga aatacggaat ctataccaag 1380
   gtgacagcct ttctgaaatg gattgaccga tctatgaaga cccgggggct gccaaaggca 1440
   aaaagtcatg cccccgaggt cattaccagt tcccctctga aagaagacca ggtggaccca 1500
   aggctgattg acggcaag 1518
<210> 94
   <211> 1500
   <212> DNA
   <213> Artificial Sequence
<220>
   <223> séquence nucléique codant SEQ ID NO:26
<400> 94
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctgatag cattacctgg aaaccttatg acgccgctga cctggacccc 600
   acagagaacc cctttgacct gctggacttc aaccagacac agcctgaaag aggcgataac 660
   aatctgacta gggacttcct ggcagaagga ggaggagtga ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 95
   <211> 1500
   <212> DNA
   <213> Artificial Sequence
<220>
   <223> séquence nucléique codant SEQ ID NO:27
<400> 95
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctgatag cattacctgg aaaccttatg acgccgctga cctggacccc 600
   acagagaacc cctttgacct gctggacttc aaccagacac agcctgaaag aggcgataac 660
   aatctgacta gggacttcct ggccgagggc ctgaccccta ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 96
   <211> 1500
   <212> DNA
   <213> Artificial Sequence
<220>
   <223> séquence nucléique codant SEQ ID NO:28
<400> 96
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctgatag cattacctgg aaaccttatg acgccgctga cctggacccc 600
   acagagaacc cctttgacct gctggacttc aaccagacac agcctgaaag aggcgataac 660
   aatctgacta ggaaggccac caacgccacc ctgtccccta ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 97
   <211> 1500
   <212> DNA
   <213> Artificial Sequence
<220>
   <223> séquence nucléique codant SEQ ID NO:29
<400> 97
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctgatag cattacctgg aaaccttatg acgccgctga cctggacccc 600
   acagagaacc cctttgacct gctggacttc aaccagacac agcctgaaag aggcgataac 660
   aatctgacta ggaaggccac ccaggccacc ctgagcccta ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 98
   <211> 1500
   <212> DNA
   <213> Artificial Sequence
<220>
   <223> séquence nucléique codant SEQ ID NO:30
<400> 98
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagcgat agcattacct ggaaacctta tgacgccgct     600
   gacctggacc ccacagagaa cccctttgac ctgctggact tcaaccagac acagcctgaa     660
   agaggcgata acaatctgac taggaccagc aagctgacca ggatcgtggg aggacaggag     720
   tgcaaggacg gagaatgtcc atggcaggcc ctgctgatta acgaggaaaa tgagggattc     780
   tgcggaggca ctatcctgag cgagttctac attctgaccg cagcccactg tctgtatcag     840
   gctaagcgat tcaaagtgcg ggtcggcgac agaaacaccg agcaggagga agggggagaa     900
   gcagtgcacg aggtcgaagt ggtcatcaag cataatcgct tcactaaaga gacctacgac     960
   tttgatatcg ctgtgctgcg cctgaagaca cctattactt tccgaatgaa cgtcgcccct    1020
   gcttgcctgc cagagcgaga ttgggccgaa agcaccctga tgacacagaa aactggcatc    1080
   gtgagcgggt ttggacggac acatgagaag ggcaggcagt ccactcgcct gaaaatgctg    1140
   gaagtgccct acgtcgaccg gaactcttgt aagctgagta gcagcttcat cattacccag    1200
   aatatgtttt gcgccgggta tgacacaaag caggaggatg cttgtcaggg agacagtggc    1260
   gggcctcacg tgactaggtt caaagatact tattttgtga ccggcatcgt cagctgggga    1320
   gagggatgcg cacgcaaggg gaaatacgga atctatacca aggtgacagc ctttctgaaa    1380
   tggattgacc gatctatgaa gacccggggg ctgccaaagg caaaaagtca tgcccccgag    1440
   gtcattacca gttcccctct gaaagaagac caggtggacc caaggctgat tgacggcaag    1500
<210> 99
   <211> 1500
   <212> DNA
   <213> Artificial Sequence
<220>
   <223> séquence nucléique codant SEQ ID NO:31
<400> 99
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagcgat agcattacct ggaaacctta tgacgccgct 600
   gacctggacc ccacagagaa cccctttgac ctgctggact tcaaccagac acagcctgaa 660
   agaggcgata acaatctgac taggttcaac gacttcacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 100
   <211> 1500
   <212> DNA
   <213> Artificial Sequence
<220>
   <223> séquence nucléique codant SEQ ID NO:32
<400> 100
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagcgat agcattacct ggaaacctta tgacgccgct     600
   gacctggacc ccacagagaa cccctttgac ctgctggact tcaaccagac acagcctgaa     660
   agaggcgata acaatctgac taggctgagc agcatgacca ggatcgtggg aggacaggag     720
   tgcaaggacg gagaatgtcc atggcaggcc ctgctgatta acgaggaaaa tgagggattc     780
   tgcggaggca ctatcctgag cgagttctac attctgaccg cagcccactg tctgtatcag     840
   gctaagcgat tcaaagtgcg ggtcggcgac agaaacaccg agcaggagga agggggagaa     900
   gcagtgcacg aggtcgaagt ggtcatcaag cataatcgct tcactaaaga gacctacgac     960
   tttgatatcg ctgtgctgcg cctgaagaca cctattactt tccgaatgaa cgtcgcccct    1020
   gcttgcctgc cagagcgaga ttgggccgaa agcaccctga tgacacagaa aactggcatc    1080
   gtgagcgggt ttggacggac acatgagaag ggcaggcagt ccactcgcct gaaaatgctg    1140
   gaagtgccct acgtcgaccg gaactcttgt aagctgagta gcagcttcat cattacccag    1200
   aatatgtttt gcgccgggta tgacacaaag caggaggatg cttgtcaggg agacagtggc    1260
   gggcctcacg tgactaggtt caaagatact tattttgtga ccggcatcgt cagctgggga    1320
   gagggatgcg cacgcaaggg gaaatacgga atctatacca aggtgacagc ctttctgaaa    1380
   tggattgacc gatctatgaa gacccggggg ctgccaaagg caaaaagtca tgcccccgag    1440
   gtcattacca gttcccctct gaaagaagac caggtggacc caaggctgat tgacggcaag    1500
<210> 101
   <211> 1500
   <212> DNA
   <213> Artificial Sequence
<220>
   <223> séquence nucléique codant SEQ ID NO:33
<400> 101
   atgggaagac ccctgcatct ggtgctgctg tccgcctcac tggctgggct gctgctgctg 60
   ggagaatctc tgtttatccg acgggagcag gctaacaata tcctggcaag agtgcggaga 120
   gccaactctt tcctggagga aatgaagaaa ggccacctgg agcgggaatg catggaggaa 180
   acctgtagtt acgaggaagc cagagaggtg ttcgaagact cagataagac aaacgagttt 240
   tggaataagt acaaagacgg cgatcagtgc gaaactagcc catgtcagaa ccaggggaag 300
   tgcaaagatg gactgggcga gtacacctgc acatgtctgg agggattcga aggcaagaat 360
   tgcgaactgt ttaccagaaa gctgtgctcc ctggataacg gcgactgcga tcagttttgt 420
   catgaggaac agaattccgt ggtctgctct tgtgccaggg gatacacact ggctgacaat 480
   ggcaaggcat gcatccccac cggcccctat ccttgtggga agcagacact ggagaggcgc 540
   aaaaggtcag tggctcaggc aactagcgat agcattacct ggaaacctta tgacgccgct 600
   gacctggacc ccacagagaa cccctttgac ctgctggact tcaaccagac acagcctgaa 660
   agaggcgata acaatctgac taggcctccc agcctgacca ggatcgtggg aggacaggag 720
   tgcaaggacg gagaatgtcc atggcaggcc ctgctgatta acgaggaaaa tgagggattc 780
   tgcggaggca ctatcctgag cgagttctac attctgaccg cagcccactg tctgtatcag 840
   gctaagcgat tcaaagtgcg ggtcggcgac agaaacaccg agcaggagga agggggagaa 900
   gcagtgcacg aggtcgaagt ggtcatcaag cataatcgct tcactaaaga gacctacgac 960
   tttgatatcg ctgtgctgcg cctgaagaca cctattactt tccgaatgaa cgtcgcccct 1020
   gcttgcctgc cagagcgaga ttgggccgaa agcaccctga tgacacagaa aactggcatc 1080
   gtgagcgggt ttggacggac acatgagaag ggcaggcagt ccactcgcct gaaaatgctg 1140
   gaagtgccct acgtcgaccg gaactcttgt aagctgagta gcagcttcat cattacccag 1200
   aatatgtttt gcgccgggta tgacacaaag caggaggatg cttgtcaggg agacagtggc 1260
   gggcctcacg tgactaggtt caaagatact tattttgtga ccggcatcgt cagctgggga 1320
   gagggatgcg cacgcaaggg gaaatacgga atctatacca aggtgacagc ctttctgaaa 1380
   tggattgacc gatctatgaa gacccggggg ctgccaaagg caaaaagtca tgcccccgag 1440
   gtcattacca gttcccctct gaaagaagac caggtggacc caaggctgat tgacggcaag 1500
<210> 102
   <211> 1500
   <212> DNA
   <213> Artificial Sequence
<220>
   <223> séquence nucléique codant SEQ ID NO:34
<400> 102
   atgggaagac ccctgcatct ggtgctgctg tccgcctcac tggctgggct gctgctgctg      60
   ggagaatctc tgtttatccg acgggagcag gctaacaata tcctggcaag agtgcggaga     120
   gccaactctt tcctggagga aatgaagaaa ggccacctgg agcgggaatg catggaggaa     180
   acctgtagtt acgaggaagc cagagaggtg ttcgaagact cagataagac aaacgagttt     240
   tggaataagt acaaagacgg cgatcagtgc gaaactagcc catgtcagaa ccaggggaag     300
   tgcaaagatg gactgggcga gtacacctgc acatgtctgg agggattcga aggcaagaat     360
   tgcgaactgt ttaccagaaa gctgtgctcc ctggataacg gcgactgcga tcagttttgt     420
   catgaggaac agaattccgt ggtctgctct tgtgccaggg gatacacact ggctgacaat     480
   ggcaaggcat gcatccccac cggcccctat ccttgtggga agcagacact ggagaggcgc     540
   aaaaggtcag tggctcaggc aactagcgat agcattacct ggaaacctta tgacgccgct     600
   gacctggacc ccacagagaa cccctttgac ctgctggact tcaaccagac acagcctgaa     660
   agaggcgata acaatctgac taggctgtcc tgcggccaga ggatcgtggg aggacaggag     720
   tgcaaggacg gagaatgtcc atggcaggcc ctgctgatta acgaggaaaa tgagggattc     780
   tgcggaggca ctatcctgag cgagttctac attctgaccg cagcccactg tctgtatcag     840
   gctaagcgat tcaaagtgcg ggtcggcgac agaaacaccg agcaggagga agggggagaa      900
   gcagtgcacg aggtcgaagt ggtcatcaag cataatcgct tcactaaaga gacctacgac     960
   tttgatatcg ctgtgctgcg cctgaagaca cctattactt tccgaatgaa cgtcgcccct    1020
   gcttgcctgc cagagcgaga ttgggccgaa agcaccctga tgacacagaa aactggcatc    1080
   gtgagcgggt ttggacggac acatgagaag ggcaggcagt ccactcgcct gaaaatgctg    1140
   gaagtgccct acgtcgaccg gaactcttgt aagctgagta gcagcttcat cattacccag    1200
   aatatgtttt gcgccgggta tgacacaaag caggaggatg cttgtcaggg agacagtggc    1260
   gggcctcacg tgactaggtt caaagatact tattttgtga ccggcatcgt cagctgggga    1320
   gagggatgcg cacgcaaggg gaaatacgga atctatacca aggtgacagc ctttctgaaa    1380
   tggattgacc gatctatgaa gacccggggg ctgccaaagg caaaaagtca tgcccccgag    1440
   gtcattacca gttcccctct gaaagaagac caggtggacc caaggctgat tgacggcaag    1500
<210> 103
   <211> 460
   <212> PRT
   <213> Artificial Sequence
<220>
   <223> SEQ ID NO:7 sans peptide signal et sans propeptide
<400> 103
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Asp Asn Asn Leu
   180 185 190
   Thr Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 104
   <211> 460
   <212> PRT
   <213> Artificial Sequence
<220>
   <223> SEQ ID NO:8 sans peptide signal et sans propeptide
<400> 104
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Asp Phe Leu Ala Glu Gly Gly Gly
   180 185 190
   Val Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 105
   <211> 460
   <212> PRT
   <213> Artificial Sequence
<220>
   <223> SEQ ID NO:9 sans peptide signal et sans propeptide
<400> 105
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Asp Phe Leu Ala Glu Gly Leu Thr
   180 185 190
   Pro Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 106
   <211> 460
   <212> PRT
   <213> Artificial Sequence
<220>
   <223> SEQ ID NO:10 sans peptide signal et sans propeptide
<400> 106
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Lys Ala Thr Asn Ala Thr Leu Ser
               180                 185                 190
   Pro Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 107
   <211> 460
   <212> PRT
   <213> Artificial Sequence
<220>
   <223> SEQ ID NO:11 sans peptide signal et sans propeptide
<400> 107
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Lys Ala Thr Gln Ala Thr Leu Ser
               180                 185                 190
   Pro Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 108
   <211> 460
   <212> PRT
   <213> Artificial Sequence
<220>
   <223> SEQ ID NO:12 sans peptide signal et sans propeptide
<400> 108
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Thr Ser Lys Leu
   180 185 190
   Thr Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 109
   <211> 460
   <212> PRT
   <213> Artificial Sequence
<220>
   <223> SEQ ID NO:13 sans peptide signal et sans propeptide
<400> 109
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Phe Asn Asp Phe
               180                 185                 190
   Thr Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 110
   <211> 460
   <212> PRT
   <213> Artificial Sequence
<220>
   <223> SEQ ID NO:14 sans peptide signal et sans propeptide
<400> 110
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Leu Ser Ser Met
   180 185 190
   Thr Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 111
   <211> 460
   <212> PRT
   <213> Artificial Sequence
<220>
   <223> SEQ ID NO:15 sans peptide signal et sans propeptide
<400> 111
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Pro Pro Ser Leu
   180 185 190
   Thr Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 112
   <211> 460
   <212> PRT
   <213> Artificial Sequence
<220>
   <223> SEQ ID NO:16 sans peptide signal et sans propeptide
<400> 112
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Leu Ser Cys Gly
   180 185 190
   Gln Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 113
   <211> 470
   <212> PRT
   <213> Artificial Sequence
<220>
   <223> SEQ ID NO:17 sans peptide signal et sans propeptide
<400> 113
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Asp Phe Leu Ala Glu Gly Gly Gly Val Arg Ile Val Gly Gly
           195                 200                 205
   Gln Glu Cys Lys Asp Gly Glu Cys Pro Trp Gln Ala Leu Leu Ile Asn
       210                 215                 220
   Glu Glu Asn Glu Gly Phe Cys Gly Gly Thr Ile Leu Ser Glu Phe Tyr
   225                 230                 235                 240
   Ile Leu Thr Ala Ala His Cys Leu Tyr Gln Ala Lys Arg Phe Lys Val
                   245                 250                 255
   Arg Val Gly Asp Arg Asn Thr Glu Gln Glu Glu Gly Gly Glu Ala Val
               260                 265                 270
   His Glu Val Glu Val Val Ile Lys His Asn Arg Phe Thr Lys Glu Thr
           275                 280                 285
   Tyr Asp Phe Asp Ile Ala Val Leu Arg Leu Lys Thr Pro Ile Thr Phe
       290                 295                 300
   Arg Met Asn Val Ala Pro Ala Cys Leu Pro Glu Arg Asp Trp Ala Glu
   305                 310                 315                 320
   Ser Thr Leu Met Thr Gln Lys Thr Gly Ile Val Ser Gly Phe Gly Arg
   325 330 335
   Thr His Glu Lys Gly Arg Gln Ser Thr Arg Leu Lys Met Leu Glu Val
   340 345 350
   Pro Tyr Val Asp Arg Asn Ser Cys Lys Leu Ser Ser Ser Phe Ile Ile
   355 360 365
   Thr Gln Asn Met Phe Cys Ala Gly Tyr Asp Thr Lys Gln Glu Asp Ala
   370 375 380
   Cys Gln Gly Asp Ser Gly Gly Pro His Val Thr Arg Phe Lys Asp Thr
   385 390 395 400
   Tyr Phe Val Thr Gly Ile Val Ser Trp Gly Glu Gly Cys Ala Arg Lys
   405 410 415
   Gly Lys Tyr Gly Ile Tyr Thr Lys Val Thr Ala Phe Leu Lys Trp Ile
   420 425 430
   Asp Arg Ser Met Lys Thr Arg Gly Leu Pro Lys Ala Lys Ser His Ala
   435 440 445
   Pro Glu Val Ile Thr Ser Ser Pro Leu Lys Glu Asp Gln Val Asp Pro
   450 455 460
   Arg Leu Ile Asp Gly Lys
   465 470
<210> 114
   <211> 470
   <212> PRT
   <213> Artificial Sequence
<220>
   <223> SEQ ID NO:18 sans peptide signal et sans propeptide
<400> 114
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Asp Phe Leu Ala Glu Gly Leu Thr Pro Arg Ile Val Gly Gly
           195                 200                 205
   Gln Glu Cys Lys Asp Gly Glu Cys Pro Trp Gln Ala Leu Leu Ile Asn
       210                 215                 220
   Glu Glu Asn Glu Gly Phe Cys Gly Gly Thr Ile Leu Ser Glu Phe Tyr
   225                 230                 235                 240
   Ile Leu Thr Ala Ala His Cys Leu Tyr Gln Ala Lys Arg Phe Lys Val
                   245                 250                 255
   Arg Val Gly Asp Arg Asn Thr Glu Gln Glu Glu Gly Gly Glu Ala Val
               260                 265                 270
   His Glu Val Glu Val Val Ile Lys His Asn Arg Phe Thr Lys Glu Thr
           275                 280                 285
   Tyr Asp Phe Asp Ile Ala Val Leu Arg Leu Lys Thr Pro Ile Thr Phe
       290                 295                 300
   Arg Met Asn Val Ala Pro Ala Cys Leu Pro Glu Arg Asp Trp Ala Glu
   305 310 315 320
   Ser Thr Leu Met Thr Gln Lys Thr Gly Ile Val Ser Gly Phe Gly Arg
   325 330 335
   Thr His Glu Lys Gly Arg Gln Ser Thr Arg Leu Lys Met Leu Glu Val
   340 345 350
   Pro Tyr Val Asp Arg Asn Ser Cys Lys Leu Ser Ser Ser Phe Ile Ile
   355 360 365
   Thr Gln Asn Met Phe Cys Ala Gly Tyr Asp Thr Lys Gln Glu Asp Ala
   370 375 380
   Cys Gln Gly Asp Ser Gly Gly Pro His Val Thr Arg Phe Lys Asp Thr
   385 390 395 400
   Tyr Phe Val Thr Gly Ile Val Ser Trp Gly Glu Gly Cys Ala Arg Lys
   405 410 415
   Gly Lys Tyr Gly Ile Tyr Thr Lys Val Thr Ala Phe Leu Lys Trp Ile
   420 425 430
   Asp Arg Ser Met Lys Thr Arg Gly Leu Pro Lys Ala Lys Ser His Ala
   435 440 445
   Pro Glu Val Ile Thr Ser Ser Pro Leu Lys Glu Asp Gln Val Asp Pro
   450 455 460
   Arg Leu Ile Asp Gly Lys
   465 470
<210> 115
   <211> 470
   <212> PRT
   <213> Artificial Sequence
<220>
   <223> SEQ ID NO:19 sans peptide signal et sans propeptide
<400> 115
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Lys Ala Thr Asn Ala Thr Leu Ser Pro Arg Ile Val Gly Gly
           195                 200                 205
   Gln Glu Cys Lys Asp Gly Glu Cys Pro Trp Gln Ala Leu Leu Ile Asn
       210                 215                 220
   Glu Glu Asn Glu Gly Phe Cys Gly Gly Thr Ile Leu Ser Glu Phe Tyr
   225                 230                 235                 240
   Ile Leu Thr Ala Ala His Cys Leu Tyr Gln Ala Lys Arg Phe Lys Val
                   245                 250                 255
   Arg Val Gly Asp Arg Asn Thr Glu Gln Glu Glu Gly Gly Glu Ala Val
               260                 265                 270
   His Glu Val Glu Val Val Ile Lys His Asn Arg Phe Thr Lys Glu Thr
           275                 280                 285
   Tyr Asp Phe Asp Ile Ala Val Leu Arg Leu Lys Thr Pro Ile Thr Phe
       290                 295                 300
   Arg Met Asn Val Ala Pro Ala Cys Leu Pro Glu Arg Asp Trp Ala Glu
   305                 310                 315                 320
   Ser Thr Leu Met Thr Gln Lys Thr Gly Ile Val Ser Gly Phe Gly Arg
                   325                 330                 335
   Thr His Glu Lys Gly Arg Gln Ser Thr Arg Leu Lys Met Leu Glu Val
               340                 345                 350
   Pro Tyr Val Asp Arg Asn Ser Cys Lys Leu Ser Ser Ser Phe Ile Ile
           355                 360                 365
   Thr Gln Asn Met Phe Cys Ala Gly Tyr Asp Thr Lys Gln Glu Asp Ala
       370                 375                 380
   Cys Gln Gly Asp Ser Gly Gly Pro His Val Thr Arg Phe Lys Asp Thr
   385                 390                 395                 400
   Tyr Phe Val Thr Gly Ile Val Ser Trp Gly Glu Gly Cys Ala Arg Lys
                   405                 410                 415
   Gly Lys Tyr Gly Ile Tyr Thr Lys Val Thr Ala Phe Leu Lys Trp Ile
               420                 425                 430
   Asp Arg Ser Met Lys Thr Arg Gly Leu Pro Lys Ala Lys Ser His Ala
           435                 440                 445
   Pro Glu Val Ile Thr Ser Ser Pro Leu Lys Glu Asp Gln Val Asp Pro
       450                 455                 460
   Arg Leu Ile Asp Gly Lys
   465                 470
<210> 116
   <211> 470
   <212> PRT
   <213> Artificial Sequence
<220>
   <223> SEQ ID NO:20 sans peptide signal et sans propeptide
<400> 116
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
   35 40 45
   Gln Cys Glu Thr Ser Pro Cys Gln Asn Gln Gly Lys Cys Lys Asp Gly
   50 55 60
   Leu Gly Glu Tyr Thr Cys Thr Cys Leu Glu Gly Phe Glu Gly Lys Asn
   65 70 75 80
   Cys Glu Leu Phe Thr Arg Lys Leu Cys Ser Leu Asp Asn Gly Asp Cys
   85 90 95
   Asp Gln Phe Cys His Glu Glu Gln Asn Ser Val Val Cys Ser Cys Ala
   100 105 110
   Arg Gly Tyr Thr Leu Ala Asp Asn Gly Lys Ala Cys Ile Pro Thr Gly
   115 120 125
   Pro Tyr Pro Cys Gly Lys Gln Thr Leu Glu Arg Arg Lys Arg Ser Val
   130 135 140
   Ala Gln Ala Thr Ser Ser Ser Gly Glu Ala Pro Asp Ser Ile Thr Trp
   145 150 155 160
   Lys Pro Tyr Asp Ala Ala Asp Leu Asp Pro Thr Glu Asn Pro Phe Asp
   165 170 175
   Leu Leu Asp Phe Asn Gln Thr Gln Pro Glu Arg Gly Asp Asn Asn Leu
   180 185 190
   Thr Arg Lys Ala Thr Gln Ala Thr Leu Ser Pro Arg Ile Val Gly Gly
   195 200 205
   Gln Glu Cys Lys Asp Gly Glu Cys Pro Trp Gln Ala Leu Leu Ile Asn
   210 215 220
   Glu Glu Asn Glu Gly Phe Cys Gly Gly Thr Ile Leu Ser Glu Phe Tyr
   225 230 235 240
   Ile Leu Thr Ala Ala His Cys Leu Tyr Gln Ala Lys Arg Phe Lys Val
   245 250 255
   Arg Val Gly Asp Arg Asn Thr Glu Gln Glu Glu Gly Gly Glu Ala Val
   260 265 270
   His Glu Val Glu Val Val Ile Lys His Asn Arg Phe Thr Lys Glu Thr
           275                 280                 285
   Tyr Asp Phe Asp Ile Ala Val Leu Arg Leu Lys Thr Pro Ile Thr Phe
       290                 295                 300
   Arg Met Asn Val Ala Pro Ala Cys Leu Pro Glu Arg Asp Trp Ala Glu
   305                 310                 315                 320
   Ser Thr Leu Met Thr Gln Lys Thr Gly Ile Val Ser Gly Phe Gly Arg
                   325                 330                 335
   Thr His Glu Lys Gly Arg Gln Ser Thr Arg Leu Lys Met Leu Glu Val
               340                 345                 350
   Pro Tyr Val Asp Arg Asn Ser Cys Lys Leu Ser Ser Ser Phe Ile Ile
           355                 360                 365
   Thr Gln Asn Met Phe Cys Ala Gly Tyr Asp Thr Lys Gln Glu Asp Ala
       370                 375                 380
   Cys Gln Gly Asp Ser Gly Gly Pro His Val Thr Arg Phe Lys Asp Thr
   385                 390                 395                 400
   Tyr Phe Val Thr Gly Ile Val Ser Trp Gly Glu Gly Cys Ala Arg Lys
                   405                 410                 415
   Gly Lys Tyr Gly Ile Tyr Thr Lys Val Thr Ala Phe Leu Lys Trp Ile
               420                 425                 430
   Asp Arg Ser Met Lys Thr Arg Gly Leu Pro Lys Ala Lys Ser His Ala
           435                 440                 445
   Pro Glu Val Ile Thr Ser Ser Pro Leu Lys Glu Asp Gln Val Asp Pro
       450                 455                 460
   Arg Leu Ile Asp Gly Lys
   465                 470
<210> 117
   <211> 466
   <212> PRT
   <213> Artificial Sequence
<220>
   <223> SEQ ID NO:21 sans peptide signal et sans propeptide
<400> 117
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Thr Ser Lys Leu Thr Arg Ile Val Gly Gly Gln Glu Cys Lys
           195                 200                 205
   Asp Gly Glu Cys Pro Trp Gln Ala Leu Leu Ile Asn Glu Glu Asn Glu
       210                 215                 220
   Gly Phe Cys Gly Gly Thr Ile Leu Ser Glu Phe Tyr Ile Leu Thr Ala
   225                 230                 235                 240
   Ala His Cys Leu Tyr Gln Ala Lys Arg Phe Lys Val Arg Val Gly Asp
                   245                 250                 255
   Arg Asn Thr Glu Gln Glu Glu Gly Gly Glu Ala Val His Glu Val Glu
   260 265 270
   Val Val Ile Lys His Asn Arg Phe Thr Lys Glu Thr Tyr Asp Phe Asp
   275 280 285
   Ile Ala Val Leu Arg Leu Lys Thr Pro Ile Thr Phe Arg Met Asn Val
   290 295 300
   Ala Pro Ala Cys Leu Pro Glu Arg Asp Trp Ala Glu Ser Thr Leu Met
   305 310 315 320
   Thr Gln Lys Thr Gly Ile Val Ser Gly Phe Gly Arg Thr His Glu Lys
   325 330 335
   Gly Arg Gln Ser Thr Arg Leu Lys Met Leu Glu Val Pro Tyr Val Asp
   340 345 350
   Arg Asn Ser Cys Lys Leu Ser Ser Ser Phe Ile Ile Thr Gln Asn Met
   355 360 365
   Phe Cys Ala Gly Tyr Asp Thr Lys Gln Glu Asp Ala Cys Gln Gly Asp
   370 375 380
   Ser Gly Gly Pro His Val Thr Arg Phe Lys Asp Thr Tyr Phe Val Thr
   385 390 395 400
   Gly Ile Val Ser Trp Gly Glu Gly Cys Ala Arg Lys Gly Lys Tyr Gly
   405 410 415
   Ile Tyr Thr Lys Val Thr Ala Phe Leu Lys Trp Ile Asp Arg Ser Met
   420 425 430
   Lys Thr Arg Gly Leu Pro Lys Ala Lys Ser His Ala Pro Glu Val Ile
   435 440 445
   Thr Ser Ser Pro Leu Lys Glu Asp Gln Val Asp Pro Arg Leu Ile Asp
   450 455 460
   Gly Lys
   465
<210> 118
   <211> 466
   <212> PRT
   <213> Artificial Sequence
<220>
   <223> SEQ ID NO:22 sans peptide signal et sans propeptide
<400> 118
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Phe Asn Asp Phe Thr Arg Ile Val Gly Gly Gln Glu Cys Lys
           195                 200                 205
   Asp Gly Glu Cys Pro Trp Gln Ala Leu Leu Ile Asn Glu Glu Asn Glu
       210                 215                 220
   Gly Phe Cys Gly Gly Thr Ile Leu Ser Glu Phe Tyr Ile Leu Thr Ala
   225                 230                 235                 240
   Ala His Cys Leu Tyr Gln Ala Lys Arg Phe Lys Val Arg Val Gly Asp
                   245                 250                 255
   Arg Asn Thr Glu Gln Glu Glu Gly Gly Glu Ala Val His Glu Val Glu
               260                 265                 270
   Val Val Ile Lys His Asn Arg Phe Thr Lys Glu Thr Tyr Asp Phe Asp
           275                 280                 285
   Ile Ala Val Leu Arg Leu Lys Thr Pro Ile Thr Phe Arg Met Asn Val
       290                 295                 300
   Ala Pro Ala Cys Leu Pro Glu Arg Asp Trp Ala Glu Ser Thr Leu Met
   305                 310                 315                 320
   Thr Gln Lys Thr Gly Ile Val Ser Gly Phe Gly Arg Thr His Glu Lys
                   325                 330                 335
   Gly Arg Gln Ser Thr Arg Leu Lys Met Leu Glu Val Pro Tyr Val Asp
               340                 345                 350
   Arg Asn Ser Cys Lys Leu Ser Ser Ser Phe Ile Ile Thr Gln Asn Met
           355                 360                 365
   Phe Cys Ala Gly Tyr Asp Thr Lys Gln Glu Asp Ala Cys Gln Gly Asp
       370                 375                 380
   Ser Gly Gly Pro His Val Thr Arg Phe Lys Asp Thr Tyr Phe Val Thr
   385                 390                 395                 400
   Gly Ile Val Ser Trp Gly Glu Gly Cys Ala Arg Lys Gly Lys Tyr Gly
                   405                 410                 415
   Ile Tyr Thr Lys Val Thr Ala Phe Leu Lys Trp Ile Asp Arg Ser Met
               420                 425                 430
   Lys Thr Arg Gly Leu Pro Lys Ala Lys Ser His Ala Pro Glu Val Ile
           435                 440                 445
   Thr Ser Ser Pro Leu Lys Glu Asp Gln Val Asp Pro Arg Leu Ile Asp
       450                 455                 460
   Gly Lys
   465
<210> 119
   <211> 466
   <212> PRT
   <213> Artificial Sequence
<220>
   <223> SEQ ID NO:23 sans peptide signal et sans propeptide
<400> 119
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Leu Ser Ser Met Thr Arg Ile Val Gly Gly Gln Glu Cys Lys
           195                 200                 205
   Asp Gly Glu Cys Pro Trp Gln Ala Leu Leu Ile Asn Glu Glu Asn Glu
       210                 215                 220
   Gly Phe Cys Gly Gly Thr Ile Leu Ser Glu Phe Tyr Ile Leu Thr Ala
   225 230 235 240
   Ala His Cys Leu Tyr Gln Ala Lys Arg Phe Lys Val Arg Val Gly Asp
   245 250 255
   Arg Asn Thr Glu Gln Glu Glu Gly Gly Glu Ala Val His Glu Val Glu
   260 265 270
   Val Val Ile Lys His Asn Arg Phe Thr Lys Glu Thr Tyr Asp Phe Asp
   275 280 285
   Ile Ala Val Leu Arg Leu Lys Thr Pro Ile Thr Phe Arg Met Asn Val
   290 295 300
   Ala Pro Ala Cys Leu Pro Glu Arg Asp Trp Ala Glu Ser Thr Leu Met
   305 310 315 320
   Thr Gln Lys Thr Gly Ile Val Ser Gly Phe Gly Arg Thr His Glu Lys
   325 330 335
   Gly Arg Gln Ser Thr Arg Leu Lys Met Leu Glu Val Pro Tyr Val Asp
   340 345 350
   Arg Asn Ser Cys Lys Leu Ser Ser Ser Phe Ile Ile Thr Gln Asn Met
   355 360 365
   Phe Cys Ala Gly Tyr Asp Thr Lys Gln Glu Asp Ala Cys Gln Gly Asp
   370 375 380
   Ser Gly Gly Pro His Val Thr Arg Phe Lys Asp Thr Tyr Phe Val Thr
   385 390 395 400
   Gly Ile Val Ser Trp Gly Glu Gly Cys Ala Arg Lys Gly Lys Tyr Gly
   405 410 415
   Ile Tyr Thr Lys Val Thr Ala Phe Leu Lys Trp Ile Asp Arg Ser Met
   420 425 430
   Lys Thr Arg Gly Leu Pro Lys Ala Lys Ser His Ala Pro Glu Val Ile
   435 440 445
   Thr Ser Ser Pro Leu Lys Glu Asp Gln Val Asp Pro Arg Leu Ile Asp
   450 455 460
   Gly Lys
   465
<210> 120
   <211> 466
   <212> PRT
   <213> Artificial Sequence
<220>
   <223> SEQ ID NO:24 sans peptide signal et sans propeptide
<400> 120
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Pro Pro Ser Leu Thr Arg Ile Val Gly Gly Gln Glu Cys Lys
           195                 200                 205
   Asp Gly Glu Cys Pro Trp Gln Ala Leu Leu Ile Asn Glu Glu Asn Glu
   210 215 220
   Gly Phe Cys Gly Gly Thr Ile Leu Ser Glu Phe Tyr Ile Leu Thr Ala
   225 230 235 240
   Ala His Cys Leu Tyr Gln Ala Lys Arg Phe Lys Val Arg Val Gly Asp
   245 250 255
   Arg Asn Thr Glu Gln Glu Glu Gly Gly Glu Ala Val His Glu Val Glu
   260 265 270
   Val Val Ile Lys His Asn Arg Phe Thr Lys Glu Thr Tyr Asp Phe Asp
   275 280 285
   Ile Ala Val Leu Arg Leu Lys Thr Pro Ile Thr Phe Arg Met Asn Val
   290 295 300
   Ala Pro Ala Cys Leu Pro Glu Arg Asp Trp Ala Glu Ser Thr Leu Met
   305 310 315 320
   Thr Gln Lys Thr Gly Ile Val Ser Gly Phe Gly Arg Thr His Glu Lys
   325 330 335
   Gly Arg Gln Ser Thr Arg Leu Lys Met Leu Glu Val Pro Tyr Val Asp
   340 345 350
   Arg Asn Ser Cys Lys Leu Ser Ser Ser Phe Ile Ile Thr Gln Asn Met
   355 360 365
   Phe Cys Ala Gly Tyr Asp Thr Lys Gln Glu Asp Ala Cys Gln Gly Asp
   370 375 380
   Ser Gly Gly Pro His Val Thr Arg Phe Lys Asp Thr Tyr Phe Val Thr
   385 390 395 400
   Gly Ile Val Ser Trp Gly Glu Gly Cys Ala Arg Lys Gly Lys Tyr Gly
   405 410 415
   Ile Tyr Thr Lys Val Thr Ala Phe Leu Lys Trp Ile Asp Arg Ser Met
   420 425 430
   Lys Thr Arg Gly Leu Pro Lys Ala Lys Ser His Ala Pro Glu Val Ile
   435 440 445
   Thr Ser Ser Pro Leu Lys Glu Asp Gln Val Asp Pro Arg Leu Ile Asp
   450 455 460
   Gly Lys
   465
<210> 121
   <211> 466
   <212> PRT
   <213> Artificial Sequence
<220>
   <223> SEQ ID NO:25 sans peptide signal et sans propeptide
<400> 121
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Ser Ser Gly Glu Ala Pro Asp Ser Ile Thr Trp
   145                 150                 155                 160
   Lys Pro Tyr Asp Ala Ala Asp Leu Asp Pro Thr Glu Asn Pro Phe Asp
                   165                 170                 175
   Leu Leu Asp Phe Asn Gln Thr Gln Pro Glu Arg Gly Asp Asn Asn Leu
               180                 185                 190
   Thr Arg Leu Ser Cys Gly Gln Arg Ile Val Gly Gly Gln Glu Cys Lys
               195                 200                 205
   Asp Gly Glu Cys Pro Trp Gln Ala Leu Leu Ile Asn Glu Glu Asn Glu
       210                 215                 220
   Gly Phe Cys Gly Gly Thr Ile Leu Ser Glu Phe Tyr Ile Leu Thr Ala
   225                 230                 235                 240
   Ala His Cys Leu Tyr Gln Ala Lys Arg Phe Lys Val Arg Val Gly Asp
                   245                 250                 255
   Arg Asn Thr Glu Gln Glu Glu Gly Gly Glu Ala Val His Glu Val Glu
               260                 265                 270
   Val Val Ile Lys His Asn Arg Phe Thr Lys Glu Thr Tyr Asp Phe Asp
           275                 280                 285
   Ile Ala Val Leu Arg Leu Lys Thr Pro Ile Thr Phe Arg Met Asn Val
       290                 295                 300
   Ala Pro Ala Cys Leu Pro Glu Arg Asp Trp Ala Glu Ser Thr Leu Met
   305                 310                 315                 320
   Thr Gln Lys Thr Gly Ile Val Ser Gly Phe Gly Arg Thr His Glu Lys
                   325                 330                 335
   Gly Arg Gln Ser Thr Arg Leu Lys Met Leu Glu Val Pro Tyr Val Asp
               340                 345                 350
   Arg Asn Ser Cys Lys Leu Ser Ser Ser Phe Ile Ile Thr Gln Asn Met
           355                 360                 365
   Phe Cys Ala Gly Tyr Asp Thr Lys Gln Glu Asp Ala Cys Gln Gly Asp
       370                 375                 380
   Ser Gly Gly Pro His Val Thr Arg Phe Lys Asp Thr Tyr Phe Val Thr
   385                 390                 395                 400
   Gly Ile Val Ser Trp Gly Glu Gly Cys Ala Arg Lys Gly Lys Tyr Gly
                   405                 410                 415
   Ile Tyr Thr Lys Val Thr Ala Phe Leu Lys Trp Ile Asp Arg Ser Met
               420                 425                 430
   Lys Thr Arg Gly Leu Pro Lys Ala Lys Ser His Ala Pro Glu Val Ile
           435                 440                 445
   Thr Ser Ser Pro Leu Lys Glu Asp Gln Val Asp Pro Arg Leu Ile Asp
       450                 455                 460
   Gly Lys
   465
<210> 122
   <211> 460
   <212> PRT
   <213> Artificial Sequence
<220>
   <223> SEQ ID NO:26 sans peptide signal et sans propeptide
<400> 122
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Asp Ser Ile Thr Trp Lys Pro Tyr Asp Ala Ala Asp Leu Asp Pro
   145                 150                 155                 160
   Thr Glu Asn Pro Phe Asp Leu Leu Asp Phe Asn Gln Thr Gln Pro Glu
                   165                 170                 175
   Arg Gly Asp Asn Asn Leu Thr Arg Asp Phe Leu Ala Glu Gly Gly Gly
               180                 185                 190
   Val Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 123
   <211> 460
   <212> PRT
   <213> Artificial Sequence
<220>
   <223> SEQ ID NO:27 sans peptide signal et sans propeptide
<400> 123
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Asp Ser Ile Thr Trp Lys Pro Tyr Asp Ala Ala Asp Leu Asp Pro
   145                 150                 155                 160
   Thr Glu Asn Pro Phe Asp Leu Leu Asp Phe Asn Gln Thr Gln Pro Glu
                   165                 170                 175
   Arg Gly Asp Asn Asn Leu Thr Arg Asp Phe Leu Ala Glu Gly Leu Thr
                   180                 185                 190
   Pro Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 124
   <211> 460
   <212> PRT
   <213> Artificial Sequence
<220>
   <223> SEQ ID NO:28 sans peptide signal et sans propeptide
<400> 124
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Asp Ser Ile Thr Trp Lys Pro Tyr Asp Ala Ala Asp Leu Asp Pro
   145                 150                 155                 160
   Thr Glu Asn Pro Phe Asp Leu Leu Asp Phe Asn Gln Thr Gln Pro Glu
                   165                 170                 175
   Arg Gly Asp Asn Asn Leu Thr Arg Lys Ala Thr Asn Ala Thr Leu Ser
               180                 185                 190
   Pro Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 125
   <211> 460
   <212> PRT
   <213> Artificial Sequence
<220>
   <223> SEQ ID NO:29 sans peptide signal et sans propeptide
<400> 125
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Asp Ser Ile Thr Trp Lys Pro Tyr Asp Ala Ala Asp Leu Asp Pro
   145                 150                 155                 160
   Thr Glu Asn Pro Phe Asp Leu Leu Asp Phe Asn Gln Thr Gln Pro Glu
                   165                 170                 175
   Arg Gly Asp Asn Asn Leu Thr Arg Lys Ala Thr Gln Ala Thr Leu Ser
               180                 185                 190
   Pro Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 126
   <211> 460
   <212> PRT
   <213> Artificial Sequence
<220>
   <223> SEQ ID NO:30 sans peptide signal et sans propeptide
<400> 126
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Asp Ser Ile Thr Trp Lys Pro Tyr Asp Ala Ala
   145                 150                 155                 160
   Asp Leu Asp Pro Thr Glu Asn Pro Phe Asp Leu Leu Asp Phe Asn Gln
                   165                 170                 175
   Thr Gln Pro Glu Arg Gly Asp Asn Asn Leu Thr Arg Thr Ser Lys Leu
               180                 185                 190
   Thr Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 127
   <211> 460
   <212> PRT
   <213> Artificial Sequence
<220>
   <223> SEQ ID NO:31 sans peptide signal et sans propeptide
<400> 127
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Asp Ser Ile Thr Trp Lys Pro Tyr Asp Ala Ala
   145                 150                 155                 160
   Asp Leu Asp Pro Thr Glu Asn Pro Phe Asp Leu Leu Asp Phe Asn Gln
                   165                 170                 175
   Thr Gln Pro Glu Arg Gly Asp Asn Asn Leu Thr Arg Phe Asn Asp Phe
               180                 185                 190
   Thr Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                     445
   Glu Asp Gln Val Asp Pro Arg Leu Ile Asp Gly Lys
       450                 455                 460
<210> 128
   <211> 460
   <212> PRT
   <213> Artificial Sequence
<220>
   <223> SEQ ID NO:32 sans peptide signal et sans propeptide
<400> 128
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Asp Ser Ile Thr Trp Lys Pro Tyr Asp Ala Ala
   145                 150                 155                 160
   Asp Leu Asp Pro Thr Glu Asn Pro Phe Asp Leu Leu Asp Phe Asn Gln
                   165                 170                 175
   Thr Gln Pro Glu Arg Gly Asp Asn Asn Leu Thr Arg Leu Ser Ser Met
               180                 185                 190
   Thr Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 129
   <211> 460
   <212> PRT
   <213> Artificial Sequence
<220>
   <223> SEQ ID NO:33 sans peptide signal et sans propeptide
<400> 129
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Asp Ser Ile Thr Trp Lys Pro Tyr Asp Ala Ala
   145                 150                 155                 160
   Asp Leu Asp Pro Thr Glu Asn Pro Phe Asp Leu Leu Asp Phe Asn Gln
                   165                 170                 175
   Thr Gln Pro Glu Arg Gly Asp Asn Asn Leu Thr Arg Pro Pro Ser Leu
               180                 185                 190
   Thr Arg Ile Val Gly Gly Gln Glu Cys Lys Asp Gly Glu Cys Pro Trp
           195                 200                 205
   Gln Ala Leu Leu Ile Asn Glu Glu Asn Glu Gly Phe Cys Gly Gly Thr
       210                 215                 220
   Ile Leu Ser Glu Phe Tyr Ile Leu Thr Ala Ala His Cys Leu Tyr Gln
   225                 230                 235                 240
   Ala Lys Arg Phe Lys Val Arg Val Gly Asp Arg Asn Thr Glu Gln Glu
                   245                 250                 255
   Glu Gly Gly Glu Ala Val His Glu Val Glu Val Val Ile Lys His Asn
               260                 265                 270
   Arg Phe Thr Lys Glu Thr Tyr Asp Phe Asp Ile Ala Val Leu Arg Leu
           275                 280                 285
   Lys Thr Pro Ile Thr Phe Arg Met Asn Val Ala Pro Ala Cys Leu Pro
       290                 295                 300
   Glu Arg Asp Trp Ala Glu Ser Thr Leu Met Thr Gln Lys Thr Gly Ile
   305                 310                 315                 320
   Val Ser Gly Phe Gly Arg Thr His Glu Lys Gly Arg Gln Ser Thr Arg
                   325                 330                 335
   Leu Lys Met Leu Glu Val Pro Tyr Val Asp Arg Asn Ser Cys Lys Leu
               340                 345                 350
   Ser Ser Ser Phe Ile Ile Thr Gln Asn Met Phe Cys Ala Gly Tyr Asp
           355                 360                 365
   Thr Lys Gln Glu Asp Ala Cys Gln Gly Asp Ser Gly Gly Pro His Val
       370                 375                 380
   Thr Arg Phe Lys Asp Thr Tyr Phe Val Thr Gly Ile Val Ser Trp Gly
   385                 390                 395                 400
   Glu Gly Cys Ala Arg Lys Gly Lys Tyr Gly Ile Tyr Thr Lys Val Thr
                   405                 410                 415
   Ala Phe Leu Lys Trp Ile Asp Arg Ser Met Lys Thr Arg Gly Leu Pro
               420                 425                 430
   Lys Ala Lys Ser His Ala Pro Glu Val Ile Thr Ser Ser Pro Leu Lys
           435                 440                 445
   Glu Asp Gln Val Asp Pro Arg Leu Ile Asp Gly Lys
       450                 455                 460
<210> 130
   <211> 460
   <212> PRT
   <213> Artificial Sequence
<220>
   <223> SEQ ID NO:34 sans peptide signal et sans propeptide
<400> 130
   Ala Asn Ser Phe Leu Glu Glu Met Lys Lys Gly His Leu Glu Arg Glu
   1               5                   10                  15
   Cys Met Glu Glu Thr Cys Ser Tyr Glu Glu Ala Arg Glu Val Phe Glu
               20                  25                  30
   Asp Ser Asp Lys Thr Asn Glu Phe Trp Asn Lys Tyr Lys Asp Gly Asp
           35                  40                  45
   Gln Cys Glu Thr Ser Pro Cys Gln Asn Gln Gly Lys Cys Lys Asp Gly
       50                  55                  60
   Leu Gly Glu Tyr Thr Cys Thr Cys Leu Glu Gly Phe Glu Gly Lys Asn
   65                  70                  75                  80
   Cys Glu Leu Phe Thr Arg Lys Leu Cys Ser Leu Asp Asn Gly Asp Cys
                   85                  90                  95
   Asp Gln Phe Cys His Glu Glu Gln Asn Ser Val Val Cys Ser Cys Ala
               100                 105                 110
   Arg Gly Tyr Thr Leu Ala Asp Asn Gly Lys Ala Cys Ile Pro Thr Gly
           115                 120                 125
   Pro Tyr Pro Cys Gly Lys Gln Thr Leu Glu Arg Arg Lys Arg Ser Val
       130                 135                 140
   Ala Gln Ala Thr Ser Asp Ser Ile Thr Trp Lys Pro Tyr Asp Ala Ala
   145                 150                 155                 160
   Asp Leu Asp Pro Thr Glu Asn Pro Phe Asp Leu Leu Asp Phe Asn Gln
                   165                 170                 175
   Thr Gln Pro Glu Arg Gly Asp Asn Asn Leu Thr Arg Leu Ser Cys Gly
   180 185 190
   Gln Arg Ile Val Gly Gly Gln Glu Cys Lys Asp Gly Glu Cys Pro Trp
   195 200 205
   Gln Ala Leu Leu Ile Asn Glu Glu Asn Glu Gly Phe Cys Gly Gly Thr
   210 215 220
   Ile Leu Ser Glu Phe Tyr Ile Leu Thr Ala Ala His Cys Leu Tyr Gln
   225 230 235 240
   Ala Lys Arg Phe Lys Val Arg Val Gly Asp Arg Asn Thr Glu Gln Glu
   245 250 255
   Glu Gly Gly Glu Ala Val His Glu Val Glu Val Val Ile Lys His Asn
   260 265 270
   Arg Phe Thr Lys Glu Thr Tyr Asp Phe Asp Ile Ala Val Leu Arg Leu
   275 280 285
   Lys Thr Pro Ile Thr Phe Arg Met Asn Val Ala Pro Ala Cys Leu Pro
   290 295 300
   Glu Arg Asp Trp Ala Glu Ser Thr Leu Met Thr Gln Lys Thr Gly Ile
   305 310 315 320
   Val Ser Gly Phe Gly Arg Thr His Glu Lys Gly Arg Gln Ser Thr Arg
   325 330 335
   Leu Lys Met Leu Glu Val Pro Tyr Val Asp Arg Asn Ser Cys Lys Leu
   340 345 350
   Ser Ser Ser Phe Ile Ile Thr Gln Asn Met Phe Cys Ala Gly Tyr Asp
   355 360 365
   Thr Lys Gln Glu Asp Ala Cys Gln Gly Asp Ser Gly Gly Pro His Val
   370 375 380
   Thr Arg Phe Lys Asp Thr Tyr Phe Val Thr Gly Ile Val Ser Trp Gly
   385 390 395 400
   Glu Gly Cys Ala Arg Lys Gly Lys Tyr Gly Ile Tyr Thr Lys Val Thr
   405 410 415
   Ala Phe Leu Lys Trp Ile Asp Arg Ser Met Lys Thr Arg Gly Leu Pro
   420 425 430
   Lys Ala Lys Ser His Ala Pro Glu Val Ile Thr Ser Ser Pro Leu Lys
   435 440 445
   Glu Asp Gln Val Asp Pro Arg Leu Ile Asp Gly Lys
   450 455 460
<210> 131
   <211> 1380
   <212> DNA
   <213> Artificial Sequence
<220>
   <223> acide nucléique codant SEQ ID NO:103
<400> 131
   gccaactctt tcctggagga aatgaagaaa ggccacctgg agcgggaatg catggaggaa      60
   acctgtagtt acgaggaagc cagagaggtg ttcgaagact cagataagac aaacgagttt     120
   tggaataagt acaaagacgg cgatcagtgc gaaactagcc catgtcagaa ccaggggaag     180
   tgcaaagatg gactgggcga gtacacctgc acatgtctgg agggattcga aggcaagaat     240
   tgcgaactgt ttaccagaaa gctgtgctcc ctggataacg gcgactgcga tcagttttgt     300
   catgaggaac agaattccgt ggtctgctct tgtgccaggg gatacacact ggctgacaat     360
   ggcaaggcat gcatccccac cggcccctat ccttgtggga agcagacact ggagaggcgc     420
   aaaaggtcag tggctcaggc aactagctcc tctggcgagg cccccgatag cattacctgg     480
   aaaccttatg acgccgctga cctggacccc acagagaacc cctttgacct gctggacttc     540
   aaccagacac agcctgaaag aggcgataac aatctgacta ggatcgtggg aggacaggag     600
   tgcaaggacg gagaatgtcc atggcaggcc ctgctgatta acgaggaaaa tgagggattc     660
   tgcggaggca ctatcctgag cgagttctac attctgaccg cagcccactg tctgtatcag     720
   gctaagcgat tcaaagtgcg ggtcggcgac agaaacaccg agcaggagga agggggagaa      780
   gcagtgcacg aggtcgaagt ggtcatcaag cataatcgct tcactaaaga gacctacgac     840
   tttgatatcg ctgtgctgcg cctgaagaca cctattactt tccgaatgaa cgtcgcccct     900
   gcttgcctgc cagagcgaga ttgggccgaa agcaccctga tgacacagaa aactggcatc     960
   gtgagcgggt ttggacggac acatgagaag ggcaggcagt ccactcgcct gaaaatgctg    1020
   gaagtgccct acgtcgaccg gaactcttgt aagctgagta gcagcttcat cattacccag    1080
   aatatgtttt gcgccgggta tgacacaaag caggaggatg cttgtcaggg agacagtggc    1140
   gggcctcacg tgactaggtt caaagatact tattttgtga ccggcatcgt cagctgggga    1200
   gagggatgcg cacgcaaggg gaaatacgga atctatacca aggtgacagc ctttctgaaa    1260
   tggattgacc gatctatgaa gacccggggg ctgccaaagg caaaaagtca tgcccccgag    1320
   gtcattacca gttcccctct gaaagaagac caggtggacc caaggctgat tgacggcaag    1380
<210> 132
   <211> 1380
   <212> DNA
   <213> Artificial Sequence
<220>
   <223> acide nucléique codant SEQ ID NO:104
<400> 132
   gccaactctt tcctggagga aatgaagaaa ggccacctgg agcgggaatg catggaggaa      60
   acctgtagtt acgaggaagc cagagaggtg ttcgaagact cagataagac aaacgagttt     120
   tggaataagt acaaagacgg cgatcagtgc gaaactagcc catgtcagaa ccaggggaag     180
   tgcaaagatg gactgggcga gtacacctgc acatgtctgg agggattcga aggcaagaat     240
   tgcgaactgt ttaccagaaa gctgtgctcc ctggataacg gcgactgcga tcagttttgt     300
   catgaggaac agaattccgt ggtctgctct tgtgccaggg gatacacact ggctgacaat     360
   ggcaaggcat gcatccccac cggcccctat ccttgtggga agcagacact ggagaggcgc     420
   aaaaggtcag tggctcaggc aactagctcc tctggcgagg cccccgatag cattacctgg     480
   aaaccttatg acgccgctga cctggacccc acagagaacc cctttgacct gctggacttc     540
   aaccagacac aggacttcct ggcagaagga ggaggagtga ggatcgtggg aggacaggag     600
   tgcaaggacg gagaatgtcc atggcaggcc ctgctgatta acgaggaaaa tgagggattc     660
   tgcggaggca ctatcctgag cgagttctac attctgaccg cagcccactg tctgtatcag     720
   gctaagcgat tcaaagtgcg ggtcggcgac agaaacaccg agcaggagga agggggagaa      780
   gcagtgcacg aggtcgaagt ggtcatcaag cataatcgct tcactaaaga gacctacgac     840
   tttgatatcg ctgtgctgcg cctgaagaca cctattactt tccgaatgaa cgtcgcccct     900
   gcttgcctgc cagagcgaga ttgggccgaa agcaccctga tgacacagaa aactggcatc     960
   gtgagcgggt ttggacggac acatgagaag ggcaggcagt ccactcgcct gaaaatgctg    1020
   gaagtgccct acgtcgaccg gaactcttgt aagctgagta gcagcttcat cattacccag    1080
   aatatgtttt gcgccgggta tgacacaaag caggaggatg cttgtcaggg agacagtggc    1140
   gggcctcacg tgactaggtt caaagatact tattttgtga ccggcatcgt cagctgggga    1200
   gagggatgcg cacgcaaggg gaaatacgga atctatacca aggtgacagc ctttctgaaa    1260
   tggattgacc gatctatgaa gacccggggg ctgccaaagg caaaaagtca tgcccccgag    1320
   gtcattacca gttcccctct gaaagaagac caggtggacc caaggctgat tgacggcaag    1380
<210> 133
   <211> 1380
   <212> DNA
   <213> Artificial Sequence
<220>
   <223> acide nucléique codant SEQ ID NO:105
<400> 133
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac aggacttcct ggccgagggc ctgaccccta ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 134
   <211> 1380
   <212> DNA
   <213> Artificial Sequence
<220>
   <223> acide nucléique codant SEQ ID NO:106
<400> 134
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag      180
   tgcaaagatg gactgggcga gtacacctgc acatgtctgg agggattcga aggcaagaat      240
   tgcgaactgt ttaccagaaa gctgtgctcc ctggataacg gcgactgcga tcagttttgt      300
   catgaggaac agaattccgt ggtctgctct tgtgccaggg gatacacact ggctgacaat      360
   ggcaaggcat gcatccccac cggcccctat ccttgtggga agcagacact ggagaggcgc      420
   aaaaggtcag tggctcaggc aactagctcc tctggcgagg cccccgatag cattacctgg      480
   aaaccttatg acgccgctga cctggacccc acagagaacc cctttgacct gctggacttc      540
   aaccagacac agaaggccac caacgccacc ctgtccccta ggatcgtggg aggacaggag      600
   tgcaaggacg gagaatgtcc atggcaggcc ctgctgatta acgaggaaaa tgagggattc      660
   tgcggaggca ctatcctgag cgagttctac attctgaccg cagcccactg tctgtatcag      720
   gctaagcgat tcaaagtgcg ggtcggcgac agaaacaccg agcaggagga agggggagaa      780
   gcagtgcacg aggtcgaagt ggtcatcaag cataatcgct tcactaaaga gacctacgac      840
   tttgatatcg ctgtgctgcg cctgaagaca cctattactt tccgaatgaa cgtcgcccct      900
   gcttgcctgc cagagcgaga ttgggccgaa agcaccctga tgacacagaa aactggcatc      960
   gtgagcgggt ttggacggac acatgagaag ggcaggcagt ccactcgcct gaaaatgctg     1020
   gaagtgccct acgtcgaccg gaactcttgt aagctgagta gcagcttcat cattacccag     1080
   aatatgtttt gcgccgggta tgacacaaag caggaggatg cttgtcaggg agacagtggc     1140
   gggcctcacg tgactaggtt caaagatact tattttgtga ccggcatcgt cagctgggga     1200
   gagggatgcg cacgcaaggg gaaatacgga atctatacca aggtgacagc ctttctgaaa     1260
   tggattgacc gatctatgaa gacccggggg ctgccaaagg caaaaagtca tgcccccgag     1320
   gtcattacca gttcccctct gaaagaagac caggtggacc caaggctgat tgacggcaag     1380
<210> 135
   <211> 1380
   <212> DNA
   <213> Artificial Sequence
<220>
   <223> acide nucléique codant SEQ ID NO:107
<400> 135
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agaaggccac ccaggccacc ctgagcccta ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 136
   <211> 1380
   <212> DNA
   <213> Artificial Sequence
<220>
   <223> acide nucléique codant SEQ ID NO:108
<400> 136
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcaccagc aagctgacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 137
   <211> 1380
   <212> DNA
   <213> Artificial Sequence
<220>
   <223> acide nucléique codant SEQ ID NO:109
<400> 137
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcttcaac gacttcacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 138
   <211> 1380
   <212> DNA
   <213> Artificial Sequence
<220>
   <223> acide nucléique codant SEQ ID NO:110
<400> 138
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcctgagc agcatgacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 139
   <211> 1380
   <212> DNA
   <213> Artificial Sequence
<220>
   <223> acide nucléique codant SEQ ID NO:111
<400> 139
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggccctccc agcctgacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 140
   <211> 1380
   <212> DNA
   <213> Artificial Sequence
<220>
   <223> acide nucléique codant SEQ ID NO:112
<400> 140
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcctgtcc tgcggccaga ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 141
   <211> 1410
   <212> DNA
   <213> Artificial Sequence
<220>
   <223> acide nucléique codant SEQ ID NO:113
<400> 141
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcgataac aatctgacta gggacttcct ggcagaagga 600
   ggaggagtga ggatcgtggg aggacaggag tgcaaggacg gagaatgtcc atggcaggcc 660
   ctgctgatta acgaggaaaa tgagggattc tgcggaggca ctatcctgag cgagttctac 720
   attctgaccg cagcccactg tctgtatcag gctaagcgat tcaaagtgcg ggtcggcgac 780
   agaaacaccg agcaggagga agggggagaa gcagtgcacg aggtcgaagt ggtcatcaag 840
   cataatcgct tcactaaaga gacctacgac tttgatatcg ctgtgctgcg cctgaagaca 900
   cctattactt tccgaatgaa cgtcgcccct gcttgcctgc cagagcgaga ttgggccgaa 960
   agcaccctga tgacacagaa aactggcatc gtgagcgggt ttggacggac acatgagaag 1020
   ggcaggcagt ccactcgcct gaaaatgctg gaagtgccct acgtcgaccg gaactcttgt 1080
   aagctgagta gcagcttcat cattacccag aatatgtttt gcgccgggta tgacacaaag 1140
   caggaggatg cttgtcaggg agacagtggc gggcctcacg tgactaggtt caaagatact 1200
   tattttgtga ccggcatcgt cagctgggga gagggatgcg cacgcaaggg gaaatacgga 1260
   atctatacca aggtgacagc ctttctgaaa tggattgacc gatctatgaa gacccggggg 1320
   ctgccaaagg caaaaagtca tgcccccgag gtcattacca gttcccctct gaaagaagac 1380
   caggtggacc caaggctgat tgacggcaag 1410
<210> 142
   <211> 1410
   <212> DNA
   <213> Artificial Sequence
<220>
   <223> acide nucléique codant SEQ ID NO:114
<400> 142
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcgataac aatctgacta gggacttcct ggccgagggc 600
   ctgaccccta ggatcgtggg aggacaggag tgcaaggacg gagaatgtcc atggcaggcc 660
   ctgctgatta acgaggaaaa tgagggattc tgcggaggca ctatcctgag cgagttctac 720
   attctgaccg cagcccactg tctgtatcag gctaagcgat tcaaagtgcg ggtcggcgac 780
   agaaacaccg agcaggagga agggggagaa gcagtgcacg aggtcgaagt ggtcatcaag 840
   cataatcgct tcactaaaga gacctacgac tttgatatcg ctgtgctgcg cctgaagaca 900
   cctattactt tccgaatgaa cgtcgcccct gcttgcctgc cagagcgaga ttgggccgaa 960
   agcaccctga tgacacagaa aactggcatc gtgagcgggt ttggacggac acatgagaag 1020
   ggcaggcagt ccactcgcct gaaaatgctg gaagtgccct acgtcgaccg gaactcttgt 1080
   aagctgagta gcagcttcat cattacccag aatatgtttt gcgccgggta tgacacaaag 1140
   caggaggatg cttgtcaggg agacagtggc gggcctcacg tgactaggtt caaagatact 1200
   tattttgtga ccggcatcgt cagctgggga gagggatgcg cacgcaaggg gaaatacgga 1260
   atctatacca aggtgacagc ctttctgaaa tggattgacc gatctatgaa gacccggggg 1320
   ctgccaaagg caaaaagtca tgcccccgag gtcattacca gttcccctct gaaagaagac 1380
   caggtggacc caaggctgat tgacggcaag 1410
<210> 143
   <211> 1410
   <212> DNA
   <213> Artificial Sequence
<220>
   <223> acide nucléique codant SEQ ID NO:115
<400> 143
   gccaactctt tcctggagga aatgaagaaa ggccacctgg agcgggaatg catggaggaa       60
   acctgtagtt acgaggaagc cagagaggtg ttcgaagact cagataagac aaacgagttt      120
   tggaataagt acaaagacgg cgatcagtgc gaaactagcc catgtcagaa ccaggggaag      180
   tgcaaagatg gactgggcga gtacacctgc acatgtctgg agggattcga aggcaagaat      240
   tgcgaactgt ttaccagaaa gctgtgctcc ctggataacg gcgactgcga tcagttttgt      300
   catgaggaac agaattccgt ggtctgctct tgtgccaggg gatacacact ggctgacaat      360
   ggcaaggcat gcatccccac cggcccctat ccttgtggga agcagacact ggagaggcgc      420
   aaaaggtcag tggctcaggc aactagctcc tctggcgagg cccccgatag cattacctgg      480
   aaaccttatg acgccgctga cctggacccc acagagaacc cctttgacct gctggacttc      540
   aaccagacac agcctgaaag aggcgataac aatctgacta ggaaggccac caacgccacc      600
   ctgtccccta ggatcgtggg aggacaggag tgcaaggacg gagaatgtcc atggcaggcc      660
   ctgctgatta acgaggaaaa tgagggattc tgcggaggca ctatcctgag cgagttctac      720
   attctgaccg cagcccactg tctgtatcag gctaagcgat tcaaagtgcg ggtcggcgac      780
   agaaacaccg agcaggagga agggggagaa gcagtgcacg aggtcgaagt ggtcatcaag      840
   cataatcgct tcactaaaga gacctacgac tttgatatcg ctgtgctgcg cctgaagaca      900
   cctattactt tccgaatgaa cgtcgcccct gcttgcctgc cagagcgaga ttgggccgaa      960
   agcaccctga tgacacagaa aactggcatc gtgagcgggt ttggacggac acatgagaag     1020
   ggcaggcagt ccactcgcct gaaaatgctg gaagtgccct acgtcgaccg gaactcttgt     1080
   aagctgagta gcagcttcat cattacccag aatatgtttt gcgccgggta tgacacaaag     1140
   caggaggatg cttgtcaggg agacagtggc gggcctcacg tgactaggtt caaagatact     1200
   tattttgtga ccggcatcgt cagctgggga gagggatgcg cacgcaaggg gaaatacgga     1260
   atctatacca aggtgacagc ctttctgaaa tggattgacc gatctatgaa gacccggggg     1320
   ctgccaaagg caaaaagtca tgcccccgag gtcattacca gttcccctct gaaagaagac     1380
   caggtggacc caaggctgat tgacggcaag                                      1410
<210> 144
   <211> 1410
   <212> DNA
   <213> Artificial Sequence
<220>
   <223> acide nucléique codant SEQ ID NO:116
<400> 144
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagctcc tctggcgagg cccccgatag cattacctgg 480
   aaaccttatg acgccgctga cctggacccc acagagaacc cctttgacct gctggacttc 540
   aaccagacac agcctgaaag aggcgataac aatctgacta ggaaggccac ccaggccacc 600
   ctgagcccta ggatcgtggg aggacaggag tgcaaggacg gagaatgtcc atggcaggcc 660
   ctgctgatta acgaggaaaa tgagggattc tgcggaggca ctatcctgag cgagttctac 720
   attctgaccg cagcccactg tctgtatcag gctaagcgat tcaaagtgcg ggtcggcgac 780
   agaaacaccg agcaggagga agggggagaa gcagtgcacg aggtcgaagt ggtcatcaag 840
   cataatcgct tcactaaaga gacctacgac tttgatatcg ctgtgctgcg cctgaagaca 900
   cctattactt tccgaatgaa cgtcgcccct gcttgcctgc cagagcgaga ttgggccgaa 960
   agcaccctga tgacacagaa aactggcatc gtgagcgggt ttggacggac acatgagaag 1020
   ggcaggcagt ccactcgcct gaaaatgctg gaagtgccct acgtcgaccg gaactcttgt 1080
   aagctgagta gcagcttcat cattacccag aatatgtttt gcgccgggta tgacacaaag 1140
   caggaggatg cttgtcaggg agacagtggc gggcctcacg tgactaggtt caaagatact 1200
   tattttgtga ccggcatcgt cagctgggga gagggatgcg cacgcaaggg gaaatacgga 1260
   atctatacca aggtgacagc ctttctgaaa tggattgacc gatctatgaa gacccggggg 1320
   ctgccaaagg caaaaagtca tgcccccgag gtcattacca gttcccctct gaaagaagac 1380
   caggtggacc caaggctgat tgacggcaag 1410
<210> 145
   <211> 1398
   <212> DNA
   <213> Artificial Sequence
<220>
   <223> acide nucléique codant SEQ ID NO:117
<400> 145
   gccaactctt tcctggagga aatgaagaaa ggccacctgg agcgggaatg catggaggaa        60
   acctgtagtt acgaggaagc cagagaggtg ttcgaagact cagataagac aaacgagttt       120
   tggaataagt acaaagacgg cgatcagtgc gaaactagcc catgtcagaa ccaggggaag       180
   tgcaaagatg gactgggcga gtacacctgc acatgtctgg agggattcga aggcaagaat       240
   tgcgaactgt ttaccagaaa gctgtgctcc ctggataacg gcgactgcga tcagttttgt       300
   catgaggaac agaattccgt ggtctgctct tgtgccaggg gatacacact ggctgacaat       360
   ggcaaggcat gcatccccac cggcccctat ccttgtggga agcagacact ggagaggcgc       420
   aaaaggtcag tggctcaggc aactagctcc tctggcgagg cccccgatag cattacctgg       480
   aaaccttatg acgccgctga cctggacccc acagagaacc cctttgacct gctggacttc       540
   aaccagacac agcctgaaag aggcgataac aatctgacta ggaccagcaa gctgaccagg       600
   atcgtgggag gacaggagtg caaggacgga gaatgtccat ggcaggccct gctgattaac       660
   gaggaaaatg agggattctg cggaggcact atcctgagcg agttctacat tctgaccgca       720
   gcccactgtc tgtatcaggc taagcgattc aaagtgcggg tcggcgacag aaacaccgag       780
   caggaggaag ggggagaagc agtgcacgag gtcgaagtgg tcatcaagca taatcgcttc       840
   actaaagaga cctacgactt tgatatcgct gtgctgcgcc tgaagacacc tattactttc       900
   cgaatgaacg tcgcccctgc ttgcctgcca gagcgagatt gggccgaaag caccctgatg       960
   acacagaaaa ctggcatcgt gagcgggttt ggacggacac atgagaaggg caggcagtcc      1020
   actcgcctga aaatgctgga agtgccctac gtcgaccgga actcttgtaa gctgagtagc      1080
   agcttcatca ttacccagaa tatgttttgc gccgggtatg acacaaagca ggaggatgct      1140
   tgtcagggag acagtggcgg gcctcacgtg actaggttca aagatactta ttttgtgacc      1200
   ggcatcgtca gctggggaga gggatgcgca cgcaagggga aatacggaat ctataccaag      1260
   gtgacagcct ttctgaaatg gattgaccga tctatgaaga cccgggggct gccaaaggca      1320
   aaaagtcatg cccccgaggt cattaccagt tcccctctga aagaagacca ggtggaccca      1380
   aggctgattg acggcaag                                                    1398
<210> 146
   <211> 1398
   <212> DNA
   <213> Artificial Sequence
<220>
   <223> acide nucléique codant SEQ ID NO:118
<400> 146
<210> 148
   <211> 1398
   <212> DNA
   <213> Artificial Sequence
<220>
   <223> acide nucléique codant SEQ ID NO:120
<400> 148
   gccaactctt tcctggagga aatgaagaaa ggccacctgg agcgggaatg catggaggaa      60
   acctgtagtt acgaggaagc cagagaggtg ttcgaagact cagataagac aaacgagttt     120
   tggaataagt acaaagacgg cgatcagtgc gaaactagcc catgtcagaa ccaggggaag     180
   tgcaaagatg gactgggcga gtacacctgc acatgtctgg agggattcga aggcaagaat     240
   tgcgaactgt ttaccagaaa gctgtgctcc ctggataacg gcgactgcga tcagttttgt     300
   catgaggaac agaattccgt ggtctgctct tgtgccaggg gatacacact ggctgacaat     360
   ggcaaggcat gcatccccac cggcccctat ccttgtggga agcagacact ggagaggcgc     420
   aaaaggtcag tggctcaggc aactagctcc tctggcgagg cccccgatag cattacctgg     480
   aaaccttatg acgccgctga cctggacccc acagagaacc cctttgacct gctggacttc     540
   aaccagacac agcctgaaag aggcgataac aatctgacta ggcctcccag cctgaccagg     600
   atcgtgggag gacaggagtg caaggacgga gaatgtccat ggcaggccct gctgattaac     660
   gaggaaaatg agggattctg cggaggcact atcctgagcg agttctacat tctgaccgca     720
   gcccactgtc tgtatcaggc taagcgattc aaagtgcggg tcggcgacag aaacaccgag     780
   caggaggaag ggggagaagc agtgcacgag gtcgaagtgg tcatcaagca taatcgcttc      840
   actaaagaga cctacgactt tgatatcgct gtgctgcgcc tgaagacacc tattactttc     900
   cgaatgaacg tcgcccctgc ttgcctgcca gagcgagatt gggccgaaag caccctgatg     960
   acacagaaaa ctggcatcgt gagcgggttt ggacggacac atgagaaggg caggcagtcc    1020
   actcgcctga aaatgctgga agtgccctac gtcgaccgga actcttgtaa gctgagtagc    1080
   agcttcatca ttacccagaa tatgttttgc gccgggtatg acacaaagca ggaggatgct    1140
   tgtcagggag acagtggcgg gcctcacgtg actaggttca aagatactta ttttgtgacc    1200
   ggcatcgtca gctggggaga gggatgcgca cgcaagggga aatacggaat ctataccaag    1260
   gtgacagcct ttctgaaatg gattgaccga tctatgaaga cccgggggct gccaaaggca    1320
   aaaagtcatg cccccgaggt cattaccagt tcccctctga aagaagacca ggtggaccca    1380
   aggctgattg acggcaag                                                  1398
<210> 149
   <211> 1398
   <212> DNA
   <213> Artificial Sequence
<220>
   <223> acide nucléique codant SEQ ID NO:121
<400> 149
<210> 150
   <211> 1380
   <212> DNA
   <213> Artificial Sequence
<220>
   <223> acide nucléique codant SEQ ID NO:122
<400> 150
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctgatag cattacctgg aaaccttatg acgccgctga cctggacccc 480
   acagagaacc cctttgacct gctggacttc aaccagacac agcctgaaag aggcgataac 540
   aatctgacta gggacttcct ggcagaagga ggaggagtga ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 151
   <211> 1380
   <212> DNA
   <213> Artificial Sequence
<220>
   <223> acide nucléique codant SEQ ID NO:123
<400> 151
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctgatag cattacctgg aaaccttatg acgccgctga cctggacccc 480
   acagagaacc cctttgacct gctggacttc aaccagacac agcctgaaag aggcgataac 540
   aatctgacta gggacttcct ggccgagggc ctgaccccta ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 152
   <211> 1380
   <212> DNA
   <213> Artificial Sequence
<220>
   <223> acide nucléique codant SEQ ID NO:124
<400> 152
   gccaactctt tcctggagga aatgaagaaa ggccacctgg agcgggaatg catggaggaa      60
   acctgtagtt acgaggaagc cagagaggtg ttcgaagact cagataagac aaacgagttt     120
   tggaataagt acaaagacgg cgatcagtgc gaaactagcc catgtcagaa ccaggggaag     180
   tgcaaagatg gactgggcga gtacacctgc acatgtctgg agggattcga aggcaagaat     240
   tgcgaactgt ttaccagaaa gctgtgctcc ctggataacg gcgactgcga tcagttttgt     300
   catgaggaac agaattccgt ggtctgctct tgtgccaggg gatacacact ggctgacaat     360
   ggcaaggcat gcatccccac cggcccctat ccttgtggga agcagacact ggagaggcgc     420
   aaaaggtcag tggctgatag cattacctgg aaaccttatg acgccgctga cctggacccc     480
   acagagaacc cctttgacct gctggacttc aaccagacac agcctgaaag aggcgataac     540
   aatctgacta ggaaggccac caacgccacc ctgtccccta ggatcgtggg aggacaggag     600
   tgcaaggacg gagaatgtcc atggcaggcc ctgctgatta acgaggaaaa tgagggattc     660
   tgcggaggca ctatcctgag cgagttctac attctgaccg cagcccactg tctgtatcag     720
   gctaagcgat tcaaagtgcg ggtcggcgac agaaacaccg agcaggagga agggggagaa      780
   gcagtgcacg aggtcgaagt ggtcatcaag cataatcgct tcactaaaga gacctacgac     840
   tttgatatcg ctgtgctgcg cctgaagaca cctattactt tccgaatgaa cgtcgcccct     900
   gcttgcctgc cagagcgaga ttgggccgaa agcaccctga tgacacagaa aactggcatc     960
   gtgagcgggt ttggacggac acatgagaag ggcaggcagt ccactcgcct gaaaatgctg    1020
   gaagtgccct acgtcgaccg gaactcttgt aagctgagta gcagcttcat cattacccag    1080
   aatatgtttt gcgccgggta tgacacaaag caggaggatg cttgtcaggg agacagtggc    1140
   gggcctcacg tgactaggtt caaagatact tattttgtga ccggcatcgt cagctgggga    1200
   gagggatgcg cacgcaaggg gaaatacgga atctatacca aggtgacagc ctttctgaaa    1260
   tggattgacc gatctatgaa gacccggggg ctgccaaagg caaaaagtca tgcccccgag    1320
   gtcattacca gttcccctct gaaagaagac caggtggacc caaggctgat tgacggcaag    1380
<210> 153
   <211> 1380
   <212> DNA
   <213> Artificial Sequence
<220>
   <223> acide nucléique codant SEQ ID NO:125
<400> 153
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctgatag cattacctgg aaaccttatg acgccgctga cctggacccc 480
   acagagaacc cctttgacct gctggacttc aaccagacac agcctgaaag aggcgataac 540
   aatctgacta ggaaggccac ccaggccacc ctgagcccta ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 154
   <211> 1380
   <212> DNA
   <213> Artificial Sequence
<220>
   <223> acide nucléique codant SEQ ID NO:126
<400> 154
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagcgat agcattacct ggaaacctta tgacgccgct 480
   gacctggacc ccacagagaa cccctttgac ctgctggact tcaaccagac acagcctgaa 540
   agaggcgata acaatctgac taggaccagc aagctgacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 155
   <211> 1380
   <212> DNA
   <213> Artificial Sequence
<220>
   <223> acide nucléique codant SEQ ID NO:127
<400> 155
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagcgat agcattacct ggaaacctta tgacgccgct 480
   gacctggacc ccacagagaa cccctttgac ctgctggact tcaaccagac acagcctgaa 540
   agaggcgata acaatctgac taggttcaac gacttcacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 156
   <211> 1380
   <212> DNA
   <213> Artificial Sequence
<220>
   <223> acide nucléique codant SEQ ID NO:128
<400> 156
   gccaactctt tcctggagga aatgaagaaa ggccacctgg agcgggaatg catggaggaa 60
   acctgtagtt acgaggaagc cagagaggtg ttcgaagact cagataagac aaacgagttt 120
   tggaataagt acaaagacgg cgatcagtgc gaaactagcc catgtcagaa ccaggggaag 180
   tgcaaagatg gactgggcga gtacacctgc acatgtctgg agggattcga aggcaagaat 240
   tgcgaactgt ttaccagaaa gctgtgctcc ctggataacg gcgactgcga tcagttttgt 300
   catgaggaac agaattccgt ggtctgctct tgtgccaggg gatacacact ggctgacaat 360
   ggcaaggcat gcatccccac cggcccctat ccttgtggga agcagacact ggagaggcgc 420
   aaaaggtcag tggctcaggc aactagcgat agcattacct ggaaacctta tgacgccgct 480
   gacctggacc ccacagagaa cccctttgac ctgctggact tcaaccagac acagcctgaa 540
   agaggcgata acaatctgac taggctgagc agcatgacca ggatcgtggg aggacaggag 600
   tgcaaggacg gagaatgtcc atggcaggcc ctgctgatta acgaggaaaa tgagggattc 660
   tgcggaggca ctatcctgag cgagttctac attctgaccg cagcccactg tctgtatcag 720
   gctaagcgat tcaaagtgcg ggtcggcgac agaaacaccg agcaggagga agggggagaa 780
   gcagtgcacg aggtcgaagt ggtcatcaag cataatcgct tcactaaaga gacctacgac 840
   tttgatatcg ctgtgctgcg cctgaagaca cctattactt tccgaatgaa cgtcgcccct 900
   gcttgcctgc cagagcgaga ttgggccgaa agcaccctga tgacacagaa aactggcatc 960
   gtgagcgggt ttggacggac acatgagaag ggcaggcagt ccactcgcct gaaaatgctg 1020
   gaagtgccct acgtcgaccg gaactcttgt aagctgagta gcagcttcat cattacccag 1080
   aatatgtttt gcgccgggta tgacacaaag caggaggatg cttgtcaggg agacagtggc 1140
   gggcctcacg tgactaggtt caaagatact tattttgtga ccggcatcgt cagctgggga 1200
   gagggatgcg cacgcaaggg gaaatacgga atctatacca aggtgacagc ctttctgaaa 1260
   tggattgacc gatctatgaa gacccggggg ctgccaaagg caaaaagtca tgcccccgag 1320
   gtcattacca gttcccctct gaaagaagac caggtggacc caaggctgat tgacggcaag 1380
<210> 157
   <211> 1380
   <212> DNA
   <213> Artificial Sequence
<220>
   <223> acide nucléique codant SEQ ID NO:129
<400> 157
   gccaactctt tcctggagga aatgaagaaa ggccacctgg agcgggaatg catggaggaa      60
   acctgtagtt acgaggaagc cagagaggtg ttcgaagact cagataagac aaacgagttt     120
   tggaataagt acaaagacgg cgatcagtgc gaaactagcc catgtcagaa ccaggggaag     180
   tgcaaagatg gactgggcga gtacacctgc acatgtctgg agggattcga aggcaagaat     240
   tgcgaactgt ttaccagaaa gctgtgctcc ctggataacg gcgactgcga tcagttttgt     300
   catgaggaac agaattccgt ggtctgctct tgtgccaggg gatacacact ggctgacaat     360
   ggcaaggcat gcatccccac cggcccctat ccttgtggga agcagacact ggagaggcgc     420
   aaaaggtcag tggctcaggc aactagcgat agcattacct ggaaacctta tgacgccgct     480
   gacctggacc ccacagagaa cccctttgac ctgctggact tcaaccagac acagcctgaa     540
   agaggcgata acaatctgac taggcctccc agcctgacca ggatcgtggg aggacaggag     600
   tgcaaggacg gagaatgtcc atggcaggcc ctgctgatta acgaggaaaa tgagggattc     660
   tgcggaggca ctatcctgag cgagttctac attctgaccg cagcccactg tctgtatcag     720
   gctaagcgat tcaaagtgcg ggtcggcgac agaaacaccg agcaggagga agggggagaa     780
   gcagtgcacg aggtcgaagt ggtcatcaag cataatcgct tcactaaaga gacctacgac     840
   tttgatatcg ctgtgctgcg cctgaagaca cctattactt tccgaatgaa cgtcgcccct     900
   gcttgcctgc cagagcgaga ttgggccgaa agcaccctga tgacacagaa aactggcatc     960
   gtgagcgggt ttggacggac acatgagaag ggcaggcagt ccactcgcct gaaaatgctg    1020
   gaagtgccct acgtcgaccg gaactcttgt aagctgagta gcagcttcat cattacccag    1080
   aatatgtttt gcgccgggta tgacacaaag caggaggatg cttgtcaggg agacagtggc    1140
   gggcctcacg tgactaggtt caaagatact tattttgtga ccggcatcgt cagctgggga    1200
   gagggatgcg cacgcaaggg gaaatacgga atctatacca aggtgacagc ctttctgaaa    1260
   tggattgacc gatctatgaa gacccggggg ctgccaaagg caaaaagtca tgcccccgag    1320
   gtcattacca gttcccctct gaaagaagac caggtggacc caaggctgat tgacggcaag    1380
<210> 158
   <211> 1380
   <212> DNA
   <213> Artificial Sequence
<220>
   <223> acide nucléique codant SEQ ID NO:130
<400> 158
   gccaactctt tcctggagga aatgaagaaa ggccacctgg agcgggaatg catggaggaa    60
   acctgtagtt acgaggaagc cagagaggtg ttcgaagact cagataagac aaacgagttt   120
   tggaataagt acaaagacgg cgatcagtgc gaaactagcc catgtcagaa ccaggggaag   180
   tgcaaagatg gactgggcga gtacacctgc acatgtctgg agggattcga aggcaagaat   240
   tgcgaactgt ttaccagaaa gctgtgctcc ctggataacg gcgactgcga tcagttttgt   300
   catgaggaac agaattccgt ggtctgctct tgtgccaggg gatacacact ggctgacaat   360
   ggcaaggcat gcatccccac cggcccctat ccttgtggga agcagacact ggagaggcgc   420
   aaaaggtcag tggctcaggc aactagcgat agcattacct ggaaacctta tgacgccgct   480
   gacctggacc ccacagagaa cccctttgac ctgctggact tcaaccagac acagcctgaa   540
   agaggcgata acaatctgac taggctgtcc tgcggccaga ggatcgtggg aggacaggag   600
   tgcaaggacg gagaatgtcc atggcaggcc ctgctgatta acgaggaaaa tgagggattc   660
   tgcggaggca ctatcctgag cgagttctac attctgaccg cagcccactg tctgtatcag   720
   gctaagcgat tcaaagtgcg ggtcggcgac agaaacaccg agcaggagga agggggagaa   780
   gcagtgcacg aggtcgaagt ggtcatcaag cataatcgct tcactaaaga gacctacgac   840
   tttgatatcg ctgtgctgcg cctgaagaca cctattactt tccgaatgaa cgtcgcccct   900
   gcttgcctgc cagagcgaga ttgggccgaa agcaccctga tgacacagaa aactggcatc   960
   gtgagcgggt ttggacggac acatgagaag ggcaggcagt ccactcgcct gaaaatgctg  1020
   gaagtgccct acgtcgaccg gaactcttgt aagctgagta gcagcttcat cattacccag  1080
   aatatgtttt gcgccgggta tgacacaaag caggaggatg cttgtcaggg agacagtggc  1140
   gggcctcacg tgactaggtt caaagatact tattttgtga ccggcatcgt cagctgggga  1200
   gagggatgcg cacgcaaggg gaaatacgga atctatacca aggtgacagc ctttctgaaa  1260
   tggattgacc gatctatgaa gacccggggg ctgccaaagg caaaaagtca tgcccccgag  1320
   gtcattacca gttcccctct gaaagaagac caggtggacc caaggctgat tgacggcaag  1380

## Description

La présente invention concerne des mutants du facteur X, et leur utilisation pour le traitement des troubles de la coagulation sanguine.

Le facteur X est une protéine présente dans le sang. Cette protéine joue un rôle important dans la cascade de la coagulation. La coagulation du sang est un processus complexe qui permet d'éviter l'écoulement du sang par les vaisseaux endommagés. Aussitôt qu'un vaisseau est brisé, les éléments responsables de la coagulation interagissent entre eux pour former un bouchon, le clou plaquettaire, à l'endroit où le vaisseau est brisé. Les facteurs de la coagulation sont requis pour tenir le clou plaquettaire en place et stabiliser le caillot.

La formation d'un caillot normal s'effectue en quatre étapes :
**Étape 1** Le vaisseau sanguin est endommagé.
**Étape 2** Le vaisseau sanguin se contracte de façon à restreindre l'apport de sang vers la zone lésée.
**Étape 3** Les plaquettes adhèrent à l'espace sous-endothélial exposé lors de la lésion du vaisseau ainsi qu'aux parois des vaisseaux sanguins stimulées. Les plaquettes s'étalent, c'est ce que l'on appelle « l'adhésion plaquettaire ». Ces plaquettes étalées libèrent des substances qui activent d'autres plaquettes avoisinantes de sorte qu'elles s'agglomèrent au siège de la lésion afin de former le clou plaquettaire. C'est ce que l'on appelle « l'agrégation plaquettaire ».
**Étape 4** La surface des plaquettes activées constitue ainsi une surface sur laquelle la coagulation du sang peut s'effectuer. Les protéines de la coagulation qui circulent dans le sang (dont le facteur X) sont activées à la surface des plaquettes et forment un caillot de fibrine.

Ces protéines de la coagulation (c'est-à-dire, les facteurs I, II, V, VIII, IX, X, XI, XII et XIII, ainsi que le facteur de Von Willebrand) fonctionnent en une réaction en chaîne, *i.e.* la cascade de la coagulation.

Le facteur X sous forme activée (Xa) intervient plus particulièrement dans l'activation de la prothrombine (facteur II) en thrombine (facteur IIa), notamment lorsqu'il est complexé avec le co-facteur V activé pour former le complexe prothrombinase. Ce facteur est un élément essentiel dans la cascade de la coagulation.

Lorsque ce facteur fait défaut, des saignements se manifestent, comme une épistaxis (saignements de nez), une hémarthrose (épanchement de sang dans une cavité articulaire) ou des saignements gastro-intestinaux. La déficience en facteur X est extrêmement rare. Sa transmission est autosomique récessive, et sa prévalence est de 1/1 000 000.

WO2001010896 (BAXTER AG) divulgue des mutants du facteur X, et leur utilisation pour le traitement des troubles de la coagulation sanguine, les mutants ayant une formule générale de subsitutions entre les résidus 227-234 situés en amont du clivage du peptide d'activation, en particulier ils comprennent TSKLTR ou FNDFTR à la position 229-234. L'activation du FX intervient :
- soit très précocement lors de l'étape d'initiation de la cascade de la coagulation par le complexe facteur VIIa/facteur tissulaire, dans une réaction peu efficace qui conduit à la formation de trace de thrombine ;
- soit lors de l'étape d'amplification de la cascade de la coagulation issue d'un rétrocontrôle positif effectué par les traces de thrombine conduisant à l'activation des facteurs VIII et IX.

Ces deux derniers facteurs sont manquants chez les hémophiles A et B, causant ainsi un trouble hémorragique qui peut être fatal sans traitement. L'absence de ces facteurs ne permet pas de générer des quantités suffisantes de facteur X activé, pour juguler l'hémorragie.

Ainsi il existe un besoin pour un facteur X modifié pouvant être activé par la thrombine, qui permettrait d'avoir une coagulation efficace en absence de facteur VIII et/ou de facteur IX, par l'utilisation directe des traces de thrombine générées lors de l'initiation de la coagulation.

Les inventeurs ont identifié des mutants spécifiques du facteur X (appelés également facteurs X variants), qui sont efficacement activés par la thrombine, permettant ainsi de restaurer la coagulation en absence de facteur VIII, de facteur IX et même de facteur X. En effet, comme démontré en exemples, ces mutants du facteur X peuvent être activés par la thrombine, et permettent une coagulation efficace, et ce, même en absence de facteur VIII et/ou de facteur IX et/ou de facteur X endogène.

Les sites de clivage du peptide d'activation générés dans ces facteurs X variants peuvent aussi être la cible d'autres protéases de la coagulation, telles que le facteur VIIa, le facteur IXa, le facteur Xa, le facteur XIa, le facteur XIIa ou la kallicréine.
Par ailleurs, une modification du peptide d'activation du facteur X peut conduire à une modification additionnelle de ses propriétés pharmacologiques, différentes de la seule reconnaissance par la thrombine. Cette modification peut conférer au facteur X variant une amélioration de l'activité spécifique, de la stabilité, ou de la résistance aux protéases, ou encore une augmentation de la pharmacocinétique. En outre, la présence de glycosylations et phosphorylations additionnelles, ou au contraire l'absence de ces modifications par rapport à la molécule de type sauvage, pourront être causées par les modifications introduites dans le peptide d'activation.

La présente invention concerne donc une protéine comprenant une séquence mutée de SEQ ID NO :1, ladite séquence mutée de SEQ ID NO :1 comprenant au moins une mutation A, A', B, C ou C', dans laquelle :
la mutation A consiste en la substitution des acides aminés 43 à 52 de la séquence SEQ ID NO :1 par une séquence choisie parmi DFLAEGLTPR, KATN*ATLSPR et KATXATLSPR,
la mutation A' consiste en la substitution des acides aminés 47 à 52 de la séquence SEQ ID NO :1 par une séquence choisie parmi TSKLTR, FNDFTR, LSSMTR, PPSLTR et LSCGQR,
la mutation B consiste en l'insertion d'une séquence choisie parmi DFLAEGLTPR, KATN*ATLSPR, KATXATLSPR, TSKLTR, FNDFTR, LSSMTR, PPSLTR et LSCGQR, entre les acides aminés 52 et 53 de la séquence SEQ ID NO :1,
la mutation C consiste en l'insertion d'une séquence choisie parmi DFLAEGLTPR, KATN*ATLSPR et KATXATLSPR, entre les acides aminés 52 et 53 de la séquence SEQ ID NO :1, et en la délétion des acides aminés 4 à 13 de la séquence SEQ ID NO :1, la mutation C' consiste en l'insertion d'une séquence choisie parmi TSKLTR, FNDFTR, LSSMTR, PPSLTR et LSCGQR, entre les acides aminés 52 et 53 de la séquence SEQ ID NO :1, et en la délétion des acides aminés 4 à 9 de la séquence SEQ ID NO :1,
où N* est une asparagine éventuellement glycosylée.

Un autre objet de l'invention est un polynucléotide codant pour ladite protéine.
Un autre objet de l'invention est un vecteur d'expression comprenant ledit polynucléotide.
Un autre objet de l'invention est une cellule hôte comprenant ledit vecteur d'expression ou ledit polynucléotide.
Un autre objet de l'invention est l'utilisation de ladite protéine comme médicament. En particulier, ladite protéine peut être utilisée pour le traitement des troubles de la coagulation sanguine, notamment les troubles hémorragiques, tels que les hémophilies A, B et C (déficit en facteur XI), les déficits en facteur X voire des besoins en coagulation d'urgence pour se substituer au Facteur VIIa. Lorsqu'une réponse procoagulante puissante et rapide est requise, ladite protéine peut être utilisée en combinaison avec d'autres molécules hémostatiques, telles que le facteur VIIa et/ou le fibrinogène, voire en association avec des composés procoagulants (transfusion de plaquettes, mélange procoagulant comme FEIBA, Kaskadil, Kanokad etc), qui pourront renforcer l'efficacité du traitement.

Tel qu'il est utilisé ici, les termes «protéine» et «polypeptide» sont utilisés ici de manière interchangeable et se réfèrent à une séquence d'acides aminés ayant plus de 100 acides aminés. Tel qu'utilisé ici, le terme "protéine" comprend des séquences d'acides aminés ayant entre 100 et 1000 acides aminés, de préférence entre 120 et 500 acides aminés.

La présente invention se rapporte à une protéine comprenant une séquence mutée de SEQ ID NO :1, ladite séquence mutée de SEQ ID NO :1 comprenant au moins une mutation A, A', B, C ou C', dans laquelle :
la mutation A consiste en la substitution des acides aminés 43 à 52 de la séquence SEQ ID NO :1 par une séquence choisie parmi DFLAEGLTPR, KATN*ATLSPR et KATXATLSPR,
la mutation A' consiste en la substitution des acides aminés 47 à 52 de la séquence SEQ ID NO :1 par une séquence choisie parmi TSKLTR, FNDFTR, LSSMTR, PPSLTR et LSCGQR,
la mutation B consiste en l'insertion d'une séquence choisie parmi DFLAEGLTPR, KATN*ATLSPR, KATXATLSPR, TSKLTR, FNDFTR, LSSMTR, PPSLTR et LSCGQR, entre les acides aminés 52 et 53 de la séquence SEQ ID NO :1,
la mutation C consiste en l'insertion d'une séquence choisie parmi DFLAEGLTPR, KATN*ATLSPR et KATXATLSPR, entre les acides aminés 52 et 53 de la séquence SEQ ID NO :1, et en la délétion des acides aminés 4 à 13 de la séquence SEQ ID NO :1, la mutation C' consiste en l'insertion d'une séquence choisie parmi TSKLTR, FNDFTR, LSSMTR, PPSLTR et LSCGQR, entre les acides aminés 52 et 53 de la séquence SEQ ID NO :1, et en la délétion des acides aminés 4 à 9 de la séquence SEQ ID NO :1,
où N* est une asparagine éventuellement glycosylée.

De préférence, ladite protéine comprend, de préférence consiste en, la séquence SEQ ID NO :7, avec au moins une mutation A, A', B, C ou C' telle que décrite ci-avant.
La séquence SEQ ID NO :7 (500 acides aminés) comprend toute la séquence SEQ ID NO :1 (306 acides aminés). Plus particulièrement, la séquence SEQ ID NO :7 comprend, dans le sens N- à C-terminal, un peptide signal et un propeptide (40 acides aminés au total), la séquence SEQ ID NO :5, la séquence SEQ ID NO :1, puis un tag (des acides aminés en position 489 à 500, soit une longueur de 12 acides aminés), i.e. le tag HPC4. La séquence SEQ ID NO : 103 correspond à la séquence SEQ ID NO :7 sans peptide signal et sans propeptide.

Ladite protéine selon l'invention est un facteur X muté qui est efficace dans le traitement de troubles de la coagulation.

Le facteur X, appelé également facteur de Stuart-Prower, est codé par le gène F10 et se réfère à la sérine protéase EC3.4.21.6. Le facteur X est composé d'une chaîne lourde, de 306 acides aminés, et d'une chaîne légère, de 139 acides aminés.
Le facteur X est une protéine de 488 acides aminés, constitué d'un peptide signal, d'un propeptide, et des chaînes légère et lourde.

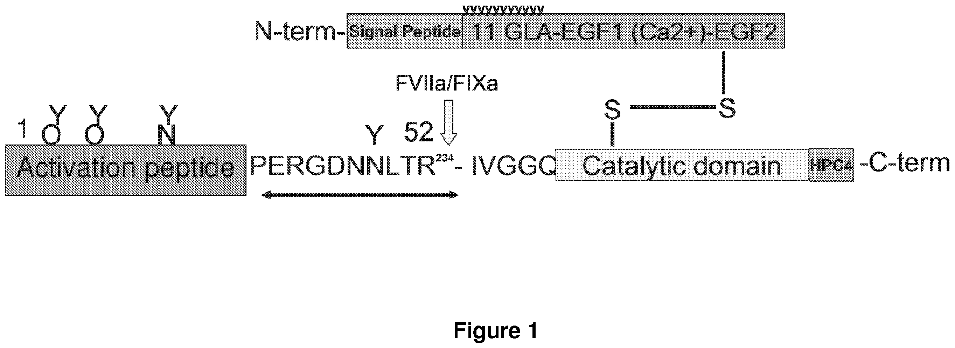

Le facteur X humain peut être trouvé dans UniProtKB sous le numéro d'accession P00742. Sa structure native est illustrée en figure 1.
La protéine est traduite sous forme de prépropeptide. Après clivage du peptide signal, le propeptide est finalement coupé, résultant en une chaîne légère et une chaîne lourde (respectivement de 142 et 306 acides aminés) (zymogène). Suite au déclenchement de la coagulation, la chaîne lourde est finalement activée par clivage du peptide d'activation, pour ne contenir que 254 acides aminés aminés (les 52 premiers acides aminés sont clivés lors du traitement): c'est la chaîne lourde du facteur Xa (SEQ ID NO :6).
Le prépropeptide de facteur X humain correspond à SEQ ID NO: 4. La chaîne lourde correspond à SEQ ID NO: 1, et la chaîne légère correspond à SEQ ID NO: 5. le peptide d'activation de la chaîne lourde correspond à SEQ ID NO :3, et comprend 52 acides aminés.
SEQ ID NO: 2 est identique aux acides aminés 1 à 182 de SEQ ID NO: 4.
SEQ ID NO: 1 est identique aux acides aminés 183 à 488 de SEQ ID NO: 4.
La chaîne lourde du facteur Xa (SEQ ID NO :6) correspond à la SEQ ID NO :1, dans laquelle le peptide SEQ ID NO :3 a été clivé.

Les protéines selon l'invention comprennent des protéines de facteur X mutées, sous forme de zymogène, comprenant, en fonction des constructions, un peptide d'activation :
- modifié par substitution d'un nombre identique de résidus au même site compris au plus large entre les résidus 42 et 52 ; ou
- ayant reçu une insertion de résidus entre les résidus 52 et 53 ; ou
- ayant reçu une insertion de résidus au plus large entre les résidus 52 et 53 couplée à une délétion d'un nombre identique de résidus au plus large entre les résidus 4 et 13.

La protéine selon l'invention peut être une protéine comprenant une séquence mutée de SEQ ID NO :1, ladite séquence mutée de SEQ ID NO :1 comprenant au moins une mutation A, dans laquelle la mutation A consiste en la substitution des acides aminés 43 à 52 de la séquence SEQ ID NO :1 par une séquence choisie parmi DFLAEGLTPR, KATN*ATLSPR et KATXATLSPR, où N* est une asparagine éventuellement glycosylée. De préférence, la protéine comprend la séquence SEQ ID NO :7 avec au moins une mutation A.
De préférence encore, dans ce cas, la protéine mutée comprend une séquence choisie parmi SEQ ID NO :9, SEQ ID NO :10 et SEQ ID NO :11.

La protéine selon l'invention peut être une protéine comprenant une séquence mutée de SEQ ID NO :1, ladite séquence mutée de SEQ ID NO :1 comprenant au moins une mutation A', dans laquelle la mutation A' consiste en la substitution des acides aminés 47 à 52 de la séquence SEQ ID NO :1 par une séquence choisie parmi TSKLTR, FNDFTR, LSSMTR, PPSLTR et LSCGQR. De préférence, la protéine comprend la séquence SEQ ID NO :7 avec au moins une mutation A'.
De préférence encore, dans ce cas, la protéine mutée comprend une séquence choisie parmi SEQ ID NO :12, SEQ ID NO :13, SEQ ID NO :14, SEQ ID NO :15 et SEQ ID NO :16.

La protéine selon l'invention peut être une protéine comprenant une séquence mutée de SEQ ID NO :1, ladite séquence mutée de SEQ ID NO :1 comprenant au moins une mutation B, dans laquelle la mutation B consiste en l'insertion d'une séquence choisie parmi DFLAEGLTPR, KATN*ATLSPR, KATXATLSPR, TSKLTR, FNDFTR, LSSMTR, PPSLTR et LSCGQR, entre les acides aminés 52 et 53 de la séquence SEQ ID NO :1, où N* est une asparagine éventuellement glycosylée. De préférence, la protéine comprend la séquence SEQ ID NO :7 avec au moins une mutation B.
De préférence encore, dans ce cas, la protéine mutée comprend une séquence choisie parmi SEQ ID NO :18, SEQ ID NO :19, SEQ ID NO :20, SEQ ID NO :21, SEQ ID NO :22, SEQ ID NO :23, SEQ ID NO :24 et SEQ ID NO :25.

La protéine selon l'invention peut être une protéine comprenant une séquence mutée de SEQ ID NO :1, ladite séquence mutée de SEQ ID NO :1 comprenant au moins une mutation C, dans laquelle la mutation C consiste en l'insertion d'une séquence choisie parmi DFLAEGLTPR, KATN*ATLSPR et KATXATLSPR, entre les acides aminés 52 et 53 de la séquence SEQ ID NO :1, et en la délétion des acides aminés 4 à 13 de la séquence SEQ ID NO :1, où N* est une asparagine éventuellement glycosylée. De préférence, la protéine comprend la séquence SEQ ID NO :7 avec au moins une mutation C.
De préférence encore, dans ce cas, la protéine mutée comprend une séquence choisie parmi SEQ ID NO :27, SEQ ID NO :28 et SEQ ID NO :29.

La protéine selon l'invention peut être une protéine comprenant une séquence mutée de SEQ ID NO :1, ladite séquence mutée de SEQ ID NO :1 comprenant au moins une mutation C', dans laquelle la mutation C' consiste en l'insertion d'une séquence choisie parmi TSKLTR, FNDFTR, LSSMTR, PPSLTR et LSCGQR, entre les acides aminés 52 et 53 de la séquence SEQ ID NO :1, et en la délétion des acides aminés 4 à 9 de la séquence SEQ ID NO :1. De préférence, la protéine comprend la séquence SEQ ID NO :7 avec au moins une mutation C'.
De préférence encore, dans ce cas, la protéine mutée comprend une séquence choisie parmi SEQ ID NO :30, SEQ ID NO :31, SEQ ID NO :32, SEQ ID NO :33 et SEQ ID NO :34.

De préférence, la protéine selon l'invention comprend, de préférence consiste en, une séquence choisie parmi SEQ ID NO: 9 à 16, 18 à 25, 27 à 34, 105 à 112, 114 à 121 et 123 à 130.

Les séquences décrites dans la présente demande peuvent être résumées comme suit:

| **SEQ ID NO :** | **Protéine** |
|---|---|
| 1 | Chaîne lourde de facteur X humain (306 acides aminés), comprenant le peptide d'activation |
| 2 | Peptide signal, propeptide, et chaîne légère de facteur X humain (182 acides aminés) |
| 3 | Peptide d'activation de la chaîne lourde (52 acides aminés) |
| 4 | Prépropeptide de facteur X humain (488 acides aminés) |
| 5 | Chaîne légère de facteur X humain (142 acides aminés) |
| 6 | Chaîne lourde du Facteur X humain activé (FXa) (254 |
| | acides aminés) |
| 7 | FX-WT (correspond à un FX humain dont la séquence nucléotidique a été optimisée) |
| 8 | FX-control+ (mutant de SEQ ID NO :7 comprenant la substitution de 10 acides aminés par une séquence correspondant au site de reconnaissance de la thrombine sur le fibrinogène = fibrinopeptide A) (comparatif) |
| 9 | FX-IIa (mutant de SEQ ID NO :7 comprenant une mutation A : insertion du site consensus de clivage de la thrombine) |
| 10 | FX-PAR1 (mutant de SEQ ID NO :7 comprenant une mutation A : insertion du site modifié de clivage de la thrombine sur le récepteur PARI) |
| 11 | FX-PAR1M (mutant de SEQ ID NO :7 comprenant une mutation A : insertion du site modifié de clivage de la thrombine sur le récepteur PAR1 sans site de glycosylation) |
| 12 | FX-FXIa1 (mutant de SEQ ID NO :7 comprenant une mutation A' : insertion du site de clivage 1 du FXIa sur le FIX) |
| 13 | FX-FXIa2 (mutant de SEQ ID NO :7 comprenant une mutation A' : insertion du site de clivage 2 du FXIa sur le FIX) |
| 14 | FX-Kal1 (mutant de SEQ ID NO :7 comprenant une mutation A' : insertion du site de clivage 1 de la kallikréine sur le FXII) |
| 15 | FX-Kal2 (mutant de SEQ ID NO :7 comprenant une mutation A' : insertion du site de clivage 2 de la kallikréine sur le FXII) |
| 16 | FX-Kal3 (mutant de SEQ ID NO :7 comprenant une mutation A' : insertion du site de clivage 3 de la kallikréine sur le FXII) |
| 17 | FX-control+ (mutant de SEQ ID NO :7 comprenant l'insertion d'une séquence correspondant au site de reconnaissance de la thrombine sur le fibrinogène = fibrinopeptide A) (comparatif) |
| 18 | FX-IIa (mutant de SEQ ID NO :7 comprenant une mutation B : insertion du site consensus de clivage de la thrombine) |
| 19 | FX-PAR1 (mutant de SEQ ID NO :7 comprenant une mutation B : insertion du site modifié de clivage de la thrombine sur le récepteur PARI) |
| 20 | FX-PAR1M (mutant de SEQ ID NO :7 comprenant une mutation B : insertion du site modifié de clivage de la thrombine sur le récepteur PAR1 sans site de glycosylation) |
| 21 | FX-FXIa1 (mutant de SEQ ID NO :7 comprenant une mutation B : insertion du site de clivage 1 du FXIa sur le FIX) |
| 22 | FX-FXIa2 (mutant de SEQ ID NO :7 comprenant une mutation B : insertion du site de clivage 2 du FXIa sur le FIX) |
| 23 | FX-Kal1 (mutant de SEQ ID NO :7 comprenant une mutation B : insertion du site de clivage 1 de la kallikréine sur le FXII) |
| 24 | FX-Kal2 (mutant de SEQ ID NO :7 comprenant une mutation B : insertion du site de clivage 2 de la kallikréine sur le FXII) |
| 25 | FX-Kal3 (mutant de SEQ ID NO :7 comprenant une mutation B : insertion du site de clivage 3 de la kallikréine sur le FXII) |
| 26 | FX-control+ (mutant de SEQ ID NO :7 comprenant l'insertion d'une séquence correspondant au site de reconnaissance de la thrombine sur le fibrinogène = |
| | fibrinopeptide A, et la délétion de 10 acides aminés) (comparatif) |
| 27 | FX-IIa (mutant de SEQ ID NO :7 comprenant une mutation C : insertion du site consensus de clivage de la thrombine) |
| 28 | FX-PAR1 (mutant de SEQ ID NO :7 comprenant une mutation C : insertion du site modifié de clivage de la thrombine sur le récepteur PARI) |
| 29 | FX-PAR1M (mutant de SEQ ID NO :7 comprenant une mutation C : insertion du site modifié de clivage de la thrombine sur le récepteur PAR1 sans site de glycosylation) |
| 30 | FX-FXIa1 (mutant de SEQ ID NO :7 comprenant une mutation C' : insertion du site de clivage 1 du FXIa sur le FIX) |
| 31 | FX-FXIa2 (mutant de SEQ ID NO :7 comprenant une mutation C' : insertion du site de clivage 2 du FXIa sur le FIX) |
| 32 | FX-Ka11 (mutant de SEQ ID NO :7 comprenant une mutation C' : insertion du site de clivage 1 de la kallikréine sur le FXII) |
| 33 | FX-Kal2 (mutant de SEQ ID NO :7 comprenant une mutation C' : insertion du site de clivage 2 de la kallikréine sur le FXII) |
| 34 | FX-Kal3 (mutant de SEQ ID NO :7 comprenant une mutation C' : insertion du site de clivage 3 de la kallikréine sur le FXII) |
| 35 | Séquence nucléique codant pour FX-WT (SEQ ID NO :7) |
| 36 à 75 | Amorces |
| 76 à 102 | Séquences nucléiques codant respectivement pour les protéines SEQ ID NO :8 à 34 |
| 103 à 130 | Séquences protéiques correspondant respectivement aux |
| | protéines SEQ ID NO :7 à 34 sans peptide signal et sans propeptide |
| 131 à 158 | Séquences nucléiques codant respectivement pour les protéines SEQ ID NO :103 à 130 |

La présente invention a également pour objet un complexe protéique comprenant :
- une protéine comprenant une séquence mutée de SEQ ID NO :1 selon l'invention, et
- au moins une protéine de séquence SEQ ID NO :2,
lesdites protéines étant liées entre elles par un pont disulfure.

Un autre objet de l'invention est un acide nucléique (polynucléotide) codant pour ladite protéine. De préférence, l'acide nucléique est choisi parmi les séquences SEQ ID NO :77 à 84, 86 à 93, 95 à 102, 133 à 140, 142 à 149 et 151 à 158.

Un autre objet de l'invention est un vecteur d'expression comprenant ledit polynucléotide codant pour ladite protéine, ou une cassette d'expression comprenant ledit polynucléotide. Selon l'invention, les vecteurs d'expression appropriés pour une utilisation selon l'invention peuvent comprendre au moins un élément de contrôle d'expression fonctionnellement lié à la séquence d'acide nucléique. Les éléments de contrôle d'expression sont insérés dans le vecteur et permettent de réguler l'expression de la séquence d'acide nucléique. Des exemples d'éléments de contrôle d'expression incluent notamment des systèmes lac, le promoteur du phage lambda, les promoteurs de levure ou les promoteurs viraux. D'autres éléments opérationnels peuvent être incorporés, comme une séquence de tête, des codons de terminaison, des signaux de polyadénylation et des séquences nécessaires pour la transcription et la traduction ultérieure de la séquence d'acide nucléique dans le système hôte. Il sera compris par l'homme de l'art que la combinaison correcte des éléments de contrôle d'expression dépend du système hôte choisi. Il sera également entendu que le vecteur d'expression doit contenir les éléments supplémentaires nécessaires pour le transfert et la réplication ultérieure du vecteur d'expression contenant la séquence d'acide nucléique dans le système hôte.

De tels vecteurs sont facilement construits en utilisant des méthodes conventionnelles ou disponibles dans le commerce.

Un autre objet de l'invention est une cellule recombinante comprenant un vecteur d'expression tel que décrit ci-dessus, ou un polynucléotide tel que décrit ci-dessus. Selon l'invention, des exemples de cellules hôtes qui peuvent être utilisées sont des cellules eucaryotes, comme les cellules animales, végétales, d'insectes et de levure ; et des cellules procaryotes, comme E. coli. Les moyens par lesquels le vecteur portant le gène peut être introduit dans les cellules comprennent notamment la microinjection, l'électroporation, la transduction ou la transfection à l'aide de DEAE-dextran, la lipofection, le phosphate de calcium ou d'autres procédures connues de l'homme de l'art. Dans un mode de réalisation préféré, les vecteurs d'expression eucaryotes qui fonctionnent dans les cellules eucaryotes sont utilisés. Des exemples de tels vecteurs comprennent les vecteurs viraux tels que les rétrovirus, adénovirus, virus de l'herpès, virus de la vaccine, virus de la variole, le poliovirus, lentivirus, les vecteurs d'expression bactériens ou des plasmides tels que pcDNA5. Les lignées cellulaires eucaryotes préférées comprennent les cellules COS, les cellules CHO, les cellules HEK, les cellules BHK, les cellules PerC6, les cellules HeLa, les cellules NIH/3T3, 293 (ATCC # CRL1573), des cellules T2, les cellules dendritiques ou les monocytes.

La protéine selon l'invention peut être produite dans le lait d'animaux transgéniques. Dans ce cas, selon un premier aspect, l'expression d'une séquence d'ADN contenant un gène codant pour la protéine selon l'invention est contrôlée par un promoteur de caséine de mammifère ou un promoteur de lactosérum de mammifère, ledit promoteur ne contrôlant pas naturellement la transcription dudit gène, et la séquence d'ADN contenant en outre une séquence de sécrétion de la protéine. La séquence de sécrétion comprend un signal de sécrétion interposé entre le gène et le promoteur.
L'animal transgénique utilisé est capable non seulement de produire la protéine désirée, mais également de transmettre cette capacité à sa descendance. La sécrétion de la protéine dans le lait facilite la purification et évite l'utilisation de produits sanguins. L'animal peut ainsi être choisi parmi la chèvre, la lapine, la brebis ou la vache.

La protéine selon l'invention peut être utilisée en tant que médicament. Par conséquent, la protéine selon l'invention peut être introduite dans une composition pharmaceutique. En particulier, la protéine selon l'invention peut être utilisée pour le traitement des troubles de la coagulation, notamment des troubles hémorragiques.

La composition pharmaceutique de l'invention peut être combinée avec des excipients pharmaceutiquement acceptables, et éventuellement des matrices à libération prolongée, comme des polymères biodégradables, pour former une composition thérapeutique.
La composition pharmaceutique de la présente invention peut être administrée par voie orale, sublinguale, sous-cutanée, intramusculaire, intraveineuse, intra-artérielle, intrathécale, intra-oculaire, intra-cérébrale, transdermique, locale ou rectale. Le principe actif, seul ou en association avec un autre principe actif, peut alors être administré sous forme unitaire d'administration, en mélange avec des supports pharmaceutiques classiques. Des formes unitaires d'administration comprennent les formes par voie orale telles que les comprimés, les gélules, les poudres, les granules et les solutions ou suspensions orales, les formes d'administration sublinguale et buccale, les aérosols, les implants sous-cutanés, transdermique, topique, intrapéritonéale, intramusculaire, intraveineuse, sous-cutanée, intrathécale, les formes d'administration par voie intranasale et les formes d'administration rectale.
De préférence, la composition pharmaceutique contient un véhicule pharmaceutiquement acceptable pour une formulation susceptible d'être injectée. Il peut s'agir en particulier de formules isotoniques, stériles, de solutions salines (avec phosphate monosodique ou disodique, chlorure de sodium, de potassium, de calcium ou de magnésium et analogues, ou des mélanges de tels sels), ou de compositions lyophilisées, qui, lors de l'addition d'eau stérilisée ou de sérum physiologique selon les cas, permettent la constitution de solutés injectables.
Les formes pharmaceutiques appropriées pour une utilisation injectable comprennent des solutions aqueuses stériles ou des dispersions, des formulations huileuses, y compris l'huile de sésame, l'huile d'arachide, et des poudres stériles pour la préparation extemporanée de solutions injectables stériles ou de dispersions. Dans tous les cas, la forme doit être stérile et doit être fluide dans la mesure où elle doit être injectée par seringue. Elle doit être stable dans les conditions de fabrication et de stockage et doit être préservée contre l'action contaminante de micro-organismes, comme les bactéries et les champignons.
Les dispersions selon l'invention peuvent être préparées dans du glycérol, des polyéthylèneglycols liquides ou leurs mélanges, ou dans des huiles. Dans des conditions normales de stockage et d'utilisation, ces préparations contiennent un conservateur pour empêcher la croissance des micro-organismes.
Le véhicule pharmaceutiquement acceptable peut être un solvant ou milieu de dispersion contenant, par exemple, l'eau, l'éthanol, un polyol (par exemple, la glycérine, le propylène glycol, le polyéthylène glycol, et analogues), des mélanges appropriés de ceux-ci, et/ou les huiles végétales. La fluidité convenable peut être maintenue, par exemple, par l'utilisation d'un tensioactif, tel que la lécithine. La prévention de l'action de micro-organismes peut être provoquée par divers agents antibactériens et antifongiques, par exemple, des parabènes, le chlorobutanol, le phénol, l'acide sorbique ou encore le thimérosal. Dans de nombreux cas, il sera préférable d'inclure des agents isotoniques, par exemple, des sucres ou du chlorure de sodium. L'absorption prolongée des compositions injectables peut être provoquée par l'utilisation dans les compositions d'agents retardant l'absorption, par exemple, le monostéarate d'aluminium ou la gélatine. Les solutions injectables stériles sont préparées en incorporant les substances actives en quantité requise dans le solvant approprié avec plusieurs des autres ingrédients énumérés ci-dessus, le cas échéant, suivie d'une stérilisation par filtration. En règle générale, les dispersions sont préparées en incorporant les divers ingrédients actifs stérilisés dans un véhicule stérile qui contient le milieu de dispersion basique et les autres ingrédients requis parmi ceux énumérés ci-dessus. Dans le cas de poudres stériles pour la préparation de solutions injectables stériles, les procédés de préparation préférés sont le séchage sous vide et la lyophilisation. Lors de la formulation, les solutions seront administrées d'une manière compatible avec la formulation posologique et en une quantité thérapeutiquement efficace. Les formulations sont facilement administrées dans une variété de formes galéniques, telles que les solutions injectables décrites ci-dessus, mais les capsules de libération de médicament et similaires peuvent également être utilisés. Pour l'administration parentérale dans une solution aqueuse par exemple, la solution doit être convenablement tamponnée et le diluant liquide rendu isotonique avec suffisamment de solution saline ou de glucose. Ces solutions aqueuses particulières conviennent particulièrement pour une administration intraveineuse, intramusculaire, sous-cutanée et intrapéritonéale. À cet égard, les milieux aqueux stériles qui peuvent être utilisés sont connus de l'homme de l'art. Par exemple, une dose peut être dissoute dans 1 ml de solution de NaCl isotonique puis ajoutée à 1000 ml de liquide approprié, ou injectée sur le site proposé de la perfusion. Certaines variations de posologie devront nécessairement se produire en fonction de l'état du sujet traité.

La composition pharmaceutique de l'invention peut être formulée dans un mélange thérapeutique comprenant environ 0.0001 à 1.0 milligrammes, soit environ 0.001 à 0.1 milligrammes, soit environ de 0.1 à 1.0 milligrammes, voire environ 10 milligrammes par dose ou plus. Des doses multiples peuvent également être administrées. Le niveau de dose thérapeutiquement efficace spécifique pour un patient particulier dépendra d'une variété de facteurs, y compris le trouble qui est traité et la gravité de la maladie, l'activité du composé spécifique employé, la composition spécifique utilisée, l'âge, le poids corporel, la santé générale, le sexe et le régime alimentaire du patient, le moment de l'administration, la voie d'administration, le taux d'excrétion du composé spécifique utilisé, la durée du traitement, ou encore les médicaments utilisés en parallèle.

Les exemples suivants sont donnés en vue d'illustrer divers modes de réalisation de l'invention.

### Légende des figures

**Figure 1** **: Structure du facteur X natif humain.**
**Figure 2** **: Stratégies de développement des mutants.**
   La famille 1 englobe les mutants comprenant les mutations A ou A'.
   La famille 2 regroupe les mutants comprenant la mutation B.
   La famille 3 regroupe les mutants comprenant la mutation C ou C'.
**Figure 3** **: Niveau d'expression des facteurs variants produits dans CHO.**
   Les 3 familles de FX ont été exprimées suite à une transfection dans CHO. Les surnageants à jour 7 ont été analysés en triplicat avec le kit Zymutest FX (Hyphen). Les concentrations (µg/ml) sont indiquées en ordonnées. Les déviations standards sont indiquées au-dessus des histogrammes. Famille 1, barre grise ; famille 2, barre noire ; famille 3, barre blanche.

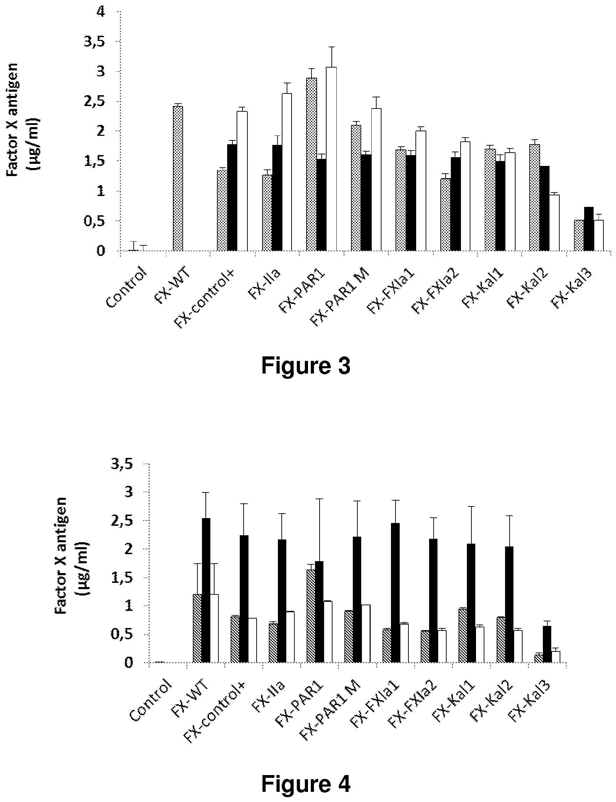

**Figure 4** **: Niveau d'expression des facteurs X et variants produits dans HEK.**
   Les 3 familles de FX ont été exprimées suite à une transfection dans HEK293S. Les surnageants à jour 7 ont été analysés en triplicat avec le kit Zymutest FX (Hyphen). Les concentrations (µg/ml) sont indiquées en ordonnées. Les déviations standards sont indiquées au-dessus des histogrammes. Famille 1, barre grise ; famille 2, barre noire ; famille 3, barre blanche.

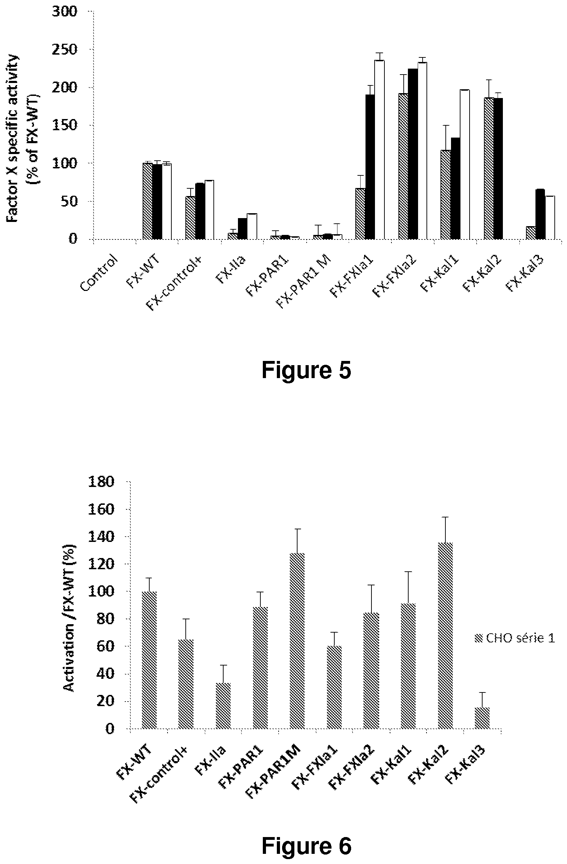

**Figure 5** **: Activité chronométrique des facteurs X et variants produits dans CHO dans du plasma déficient en facteur X.**
   Les 3 familles de FX ont été exprimées suite à une transfection dans CHO-S. Les surnageants à jour 7 ont été analysés au moins en duplicat par un test TP sur automate STAR (Stago). Les temps de coagulation ont permis de calculer une activité spécifique (en s/(µg/ml)) puis ont été transformés en pourcentage de l'activité du facteur X recombinant sauvage. Ces valeurs sont présentées en ordonnées. Les déviations standards sont indiquées au-dessus des histogrammes. Famille 1, barre grise ; famille 2, barre noire ; famille 3, barre blanche.* FX-Kal2 famille 3, non fait.
**Figure 6** **: Activité des facteurs X variants de la famille 1 produits dans CHO après activation par la fraction RVV-X.**
   Les FX variants de la famille 1 ont été exprimés suite à une transfection dans CHO-S. Les surnageants à jour 7 ont été incubés à deux concentrations de FX (au moins en duplicat) en présence de la fraction RVV-X du venin de la vipère de Russell. L'apparition de FXa a été mesurée en suivant l'hydrolyse du substrat pNAPEP 1025 à 405 nM. Les vitesses initiales de transformation (mUDO/min/nM) ont été comparées à celle du FX-WT, fixée à 100%. La moyenne des valeurs à deux concentrations est établie et présentée en ordonnées. Les déviations standards sont indiquées au-dessus des histogrammes.

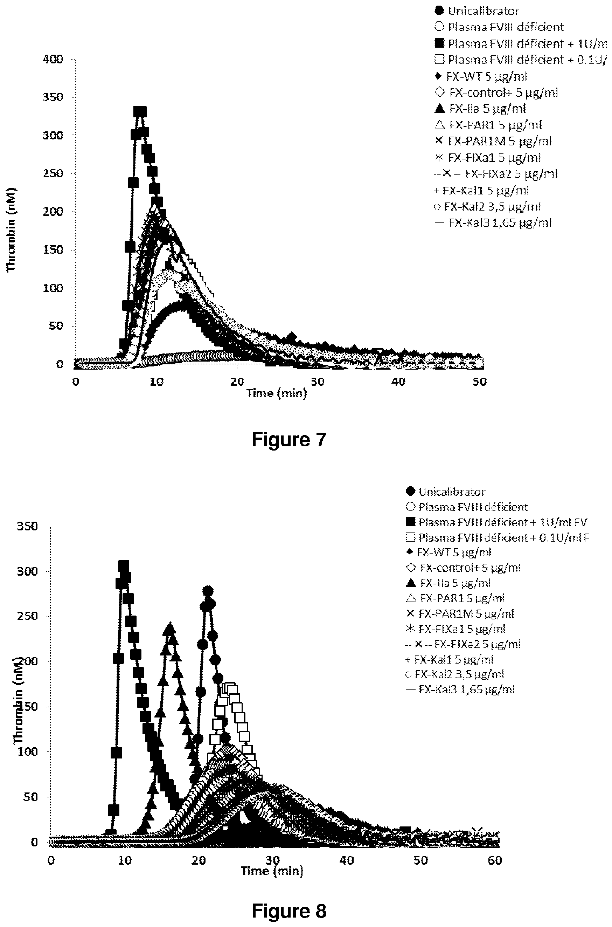

**Figure 7** **: Thrombinogrammes obtenus à partir de l'ajout de FX ou de ses variants de la famille 2 dans un pool de plasma déficient en facteur VIII suite à l'activation par du facteur tissulaire (1 pM).**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml FVIII sont représentées par les symboles ■ et □ respectivement. Les symboles représentant les FX de type sauvage et les variants sont les suivants : FX-WT (◆), FX- control+ (◇), FX-IIa (▲), FX-PAR1 (A), FX-PAR1M (× en trait plein), FX-FIXa1 ( 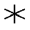 ), FX-FIXa2 (× avec courbe pointillée), FX-Ka11 ( ), FX-Kal2 ( ) et FX-Kal3 (trait plein sans symbole). Le facteur X ou ses variants ont été utilisés à 5 µg/ml sauf pour les FX-Kal2 et 3 qui pour des raisons techniques ont été utilisés à 3.5 et 1.65 µg/ml respectivement.
**Figure 8** **: Thrombinogrammes obtenus à partir de l'ajout de FX ou de ses variants de la famille 2 dans un pool de plasma déficient en facteur VIII suite à l'activation par de la céphaline.**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆), FX- control+ (◇), FX-IIa (▲), FX-PAR1 (A), FX-PAR1M (× en trait plein), FX-FIXa1 ( 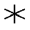 ), FX-FIXa2 (× avec courbe pointillée), FX-Ka11 ( ), FX-Kal2 ( ) et FX-Kal3 (trait plein sans symbole). Le facteur X ou ses variants ont été utilisés à 5 µg/ml sauf pour les FX-Kal2 et 3 qui pour des raisons techniques ont été utilisés à 3.5 et 1.65 µg/ml respectivement.

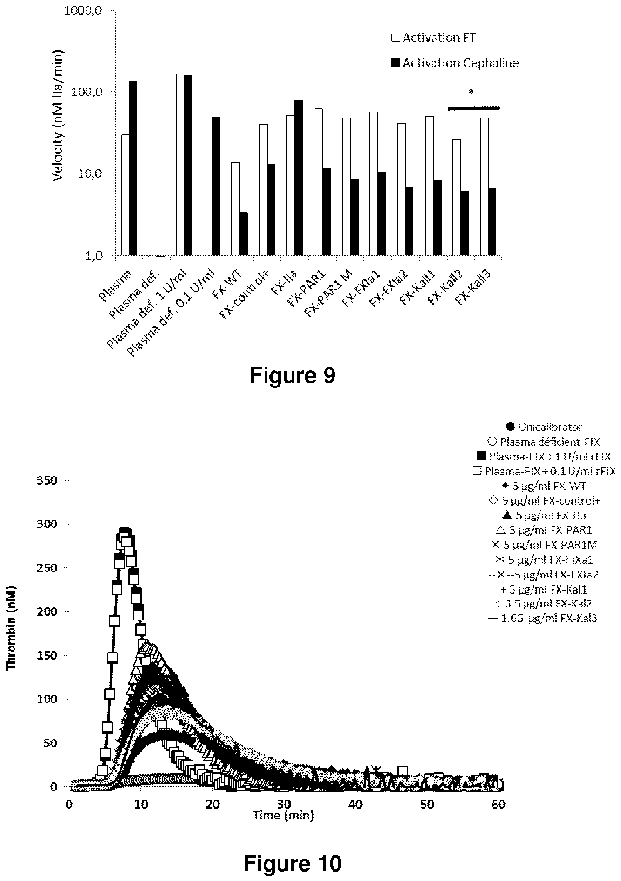

**Figure 9** **: Vélocités des thrombogrammes obtenus à partir de l'ajout de FX ou de ses variants de la famille 2 dans un pool de plasma déficient en facteur VIII, suite à l'activation par du facteur tissulaire ou de la céphaline.**
   Les valeurs des vélocités (en nM/min de thrombine générée) issues des figures 7 et 8 sont présentées. A noter que les FX-Kal2 et 3 (*) ont été utilisés à 3,5 et 1,65 µg/ml respectivement au lieu de 5 µg/ml pour les autres FX et variants. Barres blanches, valeurs obtenues par une activation au facteur tissulaire (1 pM); Barres noires valeurs obtenues par une activation à la céphaline.
**Figure 10** **: Thrombinogrammes obtenus à partir de l'ajout de FX ou de ses variants de la famille 2 dans un pool de plasma déficient en facteur IX suite à l'activation par du facteur tissulaire (1 pM).**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆), FX- control+ (◇), FX-IIa (▲), FX-PAR1 (A), FX-PAR1M (× en trait plein), FX-FIXa1 ( 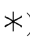 ), FX-FIXa2 (× avec courbe pointillée), FX-Ka11 ( ), FX-Kal2 ( ) et FX-Kal3 (trait plein sans symbole). ). Le facteur X ou ses variants ont été utilisés à 5 µg/ml sauf pour les FX-Kal2 et 3 qui pour des raisons techniques ont été utilisés à 3.5 et 1.65 µg/ml respectivement.

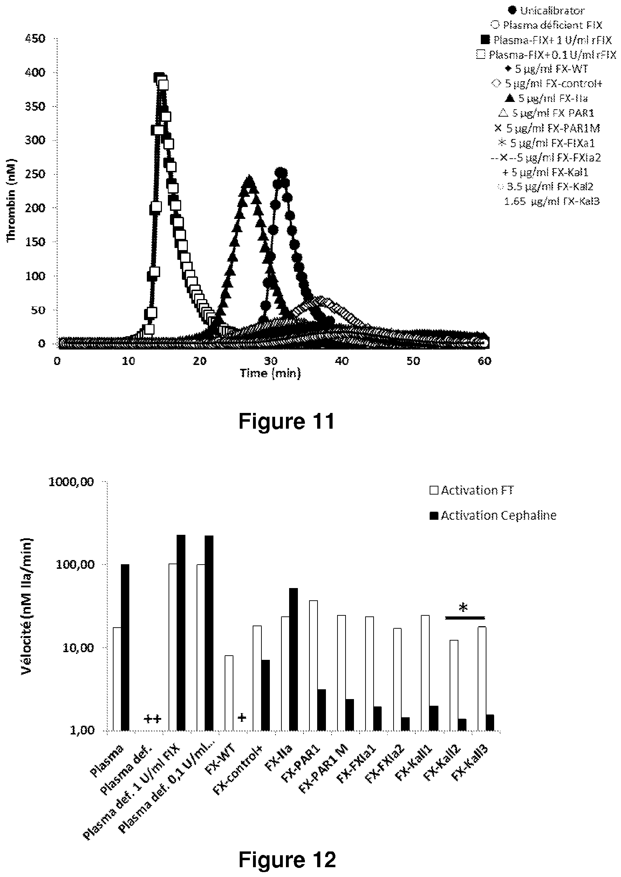

**Figure 11****: Thrombinogrammes obtenus à partir de l'ajout de FX ou de ses variants de la famille 2 dans un pool de plasma déficient en facteur IX suite à l'activation par de la céphaline.**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé en FVIII 1 U/ml ou 0.1 U/ml sont représentées par les symboles ■ et □ respectivement. Les symboles représentant les FX de type sauvage et les variants sont les suivants : FX-WT (◆), FX- control+ (◊), FX-IIa (▲), FX-PAR1 (Δ), FX-PAR1M (× en trait plein), FX-FIXa1 ( 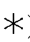 ), FX-FIXa2 (× avec courbe pointillée), FX-Ka11 ( ), FX-Kal2 ( ) et FX-Kal3 (trait plein sans symbole). Le facteur X ou ses variants ont été utilisés à 5 µg/ml sauf pour les FX-Kal2 et 3 qui pour des raisons techniques ont été utilisés à 3.5 et 1.65 µg/ml respectivement.
**Figure 12** **: Vélocités des thrombogrammes obtenus à partir de l'ajout de FX ou de ses variants de la famille 2 dans un pool de plasma déficient en facteur IX, suite à l'activation par du facteur tissulaire (1 pM) ou de la céphaline.**
   Les valeurs des vélocités (en nM/min de thrombine générée) issues des figures 10 et 11 sont présentées. A noter que les FX-Kal2 et 3 (*) ont été utilisés à 3,5 et 1,65 µg/ml respectivement au lieu de 5 µg/ml pour les autres FX et variants. Les résultats sont présentés en échelle logarithmique. Pour plus de lisibilité, les valeurs inférieures à 1 nM/min de thrombine générée ne sont pas représentées (+). Barres blanches, valeurs obtenues par une activation au facteur tissulaire (1 pM); Barres noires valeurs obtenues par une activation à la céphaline.

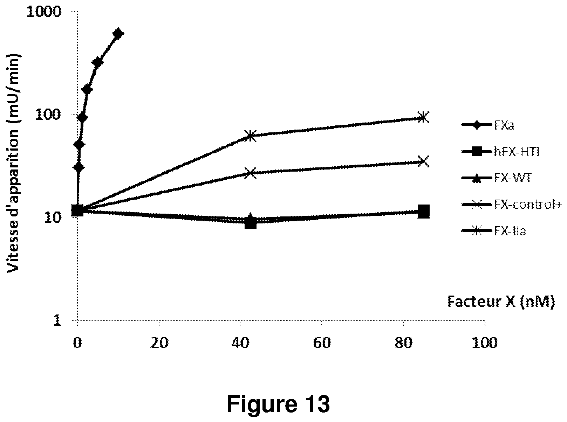

**Figure 13****: Activation par la thrombine du FX-WT et de variants de la famille 2.**
   L'activation du FX-WT et certains de ses variants de la famille 2 a été réalisée en présence de thrombine (10 nM) en tampon Hepes contenant du substrat Pefachrome FXa 8595. L'apparition du paranitroannilide libéré par le FXa généré a été suivie à 405 nm au cours du temps. Du facteur X activé, à différentes concentrations, a été utilisé en contrôle positif.

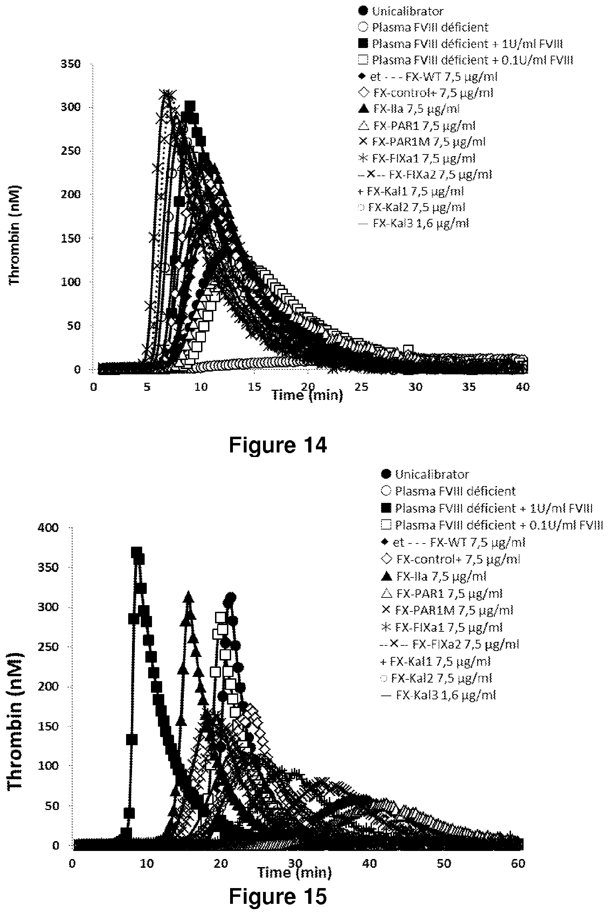

**Figure 14** **: Thrombinogrammes obtenus à partir de l'ajout de FX ou de ses variants de la famille 1 dans un pool de plasma déficient en facteur VIII suite à l'activation par le facteur tissulaire**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆), FX- control+ (◇), FX-IIa (▲), FX-PAR1 (A), FX-PAR1M (× en trait plein), FX-FIXa1 (∗), FX-FIXa2 (× avec courbe pointillée), FX-Ka11 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.
**Figure 15** **: Thrombinogrammes obtenus à partir de l'ajout de FX ou de ses variants de la famille 1 dans un pool de plasma déficient en facteur VIII suite à l'activation par de la céphaline**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆), FX- control+ (◇), FX-IIa (▲), FX-PAR1 (A), FX-PAR1M (× en trait plein), FX-FIXa1 (∗), FX-FIXa2 (× avec courbe pointillée), FX-Ka11 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.

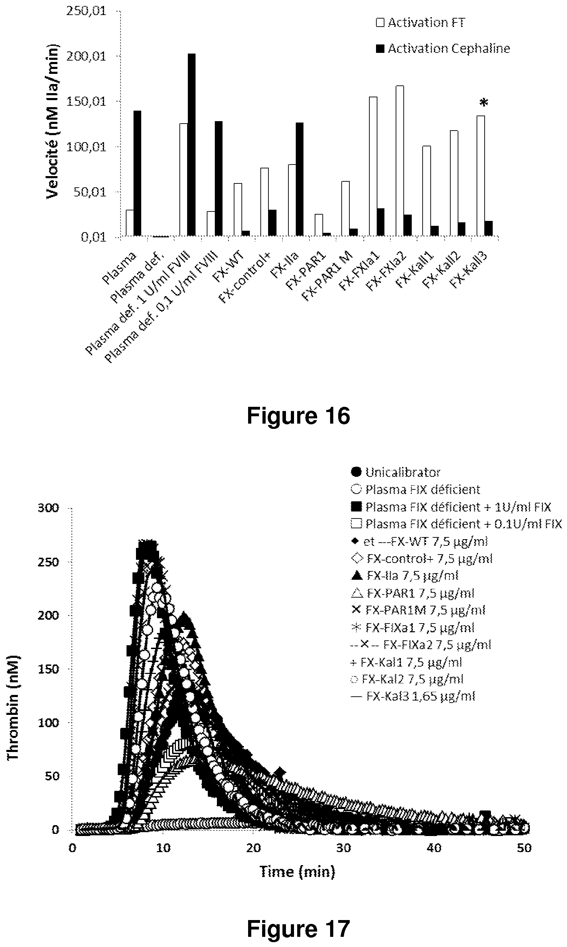

**Figure 16** **: Vélocités des thrombogrammes obtenus à partir de l'ajout de FX ou de ses variants de la famille 1 dans un pool de plasma déficient en facteur VIII, suite à l'activation par du facteur tissulaire (1 pM) ou de la céphaline**
   Les valeurs des vélocités (en nM/min de thrombine générée) issues des figures 14 et 15 sont présentées. A noter que le FX-Kal3 (*) a été utilisé à 1,65 µg/ml au lieu de 7,5 µg/ml pour les autres FX et variants. La valeur du FX-WT est la moyenne des deux expériences. Pour plus de lisibilité, les valeurs inférieures à 1 nM/min de thrombine générée ne sont pas représentées (+). Barres blanches, valeurs obtenues par une activation au facteur tissulaire (1 pM); Barres noires, valeurs obtenues par une activation à la céphaline.
**Figure 17** **: Thrombinogrammes obtenus à partir de l'ajout de FX ou de ses variants de la famille 1 dans un pool de plasma déficient en facteur IX suite à l'activation par le facteur tissulaire**
   - en abscisses : le temps (en minutes)
   - en ordonnées : la concentration maximale de thrombine observée (en nM)

   L'échantillon de pool de plasma normal est représenté par la courbe noire (●) et celui déficient en FIX par (○). Les courbes correspondant au plasma déficient surchargé par 1 U/ml ou 0.1 U/ml de FIX sont représentées par les symboles ■ et □ respectivement. Les symboles représentant les FX de type sauvage et les variants sont les suivants : FX-WT (◆), FX- control+ (◊), FX-IIa (▲), FX-PAR1 (Δ), FX-PAR1M (× en trait plein), FX-FIXa1 (∗), FX-FIXa2 (× avec courbe pointillée), FX-Ka11 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.

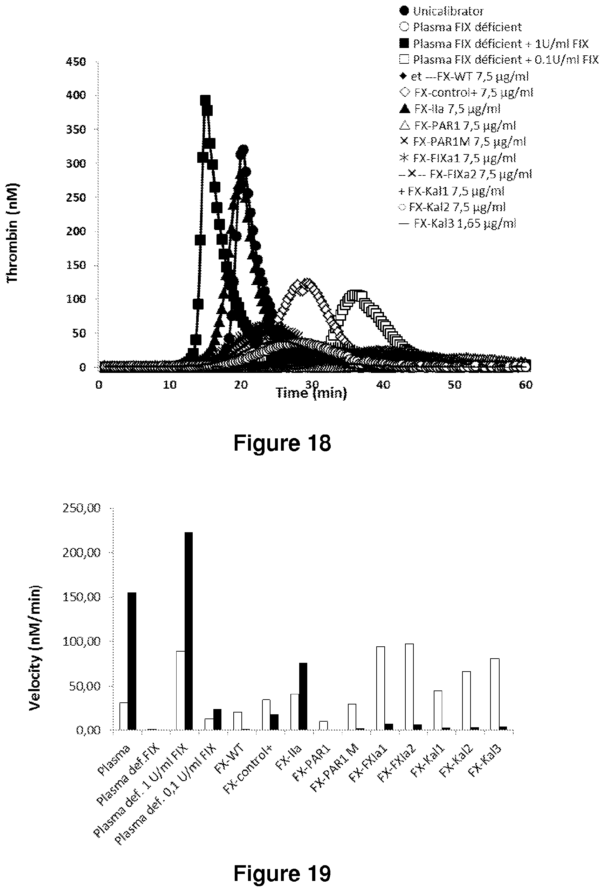

**Figure 18****: Thrombinogrammes obtenus à partir de l'ajout de FX ou de ses variants de la famille 1 dans un pool de plasma déficient en facteur IX suite à l'activation par de la céphaline**
   - en abscisses : le temps (en minutes)
   - en ordonnées : la concentration maximale de thrombine observée (en nM)

   L'échantillon de pool de plasma normal est représenté par la courbe noire (●) et celui déficient en FIX par (○). Les courbes correspondant au plasma déficient surchargé par 1 U/ml ou 0.1 U/ml de FIX sont représentées par les symboles ■ et □ respectivement. Les symboles représentant les FX de type sauvage et les variants sont les suivants : FX-WT (◆), FX- control+ (◊), FX-IIa (▲), FX-PAR1 (Δ), FX-PAR1M (× en trait plein), FX-FIXa1 (∗), FX-FIXa2 (× avec courbe pointillée), FX-Ka11 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.
**Figure 19** **: Vélocités des thrombogrammes obtenus à partir de l'ajout de FX ou de ses variants de la famille 1 dans un pool de plasma déficient en facteur IX, suite à l'activation par du facteur tissulaire (1 pM) ou de la céphaline**
   Les valeurs des vélocités (en nM/min de thrombine générée) issues des figures 17 et 18 sont présentées. A noter que le FX-Kal3 (*) a été utilisé à 1,65 µg/ml au lieu de 7,5 µg/ml pour les autres FX et variants. La valeur du FX-WT est la moyenne des deux expériences. Pour plus de lisibilité, les valeurs inférieures à 1 nM/min de thrombine générée ne sont pas représentées (+). Barres blanches, valeurs obtenues par une activation au facteur tissulaire (1 pM); Barres noires valeurs obtenues par une activation à la céphaline.

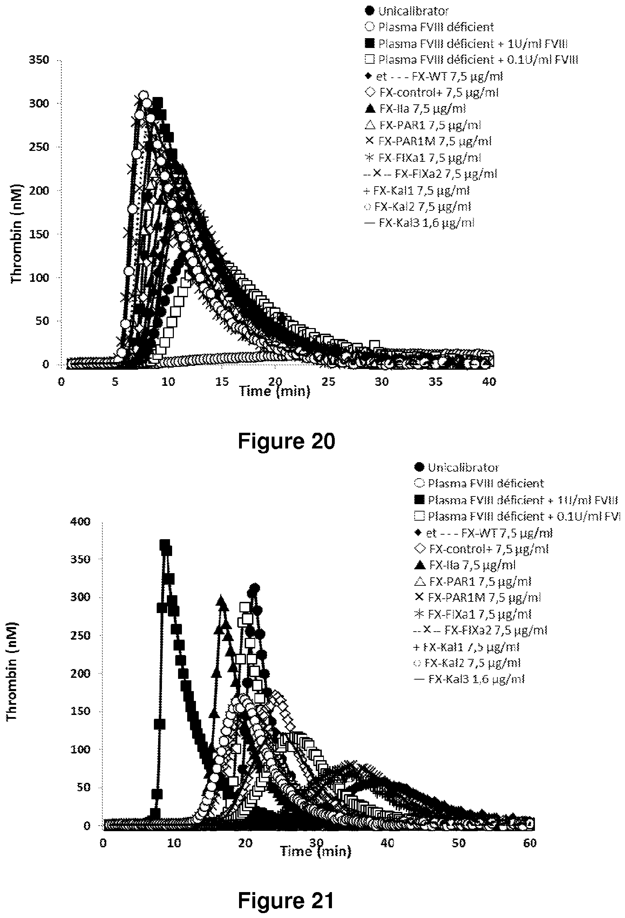

**Figure 20** **: Thrombinogrammes obtenus à partir de l'ajout de FX ou de ses variants de la famille 3 dans un pool de plasma déficient en facteur VIII suite à l'activation par le facteur tissulaire**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆ et ---), FX- control+ (◇), FX-IIa (▲), FX-PAR1 (A), FX-PAR1M (× en trait plein), FX-FIXa1 (∗), FX-FIXa2 (× avec courbe pointillée), FX-Kal1 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.
**Figure 21** **: Thrombinogrammes obtenus à partir de l'ajout de FX ou de ses variants de la famille 3 dans un pool de plasma déficient en facteur VIII suite à l'activation par de la céphaline**
   - en abscisses : le temps (en minutes)
   - en ordonnées : la concentration maximale de thrombine observée (en nM)

   L'échantillon de pool de plasma normal est représenté par la courbe noire (●) et celui déficient en FVIII par (○). Les courbes correspondant au plasma déficient surchargé par 1 U/ml ou 0.1 U/ml de FVIII sont représentées par les symboles ■ et □ respectivement. Les symboles représentant les FX de type sauvage et les variants sont les suivants : FX-WT (◆ et ---), FX- control+ (◇), FX-IIa (▲), FX-PAR1 (A), FX-PAR1M (× en trait plein), FX-FIXa1 (∗), FX-FIXa2 (× avec courbe pointillée), FX-Kal1 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.

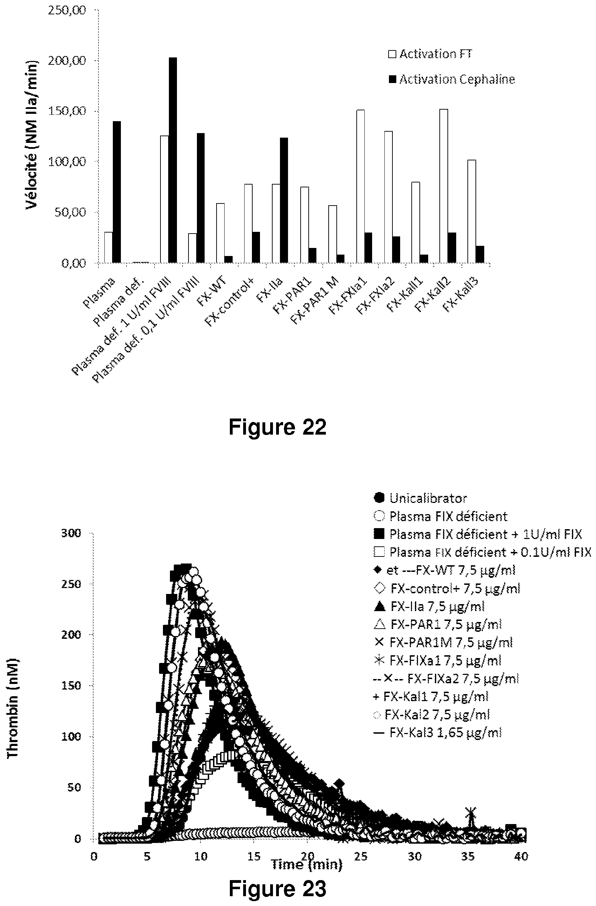

**Figure 22** **: Vélocités des thrombogrammes obtenus à partir de l'ajout de FX ou de ses variants de la famille 3 dans un pool de plasma déficient en facteur VIII, suite à l'activation par du facteur tissulaire (1 pM) ou de la céphaline**
   Les valeurs des vélocités (en nM/min de thrombine générée) issues des figures 20 et 21 sont présentées. A noter que le FX-Kal3 (*) a été utilisé à 1,65 µg/ml au lieu de 7,5 µg/ml pour les autres FX et variants. La valeur du FX-WT est la moyenne des deux expériences. Barres blanches, valeurs obtenues par une activation au facteur tissulaire (1 pM); Barres noires, valeurs obtenues par une activation à la céphaline.
**Figure 23** **: Thrombinogrammes obtenus à partir de l'ajout de FX ou de ses variants de la famille 3 dans un pool de plasma déficient en facteur IX suite à l'activation par le facteur tissulaire**
   - en abscisses : le temps (en minutes)
   - en ordonnées : la concentration maximale de thrombine observée (en nM)

   L'échantillon de pool de plasma normal est représenté par la courbe noire (●) et celui déficient en FIX par (○). Les courbes correspondant au plasma déficient surchargé par 1 U/ml ou 0.1 U/ml de FIX sont représentées par les symboles ■ et □ respectivement. Les symboles représentant les FX de type sauvage et les variants sont les suivants : FX-WT (◆), FX- control+ (◊), FX-IIa (▲), FX-PAR1 (Δ), FX-PAR1M (× en trait plein), FX-FIXa1 (∗), FX-FIXa2 (× avec courbe pointillée), FX-Kal1 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.

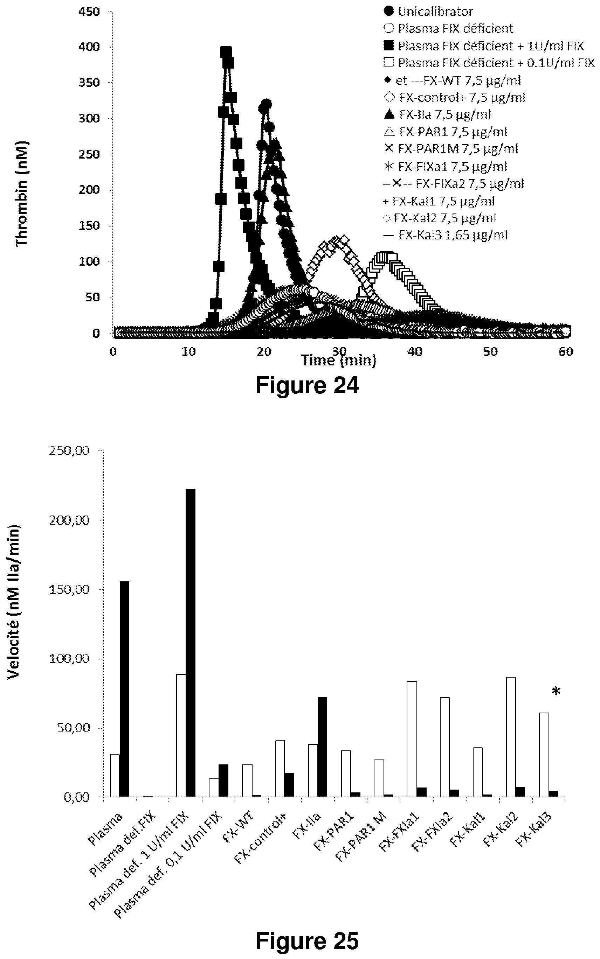

**Figure 24** **: Thrombinogrammes obtenus à partir de l'ajout de FX ou de ses variants de la famille 3 dans un pool de plasma déficient en facteur IX suite à l'activation par de la céphaline**
   - en abscisses : le temps (en minutes)
   - en ordonnées : la concentration maximale de thrombine observée (en nM)

   L'échantillon de pool de plasma normal est représenté par la courbe noire (●) et celui déficient en FIX par (O). Les courbes correspondant au plasma déficient surchargé par 1 U/ml ou 0.1 U/ml de FIX sont représentées par les symboles ■ et □ respectivement. Les symboles représentant les FX de type sauvage et les variants sont les suivants : FX-WT (◆), FX- control+ (◊), FX-IIa (▲), FX-PAR1 (Δ), FX-PAR1M (× en trait plein), FX-FIXa1 (∗), FX-FIXa2 (× avec courbe pointillée), FX-Ka11 ( ), FX-Kal2 ( ) et FX-Kal3 (trait plein sans symbole). Le facteur X ou ses variants ont été utilisés à 7,5 µg/ml sauf pour FX-Kal3 qui pour des raisons techniques a été utilisé à 1.65 µg/ml.
**Figure 25** **: Vélocités des thrombogrammes obtenus à partir de l'ajout de FX ou de ses variants de la famille 3 dans un pool de plasma déficient en facteur IX, suite à l'activation par du facteur tissulaire (1 pM) ou de la céphaline**
   Les valeurs des vélocités (en nM/min de thrombine générée) issues des figures 23 et 24 sont présentées. A noter que le FX-Kal3 (*) a été utilisé à 1,65 µg/ml au lieu de 7,5 µg/ml pour les autres FX et variants. La valeur du FX-WT est la moyenne des deux expériences. Barres blanches, valeurs obtenues par une activation au facteur tissulaire (1 pM); Barres noires, valeurs obtenues par une activation à la céphaline.

### Exemples

### Exemple 1 : Génération des ADN complémentaires des variants de facteurs X

### 1- Généralités

Les séquences nucléotidiques et protéiques des différentes constructions sont fournies dans le listage de séquences, et sont résumées dans le tableau de la description. La molécule de FX sauvage est appelée FX-WT (SEQ ID NO :7), elle correspond à un FX humain dont la séquence nucléotidique a été optimisée. Cette molécule servira de contrôle pour les trois familles de molécules mutées.

Les molécules mutées sont nommées en fonction du site de clivage placé en amont de la chaine lourde. Le FX-control+ correspond au site de reconnaissance de la thrombine sur le fibrinogène, ou fibrinopeptide A. Les molécules mutées selon l'invention se nomment respectivement FX-IIa (site consensus de clivage à la thrombine), FX-PAR1 (site modifié de clivage de la thrombine sur le récepteur PARI), FX-PAR1M (site modifié de clivage de la thrombine sur le récepteur PAR1 sans site de glycosylation), FX-FXIa1 (site de clivage 1 du FXIa sur le FIX), FX-FXIa2 (site de clivage 2 du FXIa sur le FIX), FX-Ka11 (site de clivage 1 de la Kallikreine sur le FXII), FX-Kal2 (site de clivage 2 de la Kallikreine sur le FXII) et FX-Kal3 (site de clivage 3 de la Kallikreine sur le FXII).

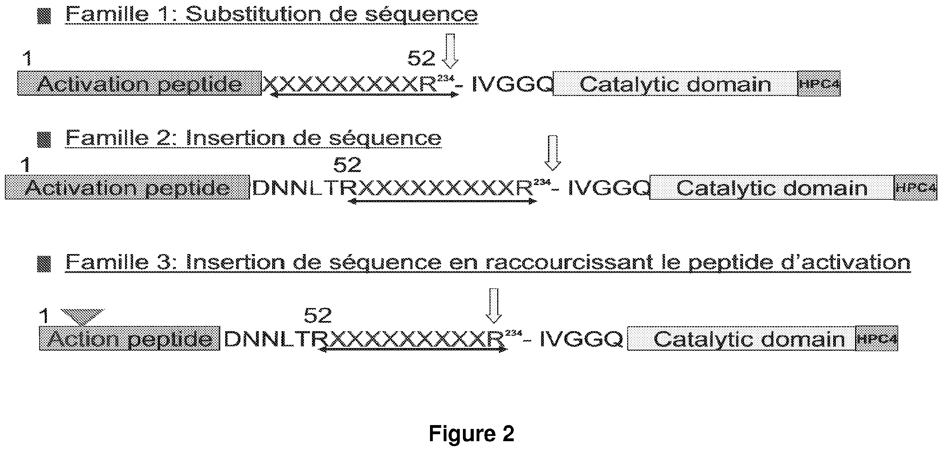

Comme montré en Figure 2, chacun de ces sites est présent dans un environnement différent (famille), à savoir :
- soit dans la famille 1 qui consiste en la substitution de 6 ou 10 acides aminés en amont du site de clivage du peptide d'activation (mutation A ou A') ;
- soit dans la famille 2 qui consiste en l'insertion des mêmes séquences en amont du site de clivage du peptide d'activation (mutation B) ; ou
- soit dans la famille 3 qui consiste en l'insertion de ces séquences en amont du site de clivage du peptide d'activation, couplée à la délétion d'une partie du peptide d'activation (mutation C ou C').

Ainsi, les protéines de séquence SEQ ID NO : 9 à 16 correspondent à la séquence SEQ ID NO :7, dans laquelle une mutation A ou A' a été insérée. Ces protéines appartiennent donc à la famille 1.

Les protéines de séquence SEQ ID NO : 18 à 25 correspondent à la séquence SEQ ID NO :7, dans laquelle une mutation B a été insérée. Ces protéines appartiennent donc à la famille 2.

Enfin, les protéines de séquence SEQ ID NO : 27 à 34 correspondent à la séquence SEQ ID NO :7, dans laquelle une mutation C ou C' a été insérée. Ces protéines appartiennent donc à la famille 3.

Les séquences SEQ ID NO :8, 17 et 26 correspondent à un FX-control+, et sont des comparatifs.

### 2- Protocole expérimental

Les séquences spécifiques à chaque variant sont introduites par PCR d'assemblage ou Infusion, à l'aide d'amorces judicieusement conçues pour permettre l'insertion et/ou la délétion de séquences nucléotidiques, au sein d'une séquence nucléotidique synthétique codant le FX optimisée pour une expression dans *Homo sapiens* (SEQ ID NO :35).

### 2.1. Préparation du vecteur pCEP4-FXWT4HS-gs codant le FX humain

Le vecteur de clonage pUC57 contenant le gène synthétique optimisé pour une expression chez *Homo sapiens* et préparé par Genescript est digéré comme le vecteur d'expression pCEP4 (Life Technologies) par les enzymes BamHI et HindIII. L'insert correspondant au gène du FX (FXWT4HSgs) et le vecteur digéré pCEP4 sont purifiés par Nucleospin extract II (Clonetech Laboratories) avant d'être ligués ensemble par la T4 ligase. Le produit de ligation est utilisé pour transformer des bactéries Top10 (Life Technologies). La présence de l'insert dans les colonies bactériennes est déterminée par digestion du plasmide par les enzymes BamHI et HindIII et passage du produit de digestion sur gel d'agarose pour y détecter une bande de 1519 pb. L'ADNc est vérifié par séquençage en utilisant les amorces CMVs1 (5'-GGGACTTTCCTACTTGGCAGT-3' SEQ ID NO :36) et SV40-3'UTR (5'-TTCACTGCATTCTAGTTGTGGT-3' SEQ ID NO :37).

### 2.2- Préparation du vecteur OptiCHO FXWT4HS codant le FX humain

Pour une expression optimale des variants, ces derniers ainsi que la molécule sauvage sont préparés dans le vecteur OptiCHO.
A partir du vecteur pCEP4-FXWT4HS-gs, l'ADNc de la séquence FXWT4HS est amplifié par PCR (kapa Hifi ; Biosystem) avec les amorces 5'FXWT et 3'FX-SwaI.

Le produit de PCR de 1551 pb est purifié par Nucleospin extract avant d'être digéré par les enzymes NheI et SwaI, tout comme le vecteur de destination OptiCHO. Ils sont à nouveau purifiés sur Nucleospin extract après digestion.
L'insert et le vecteur sont ligués ensemble par la T4 ligase avant que le produit de ligation ne soit intégré dans des bactéries compétentes Top10. Après amplification bactérienne en présence d'ampicilline, des colonies bactériennes sont prélevées sur boite de Pétri et criblées par PCR pour la recherche d'un amplicon de 296 pb avec les amorces 5'efla et 3FX, signe de la présence de l'insert codant le FX dans le vecteur OptiCHO. Le criblage par PCR est complété d'un criblage des vecteurs purifiés par digestion enzymatique par les enzymes Nhe I et SwaI pour rechercher sur gel d'agarose un fragment de 1538 pb. Le vecteur OptiCHO-FXWT4HS est séquencé avec les amorces :
- 5'efla : 5'-GTGGAGACTGAAGTTAGGCCAG-3' (SEQ ID NO :38)
- 3FX : 5'-CTTCATTTCCTCCAGGAAAGAGTTGGC-3' (SEQ ID NO :39)
- 2BGHPA : 5'-CAGATGGCTGGCAACTAGAA-3' (SEQ ID NO :40)

### 2.3. Préparation des variants de la famille 1

La préparation des inserts codant les ADNc des variants de la famille 1 est effectuée selon le tableau 1 à l'aide de PCR d'assemblage et ligation ou par la technique Infusion utilisant les amorces listées dans le tableau 2. La matrice utilisée pour les PCR1 et 2 est le vecteur OptiCHO-FXWT4HS. Les produits de PCR sont traités à la DpnI pour digérer l'ADN parental. Les amplicons d'intérêt sont purifiés par Nucléospin extract.

Pour les molécules OptiCHO-FX WTF1D, OptiCHO-FX WTF1G et OptiCHO-FX WTF1I, les amplicons purifiés des PCR 1 et 2 ont été utilisés comme matrice et assemblés par PCR d'assemblage selon le tableau 1. Les amplicons des PCR3 purifiés ainsi que le vecteur digéré par NheI et SwaI, sont assemblés par ligation à la T4 ligase.

**Tableau 1**

| | PCR1 | | PCR2 | | PCR 3 d'assemblage après digestion des PCR1 et 2 | |
|---|---|---|---|---|---|---|
| variant | amorces | Taille amplicon | amorces | Taille amplicon | amorces | Taille amplicon |
| FXWT4HS F1D | 5'FXWT+3'FX1d | 720 | 5'FXld+3'FX SwaI | 842 | 5'FX WT+3'Fx SwaI | 1551 |
| FXWT4HS F1G | 5'FXWT+3'FX1g | 716 | 5'FX1g+3'FX SwaI | 841 | 5'FX WT+3'Fx SwaI | 1551 |
| FXWT4HS F1I | 5'FXWT+3'FX1i | 716 | 5'FX1i+3'FX SwaI | 838 | 5'FX WT+3'Fx SwaI | 1551 |

**Tableau 2**

| **amorces** | **SEQ ID NO :** | **séquences** |
|---|---|---|
| 3'FX1a | 41 | 5'-TCCTTCTGCCAGGAAGTCCTGTGTCTGGTTGAAGTCCAGCAGGTCA-3' |
| 3'FX1b | 42 | |
| 3'FX1c | 43 | 5'-TGGCGTTGGTGGCCTTCTGTGTCTGGTTGAAGTCCAGCAGGTCAA-3' |
| 3'FX1d | 44 | |
| 3'FX1e | 45 | 5'-TGGTCAGCTTGCTGGTGCCTCTTTCAGGCTGTGTCTGGTTGA-3' |
| 3'FX1f | 46 | 5'-TGGTGAAGTCGTTGAAGCCTCTTTCAGGCTGTGTCTGGTTGAAG-3' |
| 3'FX1g | 47 | 5'-TGGTCATGCTGCTCAGGCCTCTTTCAGGCTGTGTCTGGTTGAAGT-3' |
| 3'FX1h | 48 | 5'-TGGTCAGGCTGGGAGGGCCTCTTTCAGGCTGTGTCTGGTTGAAG-3' |
| 3'FX1i | 49 | 5'-TCTGGCCGCAGGACAGGCCTCTTTCAGGCTGTGTCTGGTTGAAG-3' |
| 3FX-2a | 50 | |
| 3FX-2b | 51 | |
| 3FX-2c | 52 | |
| 3FX-2d | 53 | |
| 3FX-2e | 54 | 5'-TCAGCTTGCTGGTCCTAGTCAGATTGTTATCGCCTCTTTCAGGC-3' |
| 3FX-2f | 55 | 5'-GGTGAAGTCGTTGAACCTAGTCAGATTGTTATCGCCTCTTTCAGGC-3' |
| 3FX-2g | 56 | 5'-ATGCTGCTCAGCCTAGTCAGATTGTTATCGCCTCTTTCAGGC-3' |
| 3FX-2h | 57 | 5'-GTCAGGCTGGGAGGCCTAGTCAGATTGTTATCGCCTCTTTCAGGC-3' |
| 3FX-2i | 58 | 5'-TCTGGCCGCAGGACAGCCTAGTCAGATTGTTATCGCCTCTTTCAGGC-3' |
| 5'FX1a | 59 | 5'-GCAGAAGGAGGAGGAGTGAGGATCGTGGGAGGACAGGAGTGCA-3' |
| 5'FX1b | 60 | 5'-AGGGCCTGACCCCTAGGATCGTGGGAGGACAGGAGTGCAAGGA-3' |
| 5'FX1cbis | 61 | |
| 5'FX1d | 62 | 5'-CAGGCCACCCTGAGCCCTAGGATCGTGGGAGGACAGGAGTGCAAG-3' |
| 5'FX1e | 63 | 5'-ACCAGCAAGCTGACCAGGATCGTGGGAGGACAGGAGTGCAAGGA-3' |
| 5'FX1f | 64 | 5'-CAACGACTTCACCAGGATCGTGGGAGGACAGGAGTGCAAGGA-3' |
| 5'FX1g | 65 | 5'-TGAGCAGCATGACCAGGATCGTGGGAGGACAGGAGTGCAAGGA-3' |
| 5'FX1h | 66 | 5'-CCTCCCAGCCTGACCAGGATCGTGGGAGGACAGGAGTGCAAGGA-3' |
| 5'FX1i | 67 | 5'-TGTCCTGCGGCCAGAGGATCGTGGGAGGACAGGAGTGCAAGGA-3' |
| 5'fusion FX | 68 | 5'-TCTTCCATTTCAGCTAGCAAGCTTGCCGCCAC-3' |
| 3fusionF X | 69 | 5'-AGCTCTAGACAATTGATTTAAATGGATCCTCACTTGCCGTC-3' |
| 5'FXWT | 70 | 5'-ACCAGCTGCTAGCAAGCTTGCCG-3' |
| 3'FX-SwaI | 71 | 5'-GAAACTATTTAAATGGATCCTCACTTGCCGTCAATCAGC-3' |
| 3FXF3 | 72 | 5'-CCAGGTAATGCTATCAGCCACTGACCTTTTGCGCCTCTC-3' |
| 5FXF3 | 73 | 5'-TCAGTGGCTGATAGCATTACCTGGAAACCTTATGACGC-3' |
| 3FXF3bis | 74 | 5'-GCTAGTTGCCTGAGCCACTGACCTTTTG-3' |
| 5FXF3bis | 75 | 5'-GCTCAGGCAACTAGCGATAGCATTACCTGGAAACCTTATGACGC-3' |
| 5'ef1a | 38 | 5'-GTGGAGACTGAAGTTAGGCCAG-3' |
| 3FX | 39 | 5'-CTTCATTTCCTCCAGGAAAGAGTTGGC-3' |
| 2BGHPA | 40 | 5'-CAGATGGCTGGCAACTAGAA-3' |

Pour les molécules OptiCHO-FX WTF1a, OptiCHO-FX WTF1b, OptiCHO-FX WTF1c, OptiCHO-FX WTF1e, OptiCHO-FX WTF1f, et OptiCHO-FX WTF1h, les amplicons purifiés des PCR 1 et 2 ont été générés par PCR selon les conditions du tableau 3. Les amplicons purifiés des PCR 1 et 2 sont assemblés par Infusion avec le vecteur préalablement digéré par NheI et SwaI et purifié par nucleospin Extract.

**Tableau 3 :**

| | PCR1 | | PCR2 | |
|---|---|---|---|---|
| variant | amorces | Taille amplicon | amorces | Taille amplicon |
| FX4hsF1a | 5'fusionFX + 3'FX1a | 718 | 5'FX1A + 3fusionFX | 842 |
| FX4hsF1b | 5'fusionFX3'F + X1b | 722 | 5'FX1b + 3fusionFX | 838 |
| FX4hsF1c | 5'fusionFX + 3'FX1c | 718 | 5'FX1cbis + 3fusionFX | 859 |
| FX4hsF1e | 5'fusionFX + 3'FX1e | 714 | 5'FX1e + 3fusionFX | 839 |
| FX4hsF1f | 5'fusionFX + 3'FX1f | 716 | 5'FX1f + 3fusionFX | 814 |
| FX4hsF1h | 5'fusionFX + | 716 | 5'FX1h + 3fusionFX | 839 |
| | 3'FX1h | | | |

Pour chaque variant le vecteur final est inséré par transformation bactérienne dans des bactéries Top10. Après amplification bactérienne en présence d'ampicilline, des colonies bactériennes sont prélevées sur boite de Pétri et criblées par PCR pour la recherche d'un amplicon de 296 pb avec les amorces 5'efla et 3FX, signe de la présence de l'insert codant le FX dans le vecteur OptiCHO. Les vecteurs OptiCHO-FX WTF1a à OptiCHO-FX WTF1i sont séquencés avec les amorces :
- 5'efla
- 2BGHPA

### 2.4- Préparation des variants de la famille 2

La préparation des inserts codant les ADNc des variants de la famille 2 est effectuée selon le tableau 4 par la technique de PCR et assemblage par Infusion en utilisant les amorces listées dans le tableau 2. La matrice utilisée pour les PCR1 et 2 est le vecteur OptiCHO-FXWT4HS. Les produits de PCR sont traités à la DpnI pour digérer l'ADN parental. Les amplicons sont purifiés par nucleospin extract. Les amplicons purifiés des PCR 1 et 2 sont assemblés par Infusion avec le vecteur OptiCHO préalablement digéré par NheI et SwaI et purifié par nucleospin Extract.

**Tableau 4 :**

| | PCR1 | | | PCR2 | | |
|---|---|---|---|---|---|---|
| | Amorces 5' | Amorces 3' | Taille amplicon | Amorces 5' | Amorces 3' | Taille amplicon |
| Fx-a | 5'fusionF X | 3FX-2a | 781 | 5'FX-1a | 3fusion FX | 851 |
| Fx-b | 5'fusionF X | 3FX-2b | 758 | 5'FX-1b | 3fusion FX | 847 |
| Fx-c | 5'fusionF X | 3FX-2c | 755 | 5'FX-1c | 3fusion FX | 850 |
| Fx-d | 5'fusionF X | 3FX-2d | 755 | 5'FX-1d | 3fusion FX | 851 |
| Fx-e | 5'fusionF X | 3FX-2e | 748 | 5'FX-1e | 3fusion FX | 848 |
| Fx-f | 5'fusionF X | 3FX-2f | 750 | 5FX-1f | 3fusion FX | 846 |
| Fx-g | 5'fusionF X | 3FX-2g | 746 | 5'FX-1g | 3fusion FX | 847 |
| Fx-h | 5'fusionF X | 3FX-2h | 749 | 5'FX-1h | 3fusion FX | 848 |
| Fx-i | 5'fusionF X | 3FX-2i | 751 | 5'FX-1i | 3fusion FX | 847 |

Pour chaque variant le vecteur final est inséré par transformation bactérienne dans des bactéries Top10. Après amplification bactérienne en présence d'ampicilline, des colonies bactériennes sont prélevées sur boite de Pétri et criblées par PCR pour la recherche d'un amplicon de 296 pb avec les amorces 5'efla et 3FX, signe de la présence de l'insert codant le FX dans le vecteur OptiCHO. Les vecteurs OptiCHO-FX WTF2a à OptiCHO-FX WTF2i sont séquencés avec les amorces :
- 5'efla
- 2BGHPA

### 2.5.- Préparation des Variants de la famille 3

La famille 3 contient des délétions dans le peptide d'activation qu'il convient de préparer avant de pouvoir y insérer les sites de clivage enzymatique. A ce titre, deux vecteurs intermédiaires sont préparés OptiCHO FXWT F3AD pour les variants de la famille 3 F3a à F3d et OptiCHO FXWT F3EI pour les variants de la famille 3 F3e à F3i. Les inserts des vecteurs intermédiaires sont construits par PCR d'assemblage selon le tableau 5 en utilisant le vecteur OptiCHO FXWT4HS-gs pour matrice et les amorces 5'FXWT, 3'FX-SwaI, 3FXF3, 5FXF3, 3FXF3bis, 5FXF3bis listées dans le tableau 2.

**Tableau 5 :**

| | PCR1 | | PCR2 | | PCR 3 d'assemblage après digestion des PCR1 et 2 | |
|---|---|---|---|---|---|---|
| Vecteur intermédiaire | amorces | Taille amplicon | amorces | Taille amplicon | amorces | Taille amplicon |
| OptiCHO FXWT F3AD | 5'FXWT + 3FXF3 | 598 | 3FX-SwaI + 5FXF3 | 947 | 5'FXWT + 3FX-SwaI | 1521 |
| OptiCHO FXWT F3EI | 5'FXWT + 3FXF3bis | 595 | 3FX-SwaI + 5FXF3bis | 953 | 5'FXWT + 3FX-SwaI | 1533 |

Les produits des PCR d'assemblage sont purifiés par Nucleospin extract avant d'être digérés, tout comme le vecteur destination OptiCHO, par les enzymes NheI et SwaI. Ils sont à nouveau purifiés sur Nucleospin extract après digestion.

L'insert et le vecteur sont ligués ensemble par la T4 ligase avant que le produit de ligation ne soit intégré dans des bactéries compétentes Top10. Après amplification bactérienne en présence d'ampicilline, des colonies bactériennes sont prélevées sur boite de Pétri et criblées par PCR pour la recherche d'un amplicon de 296 pb avec les amorces 5'efla et 3FX signe de la présence de l'insert codant le FX variant dans le vecteur OptiCHO.

Les vecteurs intermédiaires OptiCHO FXWT F3AD et OptiCHO FXWT F3EI sont séquencés avec les amorces :
- 5'efla
- 3FX
- 2BGHPA

A l'exception de la molécule OptiCHO FXWT F3A, la préparation des inserts codant les ADNc des variants de la série 3 est effectuée selon le tableau 6 par PCR d'assemblage en utilisant les amorces listées dans le tableau 2. La matrice utilisée pour les PCR1 et 2 est le vecteur OptiCHO FXWT F3AD pour les variants de la famille 3, F3a à F3d et OptiCHO FXWT F3EI pour les variants de la famille 3 F3e à F3i. Les amplicons sont purifiés par nucleospin extract. Les amplicons purifiés des PCR 1 et 2 sont assemblés par PCR d'assemblage avec le vecteur OptiCHO préalablement digéré par NheI et SwaI et purifié par nucleospin Extract.

**Tableau 6 :**

| | PCR1 | | PCR2 | | PCR 3 d'assemblage après digestion des PCR1 et 2 | |
|---|---|---|---|---|---|---|
| | Amorces | Taille amplicon | Amorces | Taille amplicon | Amorces | Taille amplicon |
| Fx-b | 5'FXWT + 3FX-2b | 723 | 5FX-1b + 3'FX-SwaI | 838 | 5'FXWT +3'FX-SwaI | 1551 |
| Fx-c | 5'FXWT + 3FX-2c | 720 | 5FX-1c + 3'FX-SwaI | 841 | 5'FXWT + 3'FX-SwaI | 1551 |
| Fx-d | 5'FXWT + 3FX-2d | 720 | 5FX-1d + 3'FX-SwaI | 842 | 5'FXWT + 3'FX-SwaI | 1551 |
| Fx-e | 5'FXWT + 3FX-2e | 713 | 5FX-1^{e} + 3'FX-SwaI | 725 | 5'FXWT + 3'FX-SwaI | 1551 |
| Fx-f | 5'FXWT + 3FX-2f | 715 | 5FX-1f + 3'FX-SwaI | 727 | 5'FXWT + 3'FX-SwaI | 1551 |
| Fx-g | 5'FXWT + 3FX-2g | 711 | 5FX-1g + 3'FX-SwaI | 723 | 5'FXWT + 3'FX-SwaI | 1551 |
| Fx-h | 5'FXWT + 3FX-2h | 714 | 5FX-1h +3'FX-SwaI | 726 | 5'FXWT + 3'FX-SwaI | 1551 |
| Fx-i | 5'FXWT + 3FX-2i | 716 | 5FX-1i + 3'FX-SwaI | 728 | 5'FXWT + 3'FX-SwaI | 1551 |

Les produits des PCR d'assemblage sont purifiés par Nucleospin extract avant d'être digérés, tout comme le vecteur de destination OptiCHO, par les enzymes NheI et SwaI. Ils sont à nouveau purifiés sur Nucleospin extract après digestion.

L'insert et le vecteur sont ligués ensemble par la T4 ligase avant que le produit de ligation ne soit intégré dans des bactéries compétentes Top10. Après amplification bactérienne en présence d'ampicilline, des colonies bactériennes sont prélevées sur boite de Pétri et criblées par PCR pour la recherche d'un amplicon de 296 pb avec les amorces 5'efla et 3FX, signe de la présence de l'insert codant le FX variant dans le vecteur OptiCHO.

Les vecteurs finaux de la famille 3 sont séquencés avec les amorces :
- 5'efla
- 2BGHPA

### Exemple 2 : Production des facteurs X recombinants

### 1 - Protocole expérimental

### 1.1 - Réactifs

Milieu de culture ProCHO4 ((Lonza) et Freestyle™ F17 (Gibco)
L-glutamine (Gibco)
Milieu de transfection des cellules CHO-S : Opti-Pro SFM (Gibco).
Milieu de transfection des cellules HEK : Opti-MEM (Gibco).
Vitamine K1 (Sigma).

### 1.2 - Protocole

Le facteur X de type sauvage et ses variants sont produits dans des cellules eucaryotes CHO-S ou HEK-293-Freestyle (Invitrogen) en expression transitoire.

Les cellules CHO-S sont cultivées en milieu ProCHO4 et les cellules HEK 293F en milieu F17, supplémentés respectivement avec 4 mM et 8 mM de L-glutamine. Les 2 lignées cellulaires sont cultivées en conditions agitées à 135 rpm en atmosphère contrôlée (8% CO₂) à 37°C. La veille du jour de transfection, les cellules sont ensemencées à une densité de 7.10⁵ cellules/ml.

Le jour de la transfection, l'ADN (20-30 µg) et 30µg d'agent de transfection (AT) sont pré-incubés séparément en milieu Opti-Pro pour CHO-S et Opti-MEM pour HEK 293F durant 5 minutes puis mélangés et incubés durant 20 minutes pour permettre la formation du complexe ADN/AT. L'ensemble est ajouté à une préparation cellulaire de 1.10⁶ cellules/ml dans un volume de 30 ml.

Dans le cas des co-transfections des FX avec la vitamine K époxyde-reductase native (VKOR), les 2 vecteurs sont ajoutés à différents ratios pour obtenir une quantité totale d'ADN de 20-30µg. L'enzyme VKOR permet une production de FX actif dans HEK en optimisant la gamma-carboxylation. Immédiatement après la transfection, la vitamine K1 (5 µg/ml) est ajoutée dans le milieu. Les taux de transfections sont évalués le lendemain de la transfection à l'aide d'un plasmide contrôle exprimant la GFP (Green Fluorescent Protein). Les productions sont réalisées en mode «batch » durant 7 jours. En fin de production, les cellules et le surnageant sont séparés par centrifugation. Les cellules sont éliminées et le surnageant est filtré puis congelé.

### Exemple 3 : Mesure de la concentration en facteur X

### 1- Protocole expérimental

La concentration en facteur X est mesurée par l'intermédiaire de l'ELISA commercial Zymutest Factor X (HYPHEN BioMed) en suivant les recommandations du fabricant. Les concentrations sont mesurées en triplicat en utilisant des valeurs d'antigène situées dans la zone linéaire de détection de l'essai. Pour s'assurer que les mutations introduites ne perturbent pas la mesure de la concentration, les FX sont déposés en quantité identique et révélés par immunoblotting avec un anticorps polyclonal différent de celui utilisé en ELISA (Polyclonal antibody anti-human FX (CRYOPEP cat n°PAHFX-S) ou par coloration après SDS-PAGE (données non montrées).

### 2 - Résultats

### 2.1- Expression des facteurs X variants exprimés transitoirement dans CHO

Les concentrations des facteurs X (FX) variants présents dans les surnageants des cellules CHO-S transfectées avec les ADNc codant pour les familles 1 à 3 ont été mesurées par l'ELISA commercial Zymutest Factor X (figure 3).

Comme attendu, les surnageants de cellules CHO non-transfectées (control) ne contiennent pas de FX. La transfection avec les vecteurs codant pour les différents FX permet d'obtenir des taux allant de 0.5 à 3.06 µg/ml. Il n'y a pas de différence majeure dans l'expression des FX des différentes familles. Au maximum, le FX-IIa de la famille 3 est exprimé 2.1X plus fortement (2.64 µg/ml) que celui de la famille 1 (1.26 µg/ml). Certaines constructions permettent d'obtenir du facteur X en concentration plus importante que le facteur X de type sauvage : ce sont les constructions FX-IIa (famille 3) et les FX-PAR1 (famille 1 et 3). Le FX-Kal3 semble diminuer la production du FX.

### 2.2- Expression des facteurs X variants exprimés transitoirement dans HEK

Les concentrations des facteurs X variants présents dans les surnageants des cellules HEK293S transfectées avec les ADNc codant pour les familles 1 à 3 ont été mesurées par l'ELISA commercial Zymutest Factor X (figure 4). Comme attendu, les surnageants de cellules HEK293S non transfectées (control) ne contiennent pas de FX. Les molécules de la famille 1 ont été produites à un taux proche de celui du FX-WT (de 0.14 à 1.64 µg/ml). Seule la molécule FX-PAR1 est produite à un taux plus élevée. La molécule FX-Kal3 reste la molécule la moins produite. La famille 2 a été produite de manière similaire pour toutes les constructions avec des valeurs d'expression de 1.79 à 2.54 µg/ml sauf pour la construction FX-Kal3 produite à un taux plus faible (0.66 µg/ml). La famille 3 a été produite à des taux plus faibles de 0.2 à 1.2 µg/ml.

*En conclusion, certaines constructions de facteurs X variants permettent de produire le FX à des taux plus importants que le FX-WT :*
- *ce sont dans les CHO, les constructions FX-IIa (famille 3) et les FX-PAR1 (familles 1 et 3) et*
- *dans les HEK, le FX-PAR1 de la famille 1.*

### Exemple 4 : Mesure de l'activité coagulante des facteurs X variants produits dans CHO-S

### 1 - Protocole expérimental

L'activité chronométrique des FX variants produits par les cellules CHO-S a été mesurée grâce à un automate STAR (Stago) en présence de plasma déficient en FX.

Le plasma déficient en FX, la néoplastine et le tampon Owren-Koller proviennent de chez Stago (Asnières, France).

Le surnageant de culture concentré est dilué au 1/10 en tampon Owren-Koller avant d'être ajouté au plasma déficient en FX. Le mélange est incubé 240 secondes à 37°C puis le temps de prothrombine (TP) est déclenché par l'ajout de 100 µl de néoplastine. Les temps de coagulation (en s.) sont transformés en activité spécifique du FX.

### 2- Résultats

Les surnageants concentrés issus des différentes transfections dans CHO ont été évalués pour leur capacité à compenser un déficit en facteur X. Les surnageants ont été incubés dans un plasma déficient en FX et un test TP a été pratiqué. Les temps de coagulation (en s.) ont été transformés en activité spécifique (AS ; en seconde par µg de protéine) puis, le pourcentage d'activité spécifique par rapport au FX de type sauvage a été calculé (figure 5). Les résultats sont cohérents entre les différentes familles. Ce résultat indique que les différences de comportement proviennent principalement des sites de clivage et non de la manière dont ils sont clonés.
Les constructions peuvent être classées en trois catégories : une première catégorie dont l'AS est similaire à celle du FX-WT (contient le FX-control+), une seconde catégorie dont l'AS est diminuée par rapport à celle du contrôle (contient FX-IIa, FX-PAR1, FX-PAR1M et FX-Kal3) et une troisième catégorie dont l'activité en absence de FX est supérieure à celle du FX-WT (contient FX-FXIa1 et 2, FX-Kal1 et 2). Il est à noter que la construction FX-Kal2 de la famille 3 n'a pas pu être analysée pour des raisons techniques (* sur le graphe).

*Ces résultats indiquent que les modifications introduites et qui forment la famille 3 (FX-FXIa1 et 2, FX-Kal1 et 2) confèrent aux molécules de facteur X une capacité à coaguler plus efficace que le facteur X de type sauvage en absence de facteur X endogène.*

### Exemple 5: Mesure de l'activation des facteurs X variants produits dans CHO-S par la fraction de venin RVV-X

### 1- Protocole expérimental

L'activation des FX variants produits par les cellules CHO-S a été mesurée suite à l'incubation des surnageants de culture en présence de la fraction anti-facteur X du venin de la vipère de Russell (RVV-X). Le facteur X activé de contrôle, la fraction X du venin (RVV-X) et le substrat pNAPEP 1025 proviennent de Haematologic Technologies (Cryopep Montpellier, France).

L'activation a été étudiée à 37°C dans le tampon suivant : 25 mM HEPES, pH 7.4, 0.175 M NaCl, 5 mM CaCl₂, 5 mg/ml BSA. Pour des concentrations de 0 à 100 nM de FX, une concentration de 200 mU/ml de RVV-X a été utilisée. Après 5 min d'incubation, la réaction est arrêtée dans le tampon 50 mM Tris, pH 8.8, 0.475 M NaCl, 9 mM EDTA. La quantité de FXa générée est suivie en mesurant la vitesse d'hydrolyse du substrat pNAPEP 1025 (250 µM) à 405 nm.

### 2 - Résultats

Les surnageants de CHO exprimant les variants de la famille 1 ont été incubés avec le RVV-X. La génération de FXa a été mesurée suite à ce traitement à partir de différentes concentrations en FX. La présence de FXa est quantifiée par la vitesse d'apparition du produit du pNAPEP 1025 en solution (en mUDO/min). Cette génération est le reflet de la reconnaissance et du clivage des FX par le RVV-X ainsi que de la capacité du FXa généré à reconnaitre le substrat du FX. La moyenne des vitesses d'apparition est faite pour les différentes concentrations initiales de FX et cette valeur est ramenée en pourcentage de la valeur du FX-WT.

L'analyse de la famille 1 montre pour partie des résultats similaires avec les résultats obtenus en TP, notamment pour les FX- control+, FX-IIa, FX-Kal1, FX-Kal2 et FX-Kal3 (figure 6). Les molécules FX-XIa1 et 2 montrent une activité mais cette fois-ci non supérieure au FX-WT. En revanche les molécules FX-PAR1 et FX-PAR1M démontrent une activité significative et supérieure de celle observée en TP.

*Ces résultats indiquent que les FX-PAR1M et FX-Kal2 de la famille 1 sont plus efficacement activés par RVV-X que le FX-WT.*

### Exemple 6 : Mesure en temps de génération de thrombine (TGT) de la capacité procoagulante des facteurs X variants de la famille 2 : Activation de la voie extrinsèque de la coagulation (FT 1pM/PL 4µM) en plasma déficient en FVIII

### 1 - Protocole expérimental

### 1.1 - Réactifs

Thrombin calibrator, PPP reagent low, CK-Prest, Fluca Kit (Fluo-buffer + Fluo-substrat), le PNP et le plasma déficient en FIX proviennent de chez Stago (Asnières, France). Le plasma déficient en FX provient de chez Cryopep (Montpellier, France). Le plasma déficient en FVIII provient de chez Siemens Healthcare (Marburg, Germany). Le FX humain (cat n° HCX-0050), le FXa humain (cat n° HCXA-0060,) proviennent de chez Haematologic Technologies Inc. (Burlington, Vt, USA). Le Facteur VIII recombinant humain de contrôle provient de chez Baxter (Recombinate) (Maurepas, France).

### 1.2 - Protocole

On considère que 1 unité de FX (1 U /ml) = 10 µg/mL dans le plasma, correspondant à 100% de taux de FX dans le plasma.

Le test de génération de thrombine consiste à activer la coagulation *ex vivo* soit à l'aide d'un mélange de facteur tissulaire et de phospholipides (activation de la voie extrinsèque), soit à l'aide de céphaline (activation de la voie intrinsèque) et à mesurer ensuite la concentration de thrombine générée au cours du temps.

Les tests de génération de thrombine sont réalisés sur 80 µl d'un pool de plasma contenant éventuellement les surnageants de cellules ou les contrôles, en présence de 20 µl de réactif PPP (Stago) contenant au final 1 pM de Facteur Tissulaire (FT) et 4 µM de phospholipides (PL). Différents plasmas peuvent être utilisés, normal, déficient en facteur X, déficient en facteur VIII ou déficient en facteur IX.

La réaction est démarrée par l'ajout de 20 µL de Fluca-kit (substrat + CaCl₂) qui constitue le début de la mesure de l'apparition de thrombine. L'apparition de fluorescence est mesurée sur un fluorimètre de type Fluoroskan Ascent (ThermoLabsystems) à une longueur d'onde d'excitation de 390 nm et à une longueur d'émission de 460 nm. Les thrombinogrammes (courbes représentant l'intensité de fluorescence en fonction du temps) sont ensuite analysés grâce au logiciel Thrombinoscope™ qui transforme la valeur de fluorescence en nM de thrombine par calcul comparatif.

### 2 - Résultats

Les surnageants ont été concentrés environ 20 fois sur VivaSpin20-30 kDa Sartorius à 2500g pendant au moins 1h jusqu'à l'obtention de la concentration désirée. Les plasmas Unicalibrator, ainsi que les plasmas déficients en FVIII reconstitué par 0, 0.1 ou 1 U/ml sont utilisés comme témoins.

Comme attendu suite à l'activation de la coagulation par le facteur tissulaire, le plasma déficient en FVIII donne le signal le plus faible, correspondant au bruit de fond de l'expérience. Le plasma Unicalibrator fourni un signal plus faible que le plasma déficient en FVIII reconstitué par les concentrations de FVIII (0.1 ou 1 U/ml).En revanche, la reconstitution d'un plasma déficient en FVIII par le FX-WT ne permet pas de générer des quantités suffisantes de thrombine. Cette reconstitution est à peine plus forte que le plasma déficient seul (figure 7, tableau 7).

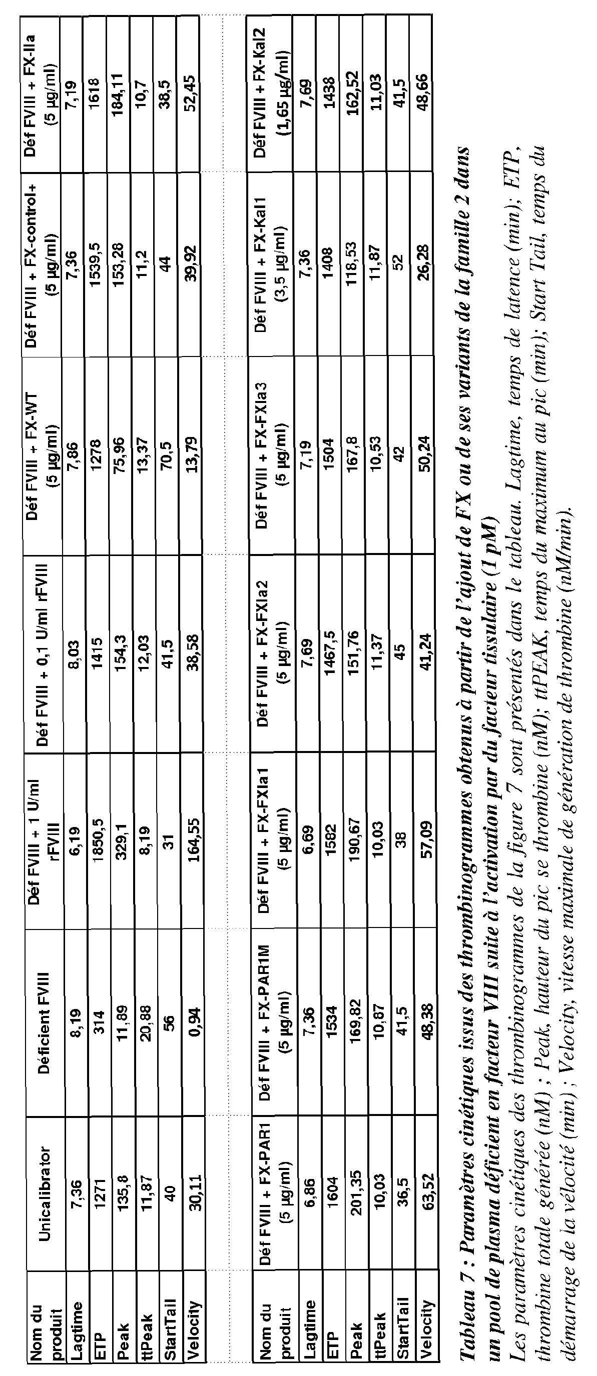

Toutes les molécules de FX variants donnent un signal plus important que le FX-WT, suggérant que leur capacité à générer de la thrombine en absence de FVIII est significativement augmentée par rapport au FX-WT (tableau 7). Les célérités des FX mutants de la famille 2 oscillent entre 2 et 4.5X celle du FX-WT (figure 9, tableau 7)). A noter qu'une célérité de 2X est obtenue avec une quantité de FX-Kal2 30% plus faible (3.5 µg/ml au lieu de 5 µg/ml).

*Les mutants permettant de générer les quantités de thrombine les plus importantes en absence de FVIII sont dans l'ordre FX-PAR1, FX-FXIa1, FX-IIa et FX-Kal1.*

### Exemple 7 : Mesure en temps de génération de thrombine (TGT) de la capacité procoagulante des facteurs X variants : voie intrinsèque de la coagulation (céphaline seule) en plasma déficient en FVIII

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma normal contenant éventuellement les surnageants de cellules et les contrôles en présence de 20 µl de céphaline (CK-Prest reconstitué avec 1 mL d'H₂O distillée) et de 20 µL fluca-kit (substrat + CaCl₂). Les plasmas utilisés sont un plasma normal et un plasma déficient en facteur VIII.

### 2 - Résultats

Les surnageants utilisés dans l'exemple 6 ont été analysés en TGT après activation par la céphaline en utilisant les mêmes témoins.

Les témoins se comportent de manière attendue, le plasma déficient en FVIII ne permet pas de génération de IIa et un gradient d'efficacité est retrouvé en augmentant la dose de FVIII (tableau 8).

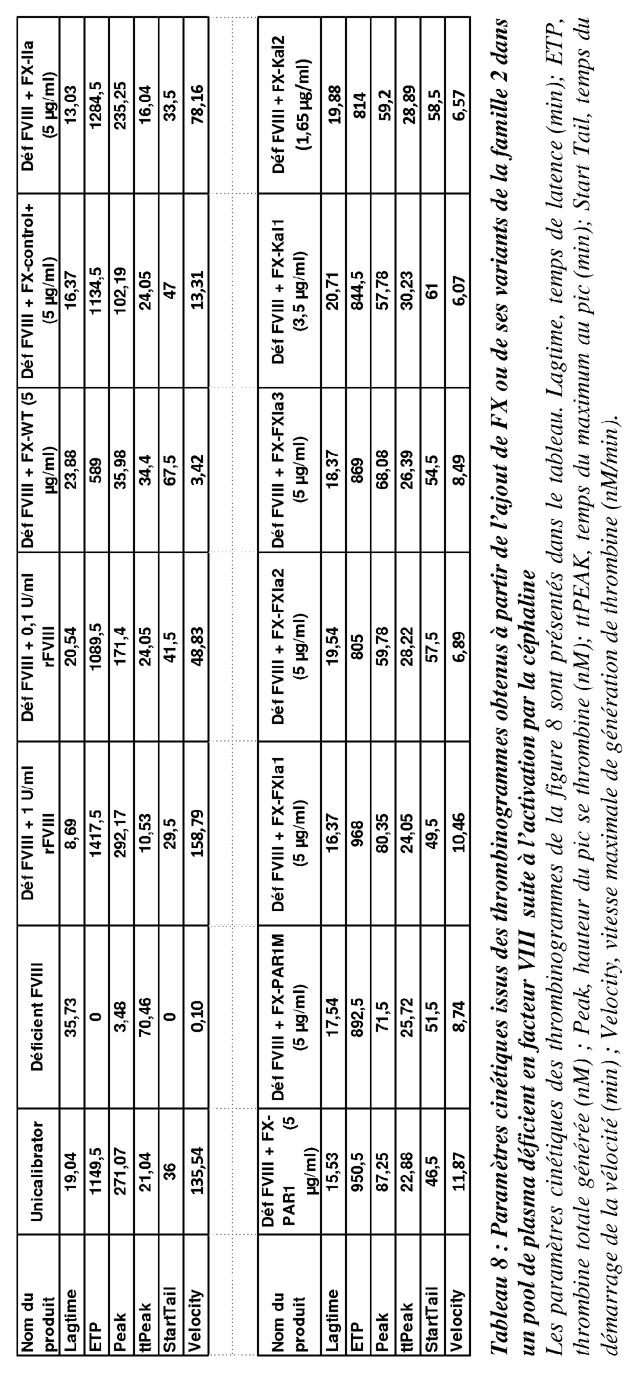

L'analyse de la famille 2 montre comme attendu que le FX de type sauvage est la molécule de FX la moins active montrant une faible activité résiduelle (3.4 nM/s). Toutes les autres molécules montrent une capacité à générer de la thrombine plus importante (tableau 8). Les molécules FX-Kal-1 à 3, FX-FXI-2 et FX-PAR1M montrent une activité un peu plus forte (6.6 à 8.7 nM/s) mais moins que les constructions FX- control+, FX-PAR1 et FX-FXI-1 (10.5-13.3 nM/s). En revanche la molécule FX-IIa permet d'obtenir un signal compris entre les valeurs obtenues pour 0.1 et 1 U/ml de FVIII (78.2 nM/s) corrigeant de manière très efficace la déficience en FVIII. Ces différences de célérités montrent pour les mutants une efficacité supérieure de 1.8X (mais pour le mutant FX-Kal2 à 3.5 µg/ml) à 22.8X (pour le mutant FX-IIa) à générer de la thrombine (figure 9).

*En conclusion, plusieurs mutants de FX ont la capacité à restaurer la coagulation en absence de FVIII suite à l'activation du plasma par le facteur tissulaire et la céphaline.*

### Exemple 8: Mesure en temps de génération de thrombine (TGT) de la capacité procoagulante des facteurs X variants de la famille 2: Activation de la voie extrinsèque de la coagulation (FT 1pM/PL 4µM) en plasma déficient en FIX

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma normal contenant éventuellement les surnageants de cellules et les contrôles en présence de 1 pM de Facteur Tissulaire (FT) et 4 µM de phospholipides (PL) et de 20µL fluca-kit (substrat + CaCl₂). Les plasmas utilisés sont soit normal soit déficient en facteur IX.

### 2 - Résultats

Les surnageants utilisés dans l'exemple 6 ont été analysés en TGT après activation par le facteur tissulaire en utilisant comme témoins, un plasma normal (Unicalibrator) ou un plasma déficient en FIX reconstitué par 0, 0.1 ou 1 U/ml de FIX plasmatique (figure 10, tableau 9).

Le plasma déficient en FIX est négatif (il ne permet pas de génération de IIa) et sa reconstitution avec du FIX lui permet de générer des quantités importante de thrombine. Il n'y a cependant pas de différence quantitative entre les deux concentrations de FIX utilisées, ce qui suggère qu'elles permettent de former toutes les deux une quantité maximale de IIa (tableau 9).

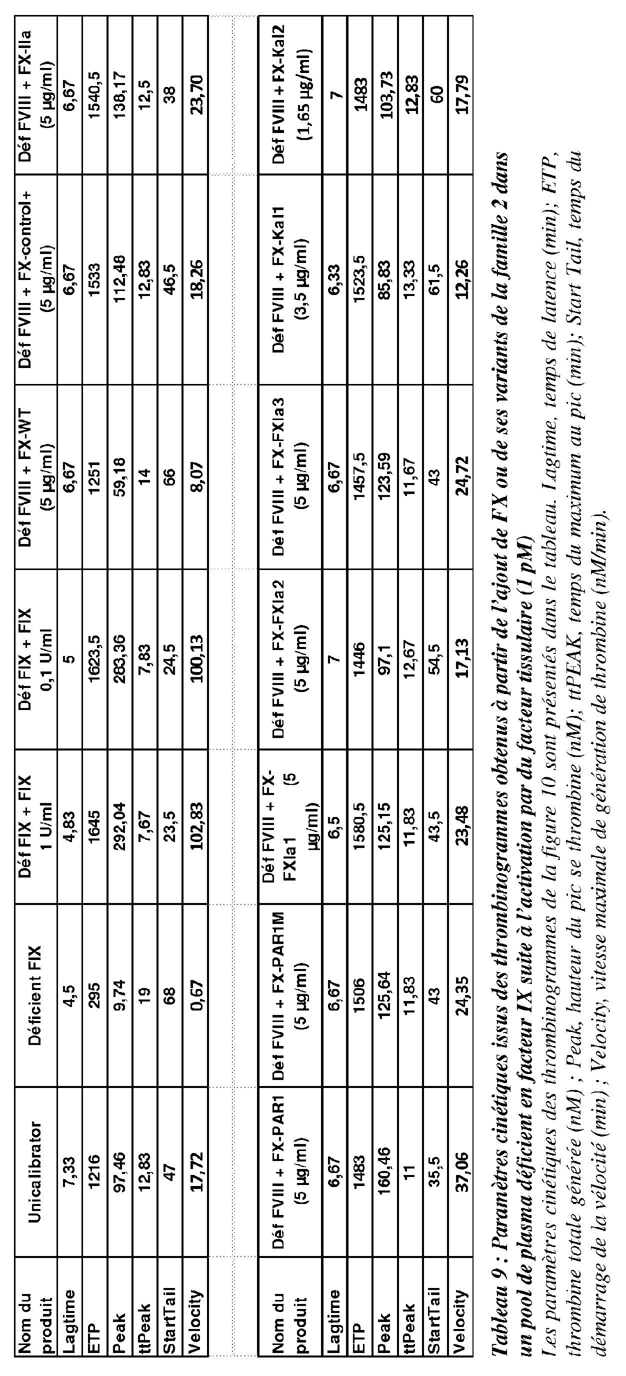

Dans cet essai, tous les mutants permettent de générer des quantités de IIa plus importantes que le FX-WT. Les différences sont cependant moins fortes qu'en absence de FVIII : ainsi la célérité varie de 1,50X (pour FX-Kal2 à 3.5 µg/ml) à 4,60X pour (FX-PAR1). En plus de ce mutant, les molécules FX-IIa, FX-PAR1M, FX-FXIa1 et FX-Kal1 sont les plus efficaces (figure 12, tableau 9).

### Exemple 9: Mesure en temps de génération de thrombine (TGT) de la capacité procoagulante des facteurs X variants de la famille 2: Activation de la voie intrinsèque de la coagulation (céphaline) en plasma déficient en FIX

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma normal contenant éventuellement les surnageants de cellules et les contrôles en présence de céphaline (CK-Prest reconstitué avec 1 mL d'H₂O distillée) et de 20 µL fluca-kit (substrat + CaCl₂). Les plasmas utilisés sont soit normal soit déficient en facteur IX.

### 2 - Résultats

Les surnageants utilisés dans l'exemple 6 ont été analysés en TGT après activation par la céphaline en utilisant comme témoins, un plasma normal (Unicalibrator) et un plasma déficient en FIX reconstitué par 0, 0.1 ou 1 U/ml de FIX plasmatique (figure 11, tableau 10).

Le plasma déficient en FIX est négatif (il ne permet pas de génération de IIa) et sa reconstitution avec du FIX lui permet de générer des quantités importantes de thrombine. Il n'y a cependant pas de différence quantitative entre les deux concentrations de FIX utilisées, ce qui suggère qu'elles forment une quantité maximale de IIa (tableau 10).

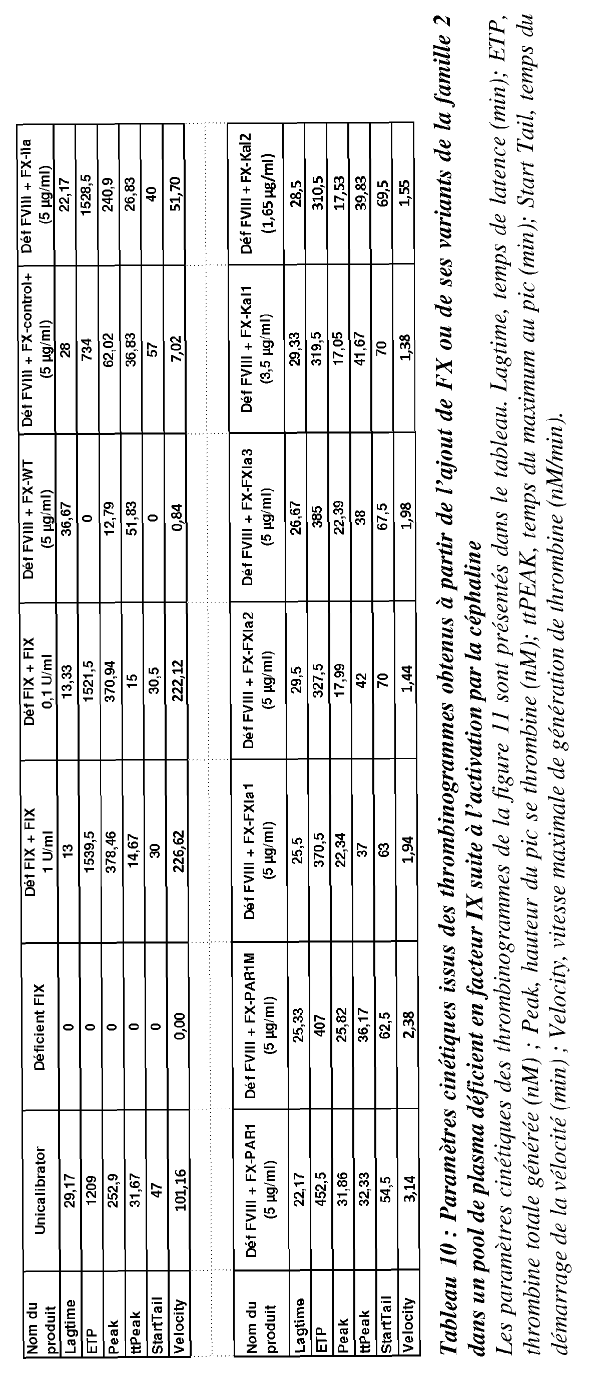

Suite à l'induction de la coagulation par la céphaline seulement trois molécules permettent de générer significativement plus de thrombine que le FX-WT, se sont les FX-IIa, FX- control+ et FX-PAR1 avec une célérité 61X, 8,3X et 3,8X plus forte que celle du FX-WT respectivement (figure 12, tableau 10). Les autres mutants ont toutefois des célérités plus importantes que celle du FX-WT.

*En conclusion, tous les mutants étudiés permettent de générer de la thrombine en absence de FIX et ce quelle que soit l'induction utilisée. Les mutants FX-IIa, FX-*control+ *et FX-PAR1 sont les plus efficaces.*

### Exemple 10: Activation par la thrombine des facteurs X variants de la famille 2 et de type sauvage produits dans HEK

### 1 - Protocole expérimental

L'activation des FX variants produits par les cellules HEK en présence de Vitamine K époxyde réductase (VKOR) a été mesurée suite à l'incubation des surnageants de culture en présence de thrombine. Le facteur X activé de contrôle, la thrombine et le substrat Pefachrome FXa8595 proviennent de Haematologic Technologies (Cryopep Montpellier, France).

Les phospholipides proviennent de Diagnostica Stago (Asnières, France).

L'activation a été étudiée à 37°C dans le tampon suivant : 25 mM HEPES, pH 7.4, 0.175 M NaCl, 5 mM CaCl₂, 5 mg/ml BSA. Pour des concentrations de 42,5 et 85 nM de FX, une concentration de 10 nM de thrombine et 4 µM de phospholipides ont été utilisées. Après 1 heure d'incubation à 37°C, la quantité de FXa générée est suivie en mesurant la vitesse d'hydrolyse du substrat Pefachrome FXa8595 (250 µM) à 405 nm.

### 2 - Résultats

Afin de vérifier que les sites destinés à être clivés par la thrombine sont bien reconnus par cette enzyme, les surnageants concentrés du FX-WT, FX- control+ et FX-IIa ont été incubés en présence de thrombine. L'apparition de FXa a été mesurée par l'apparition du produit de dégradation du substrat (figure 13). En contrôle positif, le FXa plasmatique a été utilisé. La cinétique d'apparition du produit avec les mutants ne peut pas être directement comparée avec celle du FXa car ces molécules ont besoin d'être activées avant de libérer de l'activité. Par ailleurs, les sites de clivage pour la thrombine étant composites, l'activation de molécules n'est pas forcément optimale. En effet, toutes les molécules ont conservé la zone originale en aval du site de clivage, seules les parties en amont sont modifiées. La cinétique de dégradation du substrat du FXa des molécules recombinantes est par conséquent différente de celle du FXa.

Les résultats obtenus confirment les données obtenues en TGT avec, comme attendu, les FX plasmatique et WT négatifs car sans site de reconnaissance à la thrombine. Les FX-IIa et FX-control+ sont en revanche capables d'être activés par la thrombine et de libérer du FXa. Comme en mesure de TGT, le FX-IIa est plus efficacement activé que le FX- control+.

### Exemple 11 : Mesure en temps de génération de thrombine (TGT) de la capacité procoagulante des facteurs X variants de la famille 1: voie extrinsèque de la coagulation (FT 1pM/PL 4µM) en plasma déficient en FVIII

### 1 - Protocole expérimental

Les tests de génération de thrombine sont réalisés sur 80 µl d'un pool de plasma contenant éventuellement les surnageants de cellules ou les contrôles, en présence de 20 µl de réactif PPP (Stago) contenant au final 1 pM de Facteur Tissulaire (FT) et 4 µM de phospholipides (PL). Différents plasmas sont utilisés, normal et déficient en facteur VIII.

La réaction est démarrée par l'ajout de 20 µL de Fluca-kit (substrat + CaCl₂) qui constitue le début de la mesure de l'apparition de thrombine. L'apparition de fluorescence est mesurée sur un fluorimètre de type Fluoroskan Ascent (ThermoLabsystems) à une longueur d'onde d'excitation de 390 nm et à une longueur d'émission de 460 nm. Les thrombinogrammes (courbes représentant l'intensité de fluorescence en fonction du temps) sont ensuite analysés grâce au logiciel Thrombinoscope™ qui transforme la valeur de fluorescence en nM de thrombine par calcul comparatif.

### 2 - Résultats

Les surnageants de la famille 1 issus d'une transfection de cellules HEK293F en présence de VKOR ont été analysés en TGT après activation par le facteur tissulaire en utilisant les témoins déjà décrits. Les témoins (plasma normal, plasma déficient en FVIII reconstitué ou non par du FVIII recombinant) se comportent de manière attendue. En revanche le FX-WT donne un signal modéré mais supérieur à l'attendu (figures 14 et 16 ; tableau 11). Sa vélocité moyenne (59 nM/min) est toutefois dépassée par celles de plusieurs mutants notamment les FX-FXIa1/2, les FX-Kal1/2 et les FX-control+ et FX-IIa qui ont une vélocité d'au moins 130% celle du contrôle.

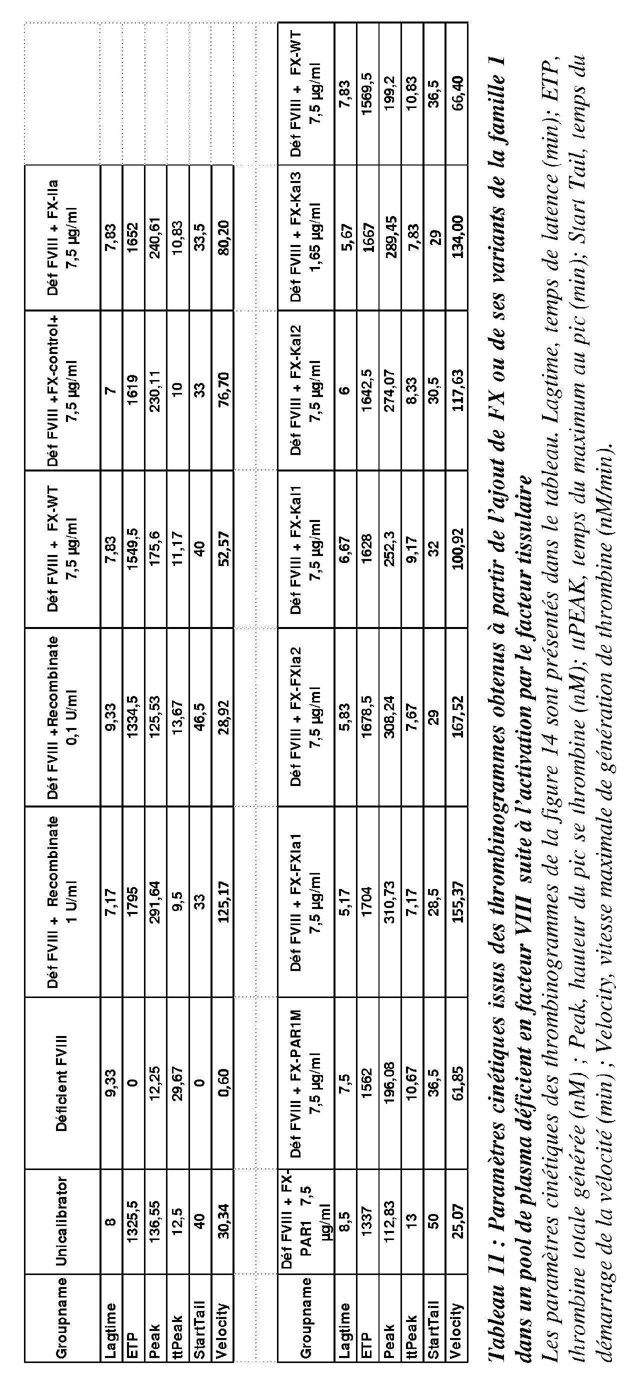

### Exemple 12 : Mesure en temps de génération de thrombine (TGT) de la capacité procoagulante des facteurs X variants de la famille 1: voie intrinsèque de la coagulation (céphaline seule) en plasma déficient en FVIII

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma contenant éventuellement les surnageants de cellules et les contrôles en présence de 20 µl de céphaline (CK-Prest reconstitué avec 1 mL d'H₂O distillée) et de 20 µL fluca-kit (substrat + CaCl₂). Les plasmas utilisés sont un plasma normal et un plasma déficient en facteur VIII.

### 2 - Résultats

Les surnageants de la famille 1 issus d'une transfection de cellules HEK293F en présence de VKOR ont été analysés en TGT après activation par la céphaline en utilisant les témoins déjà décrits.

Les témoins se comportent de manière attendue, le plasma déficient en FVIII est négatif (il ne permet pas de génération de IIa) et un gradient d'efficacité est retrouvé en augmentant la dose de FVIII (figure 15 ; tableau 12). Dans cet essai, la supplémentation en FX (2 essais ont été pratiqués) ne permet pas de générer des quantités significatives de thrombine (vélocités de 6,06 et 7,65 nM/min). En revanche les mutants FX-IIa, FX-FXIa1 et 2 et FX-control+ de la famille 1 permettent de générer des quantités significativement supérieures de thrombine d'au moins 3,5X celle du contrôle (figures 15 et 16 ; tableau 12).

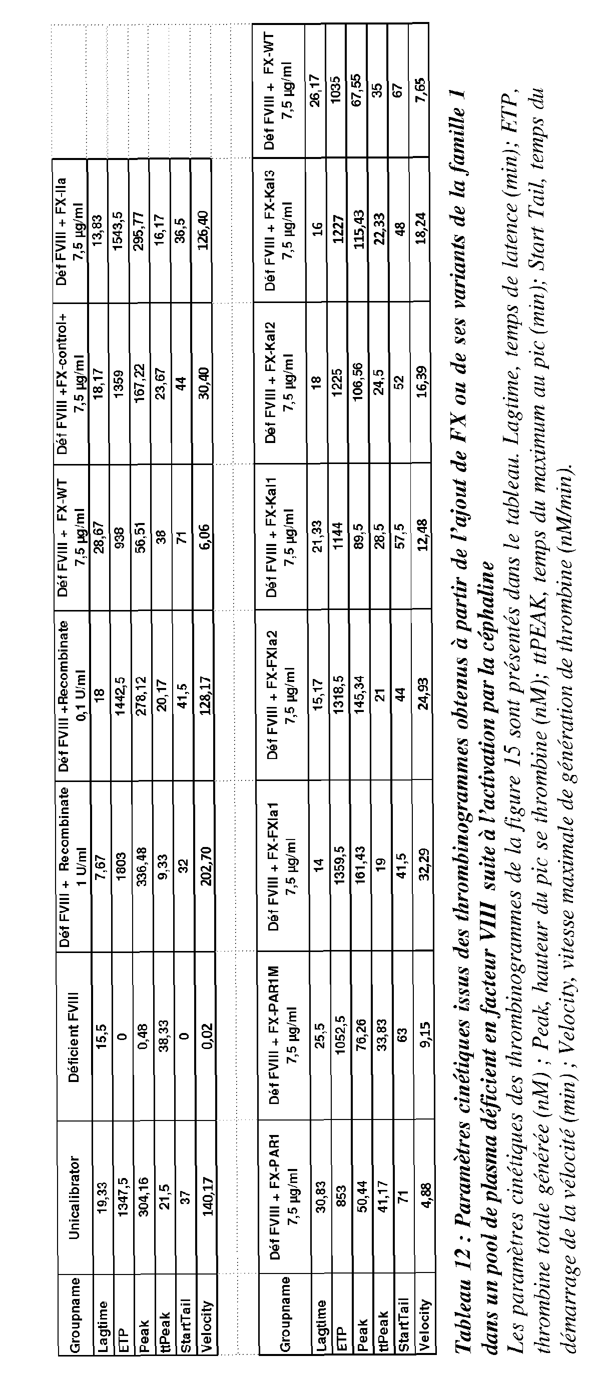

### Exemple 13 : Mesure en temps de génération de thrombine (TGT) de la capacité procoagulante des facteurs X variants de la famille 1: voie extrinsèque de la coagulation (FT 1pM/PL 4µM) en plasma déficient en FIX

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma contenant éventuellement les surnageants de cellules et les contrôles en présence de 20 µl de réactif PPP (Stago) contenant au final 1 pM de Facteur Tissulaire (FT) et 4 µM de phospholipides (PL) et de 20 µL fluca-kit (substrat + CaCl₂). Les plasmas utilisés sont un plasma normal et un plasma déficient en facteur IX.

### 2 - Résultats

Les surnageants de la famille 1 issus d'une transfection de cellules HEK293F en présence de VKOR ont été analysés en TGT en plasma déficient en FIX après activation par le facteur tissulaire en utilisant les témoins déjà décrits. Les témoins (plasma normal, plasma déficient en FIX reconstitué ou non par du FIX plasmatique (à 10 ou 100%) se comportent de manière attendue. En revanche le FX-WT donne un signal modéré mais supérieur à l'attendu (figure 17 ; tableau 13). Sa vélocité moyenne (23 nM/min) est toutefois dépassée par celles de tous les mutants sauf le FX-PAR1. Notamment les FX-FXIa1/2, les FX-Kal2/3 et les FX-control+ et FX-IIa qui ont une vélocité d'au moins 150% celle du contrôle.

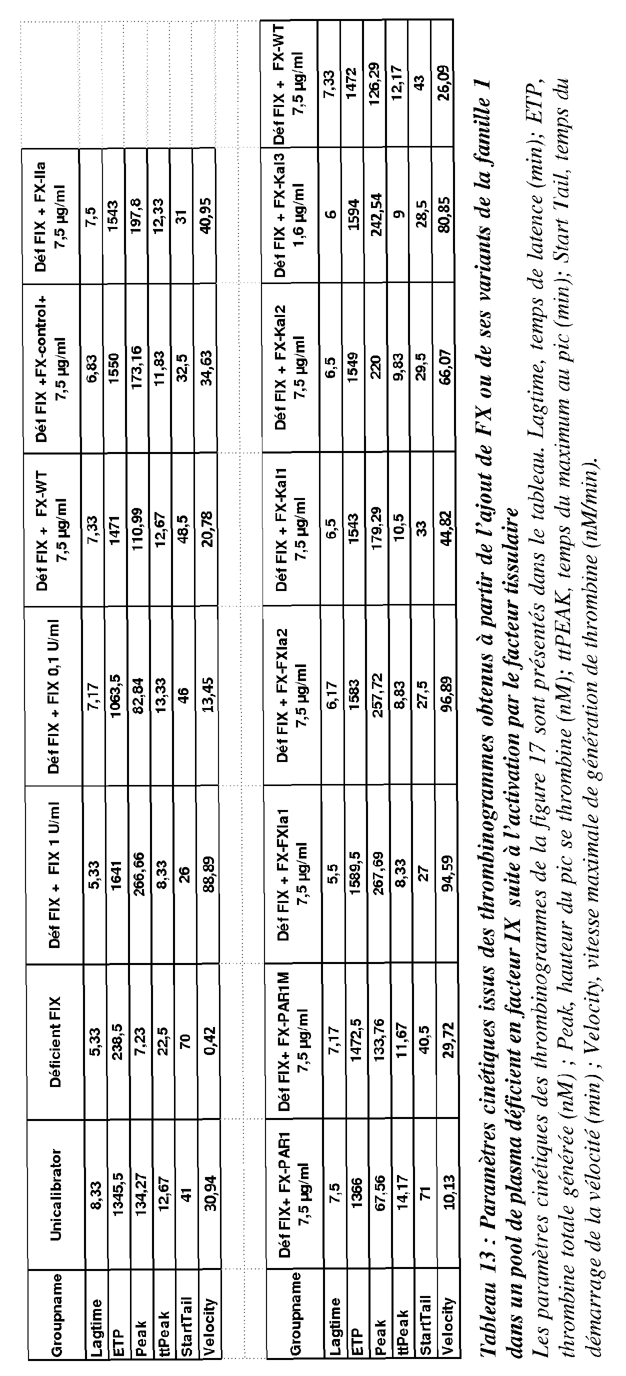

### Exemple 14 : Mesure en temps de génération de thrombine (TGT) de la capacité procoagulante des facteurs X variants de la famille 1: voie intrinsèque de la coagulation (céphaline) en plasma déficient en FIX

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma contenant éventuellement les surnageants de cellules et les contrôles en présence de 20 µl de céphaline (CK-Prest reconstitué avec 1 mL d'H₂O distillée) et de 20 µL fluca-kit (substrat + CaCl₂). Les plasmas utilisés sont un plasma normal et un plasma déficient en facteur IX.

### 2 - Résultats

Les surnageants de la famille 1 issus d'une transfection de cellules HEK293F en présence de VKOR ont été analysés en TGT après activation par la céphaline en utilisant les témoins déjà décrits.

Les témoins se comportent de manière attendue, le plasma déficient en FIX est négatif (il ne permet pas de génération de IIa) et un gradient d'efficacité est retrouvé en augmentant la dose de FIX (figure 18 ; tableau 14). Dans cet essai, la supplémentation en FX (2 essais ont été pratiqués) ne permet pas de générer des quantités significatives de thrombine (vélocités de 1,24 et 1,53 nM/min). En revanche les mutants FX-IIa, FX-FXIa1 et 2 et FX-control+ de la famille 1 permettent de générer des quantités significativement supérieures de thrombine d'au moins 4,7 X celle du contrôle à 55X (figures 18 et 19; tableau 14).

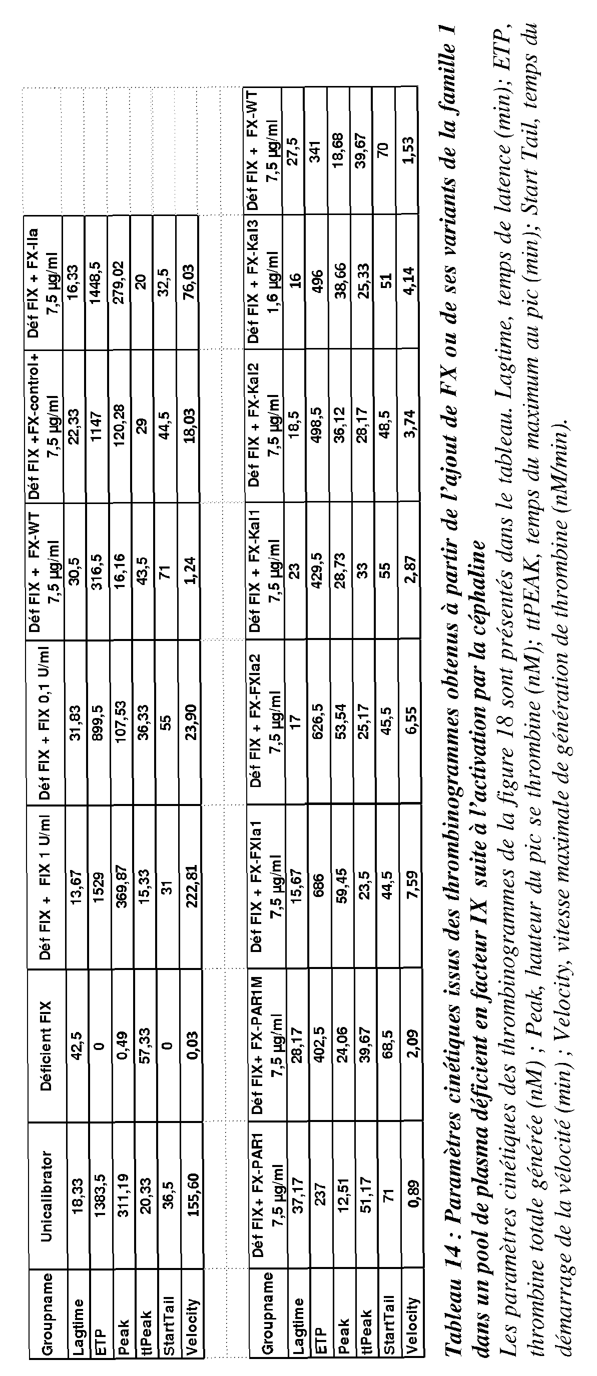

### Exemple 15 : Mesure en temps de génération de thrombine (TGT) de la capacité procoagulante des facteurs X variants de la famille 3: voie extrinsèque de la coagulation (FT 1pM/PL 4µM) en plasma déficient en FVIII

### 1 - Protocole expérimental

Les tests de génération de thrombine sont réalisés sur 80 µl d'un pool de plasma contenant éventuellement les surnageants de cellules ou les contrôles, en présence de 20 µl de réactif PPP (Stago) contenant au final 1 pM de Facteur Tissulaire (FT) et 4 µM de phospholipides (PL). Différents plasmas sont utilisés, normal et déficient en facteur VIII.

La réaction est démarrée par l'ajout de 20 µL de Fluca-kit (substrat + CaCl₂) qui constitue le début de la mesure de l'apparition de thrombine. L'apparition de fluorescence est mesurée sur un fluorimètre de type Fluoroskan Ascent (ThermoLabsystems) à une longueur d'onde d'excitation de 390 nm et à une longueur d'émission de 460 nm. Les thrombinogrammes (courbes représentant l'intensité de fluorescence en fonction du temps) sont ensuite analysés grâce au logiciel Thrombinoscope™ qui transforme la valeur de fluorescence en nM de thrombine par calcul comparatif.

### 2 - Résultats

Les surnageants de la famille 3 issus d'une transfection de cellules HEK293F en présence de VKOR ont été analysés en TGT après activation par le facteur tissulaire en utilisant les témoins déjà décrits. Les témoins (plasma normal, plasma déficient en FVIII reconstitué ou non par du FVIII recombinant) se comportent de manière attendue. En revanche le FX-WT donne un signal modéré mais supérieur à l'attendu (figures 20 et 21 ; tableau 15). Sa vélocité moyenne (59 nM/min) est toutefois dépassée par celles de plusieurs mutants notamment les FX-FXIa1/2, les FX-Kal1/2/3 et les FX-control+ et FX-IIa dont les vélocités sont au-dessus de 78 nM/min (soit au moins 130% du contrôle).

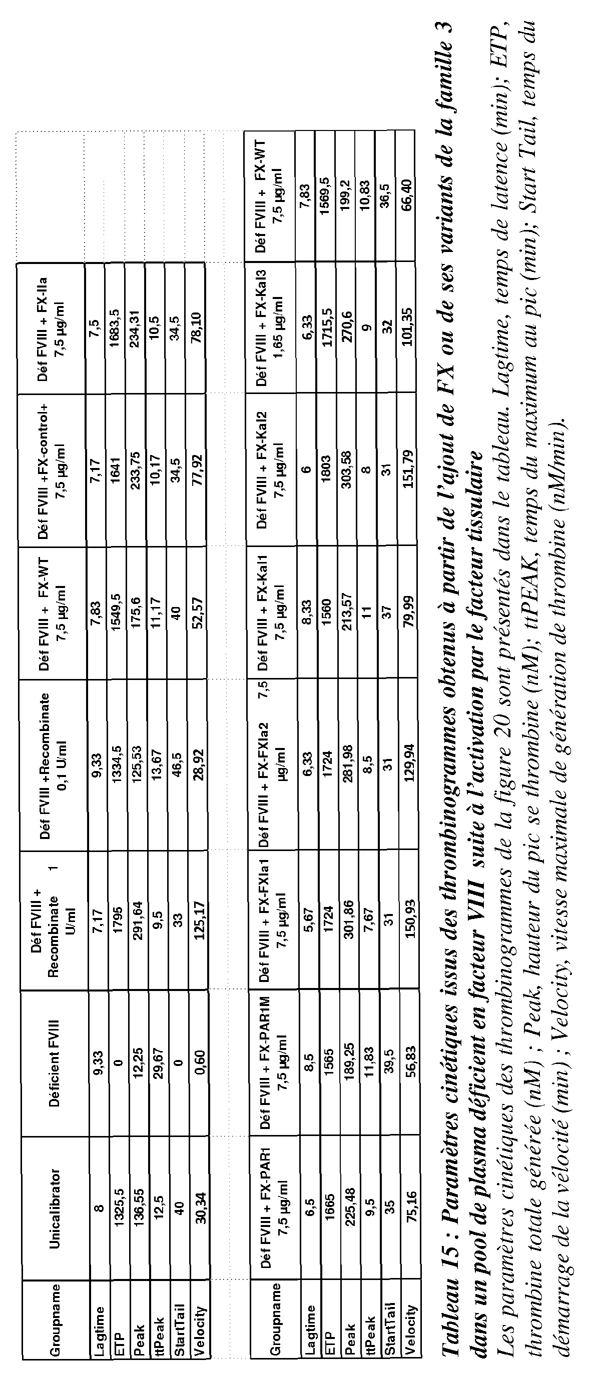

### Exemple 14 : Mesure en temps de génération de thrombine (TGT) de la capacité procoagulante des facteurs X variants de la famille 3: voie intrinsèque de la coagulation (céphaline seule) en plasma déficient en FVIII

### 1 - Protocole expérimental

Les réactifs, l'automate et le protocole expérimental sont identiques à ceux décrits dans l'exemple 6. Les tests de génération de thrombine sont réalisés sur 80 µl d'un pool de plasma contenant éventuellement les surnageants de cellules et les contrôles en présence de 20 µl de céphaline (CK-Prest reconstitué avec 1 mL d'H₂O distillée) et de 20 µL fluca-kit (substrat + CaCl₂). Les plasmas utilisés sont un plasma normal et un plasma déficient en facteur VIII.

### 2 - Résultats

Les surnageants de la famille 3 issus d'une transfection de cellules HEK293F en présence de VKOR ont été analysés en TGT après activation par la céphaline en utilisant les témoins déjà décrits.

Les témoins se comportent de manière attendue, le plasma déficient en FVIII est négatif (il ne permet pas de génération de IIa) et un gradient d'efficacité est retrouvé en augmentant la dose de FVIII (figure 21 ; tableau 16). La supplémentation en FX (2 essais ont été pratiqués) ne permet pas de générer des quantités significatives de thrombine (vélocités de 6,06 et 7,65 nM/min). En revanche les mutants FX-IIa, FX-FXIa1 et 2 et FX-control+ de la famille 3 permettent de générer des quantités significatives de thrombine avec des vélocités supérieures à 26 nM soit au moins 3,8X plus rapide que le FX-WT (figure 23 ; tableau 16).

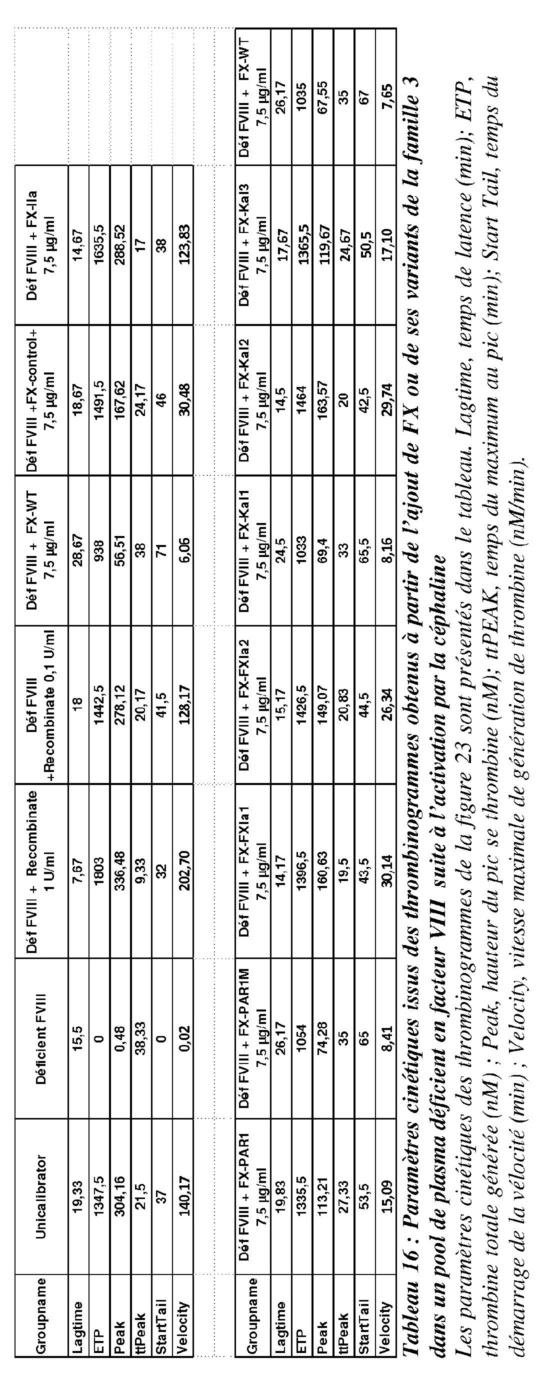

### Exemple 15 : Mesure en temps de génération de thrombine (TGT) de la capacité procoagulante des facteurs X variants de la famille 3: voie extrinsèque de la coagulation (FT 1pM/PL 4µM) en plasma déficient en FIX

### 1 - Protocole expérimental

Les réactifs, l'automate et le protocole expérimental sont identiques à ceux décrits dans l'exemple 6.

Les tests de génération de thrombine sont réalisés sur 80 µl d'un pool de plasma contenant éventuellement les surnageants de cellules et les contrôles en présence de 20 µl de réactif PPP (Stago) contenant au final 1 pM de Facteur Tissulaire (FT) et 4 µM de phospholipides (PL) et de 20 µL fluca-kit (substrat + CaCl₂). Les plasmas utilisés sont un plasma normal et un plasma déficient en facteur IX.

### 2 - Résultats

Les surnageants de la famille 3 issus d'une transfection de cellules HEK293F en présence de VKOR ont été analysés en TGT après activation par le facteur tissulaire en utilisant les témoins déjà décrits.

Les témoins se comportent de manière attendue, le plasma déficient en FIX est négatif (il ne permet pas de génération de IIa) et un gradient d'efficacité est retrouvé en augmentant la dose de FIX (figure 23 ; tableau 17). La supplémentation en FX (2 essais ont été pratiqués) ne permet pas de générer des quantités significatives de thrombine (vélocité moyenne de 23 nM/min). En revanche, tous les mutants sauf FX-PAR1M de la famille 3 permettent de générer des quantités significatives de thrombine avec des vélocités supérieures à 33 nM soit au moins 140% plus rapide que le FX-WT (figure 23 ; tableau 17).

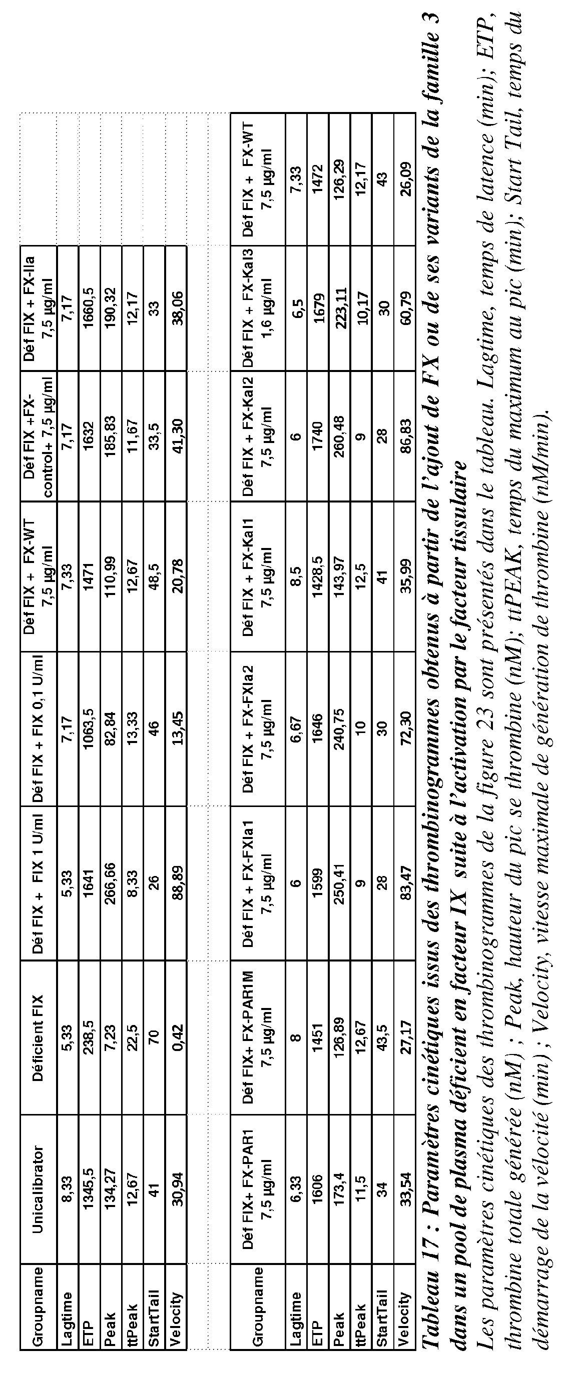

### Exemple 16: Mesure en temps de génération de thrombine (TGT) de la capacité procoagulante des facteurs X variants de la famille 3: voie intrinsèque de la coagulation (céphaline) en plasma déficient en FIX

### 1 - Protocole expérimental

Les réactifs, l'automate et le protocole expérimental sont identiques à ceux décrits dans l'exemple 6. Les tests de génération de thrombine sont réalisés sur 80 µl d'un pool de plasma normal contenant éventuellement les surnageants de cellules et les contrôles en présence de 20 µl de céphaline (CK-Prest reconstitué avec 1 mL d'H₂O distillée) et de 20 µL fluca-kit (substrat + CaCl₂). Les plasmas utilisés sont un plasma normal et un plasma déficient en facteur IX.

### 2 - Résultats

Les surnageants de la famille 3 issus d'une transfection de cellules HEK293F en présence de VKOR ont été analysés en TGT après activation par la céphaline en utilisant les témoins déjà décrits.

Les témoins se comportent de manière attendue, le plasma déficient en FIX est négatif (il ne permet pas de génération de IIa) et un gradient d'efficacité est retrouvé en augmentant la dose de FXI (figure 24 ; tableau 18). Dans cet essai, la supplémentation en FX (2 essais ont été pratiqués) ne permet pas de générer des quantités significatives de thrombine (vélocités de 1,24 et 1,53 nM/min). En revanche, les mutants FX-IIa, FX-FXIa1 et 2, FX-Kal 2 et 3, et FX-control+ de la famille 1 permettent de générer des quantités significativement supérieures de thrombine d'au moins 3,4X celle du contrôle à 52X (figures 24 et 25 ; tableau 18). Par ailleurs tous les mutants sauf les mutants FX-PAR1M et FX-Kal2 sont plus actifs que le FX-WT.

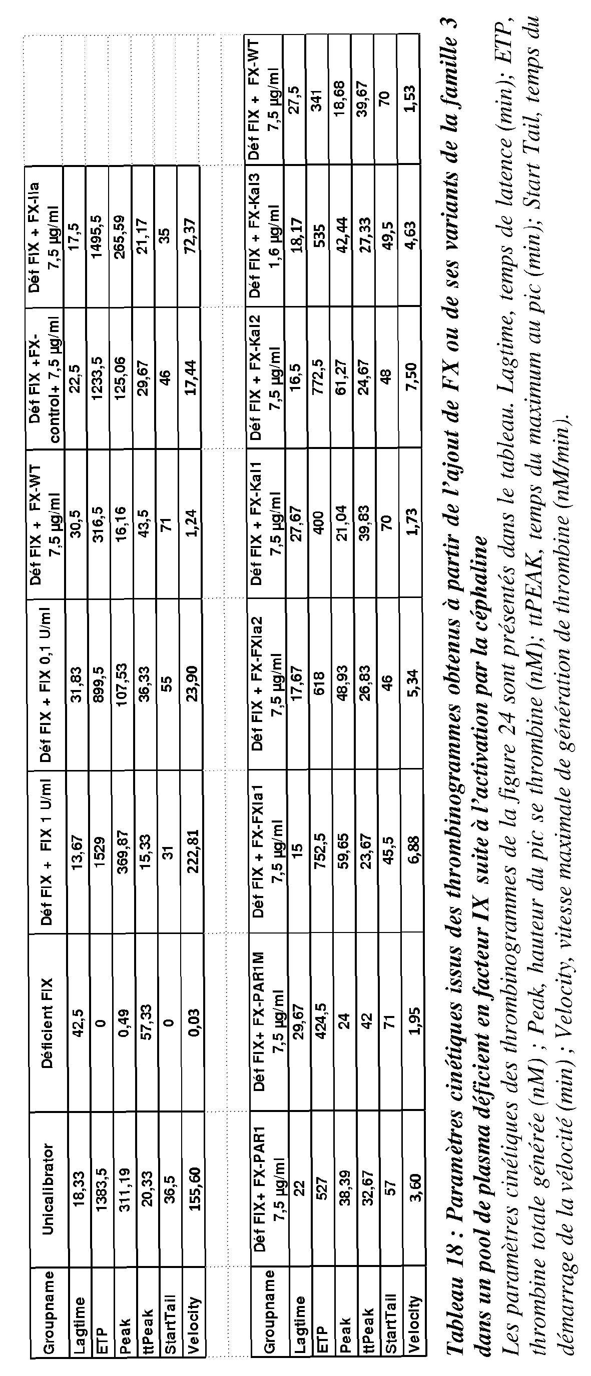

## Revendications

1. Protéine comprenant une séquence mutée de SEQ ID NO :1, ladite séquence mutée de SEQ ID NO :1 comprenant au moins une mutation A, A', B, C ou C', dans laquelle :
la mutation A consiste en la substitution des acides aminés 43 à 52 de la séquence SEQ ID NO :1 par une séquence choisie parmi DFLAEGLTPR, KATN*ATLSPR et KATXATLSPR,
la mutation A' consiste en la substitution des acides aminés 47 à 52 de la séquence SEQ ID NO :1 par une séquence choisie parmi TSKLTR, FNDFTR, LSSMTR, PPSLTR et LSCGQR,
la mutation B consiste en l'insertion d'une séquence choisie parmi DFLAEGLTPR, KATN*ATLSPR, KATXATLSPR, TSKLTR, FNDFTR, LSSMTR, PPSLTR et LSCGQR, entre les acides aminés 52 et 53 de la séquence SEQ ID NO :1,
la mutation C consiste en l'insertion d'une séquence choisie parmi DFLAEGLTPR, KATN*ATLSPR et KATXATLSPR, entre les acides aminés 52 et 53 de la séquence SEQ ID NO :1, et en la délétion des acides aminés 4 à 13 de la séquence SEQ ID NO :1, la mutation C' consiste en l'insertion d'une séquence choisie parmi TSKLTR, FNDFTR, LSSMTR, PPSLTR et LSCGQR, entre les acides aminés 52 et 53 de la séquence SEQ ID NO :1, et en la délétion des acides aminés 4 à 9 de la séquence SEQ ID NO :1,
où N* est une asparagine éventuellement glycosylée.

2. Protéine selon la revendication 1, **caractérisée en ce qu'**elle comprend, de préférence consiste en, la séquence SEQ ID NO :7, avec au moins une mutation A, A', B, C ou C'.

3. Protéine selon la revendication 1 ou 2, **caractérisée en ce qu'**elle comprend une séquence choisie parmi SEQ ID NO :9 à 16, 18 à 25, 27 à 34, 105 à 112, 114 à 121 et 123 à 130.

4. Complexe protéique comprenant une protéine selon l'une des revendications 1 à 3, et au moins une protéine de séquence SEQ ID NO :2, lesdites protéines étant liées entre elles par un pont disulfure.

5. Acide nucléique **caractérisé en ce qu'**il code pour la protéine selon l'une des revendications 1 à 4.

6. Acide nucléique selon la revendication 5, **caractérisé en ce qu'**il est choisi parmi les séquences SEQ ID NO :77 à 84, 86 à 93, 95 à 102, 133 à 140, 142 à 149 et 151 à 158.

7. Cassette d'expression comprenant l'acide nucléique selon la revendication 5 ou 6.

8. Vecteur d'expression, **caractérisé en ce qu'**il comprend la cassette d'expression selon la revendication 7.

9. Cellule recombinante comprenant l'acide nucléique selon la revendication 5 ou 6, préférentiellement ledit acide nucléique est compris dans le vecteur selon la revendication 8.

10. Protéine selon l'une des revendications 1 à 4 pour son utilisation en tant que médicament.

11. Protéine selon l'une des revendications 1 à 4 pour son utilisation pour traiter les troubles hémorragiques.

## Patentansprüche

1. Protein, umfassend eine mutierte Sequenz von SEQ ID NO 1, wobei die mutierte Sequenz von SEQ ID NO 1 wenigstens eine Mutation A, A', B, C oder C' umfasst, wobei:
die Mutation A in der Substitution der Aminosäuren 43 bis 52 der Sequenz SEQ ID NO 1 durch eine Sequenz umfasst, die gewählt ist aus DFLAEGLTPR, KATN*ATLSPR und KATXATLSPR,
wobei die Mutation A' in der Substitution der Aminosäuren 47 bis 52 der Sequenz SEQ ID NO 1 durch eine Sequenz umfasst, die gewählt ist aus TSKLTR, FNDFTR, LSSMTR, PPSLTR und LSCGQR,
wobei die Mutation B in der Einführung einer Sequenz zwischen den Aminosäuren 52 und 53 der Sequenz SEQ ID NO 1 besteht, die gewählt ist aus DFLAEGLTPR, KATN*ATLSPR, KATXATLSPR, TSKLTR, FNDFTR, LSSMTR, PPSLTR und LSCGQR,
wobei die Mutation C in der Einführung einer Sequenz zwischen den Aminosäuren 52 und 53 der Sequenz SEQ ID NO 1 besteht, die gewählt ist aus DFLAEGLTPR, KATN*ATLSPR und KATXATLSPR, und in der Deletion der Aminosäuren 4 bis 13 der Sequenz SEQ ID NO 1,
wobei die Mutation C' in der Einführung einer Sequenz zwischen den Aminosäuren 52 und 53 der Sequenz SEQ ID NO 1 besteht, die gewählt ist aus TSKLTR, FNDFTR, LSSMTR, PPSLTR und LSCGQR und in der Deletion der Aminosäuren 4 bis 9 der Sequenz SEQ ID NO 1,
wobei N* ein gegebenenfalls glycosyliertes Asparagin ist.

2. Protein nach Anspruch 1, **dadurch gekennzeichnet, dass** es die Sequenz SEQ ID NO 7 mit wenigstens einer Mutation A, A', B, C oder C' umfasst, vorzugsweise aus dieser besteht.

3. Protein nach Anspruch 1 oder 2, **dadurch gekennzeichnet, dass** es eine Sequenz umfasst, die gewählt ist aus SEQ ID NO 9 bis 16, 18 bis 25, 27 bis 34, 105 bis 112, 114 bis 121 und 123 bis 130.

4. Proteinkomplex, umfassend ein Protein nach einem der Ansprüche 1 bis 3, und wenigstens ein Protein der Sequenz SEQ ID NO 2, wobei die Proteine durch eine Disulfidbrücke miteinander verbunden sind.

5. Nukleinsäure, **dadurch gekennzeichnet dass** sie für das Protein nach einem der Ansprüche 1 bis 4 kodiert.

6. Nukleinsäure nach Anspruch 5, **dadurch gekennzeichnet, dass** sie gewählt ist aus den Sequenzen SEQ ID NO 77 bis 84, 86 bis 93, 95 bis 102, 133 bis 140, 142 bis 149 und 151 bis 158.

7. Expressionskassette, umfassend die Nukleinsäure nach Anspruch 5 oder 6.

8. Expressionsvektor, **dadurch gekennzeichnet, dass** er die Expressionskassette nach Anspruch 7 umfasst.

9. Rekombinante Zelle, umfassend die Nukleinsäure nach Anspruch 5 oder 6, wobei vorzugsweise die Nukleinsäure in dem Vektor nach Anspruch 8 enthalten ist.

10. Protein nach einem der Ansprüche 1 bis 4 zur Verwendung als Arzneimittel.

11. Protein nach einem der Ansprüche 1 bis 4 zur Verwendung in der Behandlung hämorrhagischer Störungen.

## Claims

1. A protein comprising a mutated sequence of SEQ ID No. 1, said mutated sequence of SEQ ID No. 1 comprising at least one mutation A, A', B, C or C', in which: mutation A consists of the substitution of amino acids 43 to 52 of the sequence SEQ ID No. 1 with a sequence chosen from DFLAEGLTPR, KATN*ATLSPR and KATXATLSPR, mutation A' consists of the substitution of amino acids 47 to 52 of the sequence SEQ ID No. 1 with a sequence chosen from TSKLTR, FNDFTR, LSSMTR, PPSLTR and LSCGQR,
mutation B consists of the insertion of a sequence chosen from DFLAEGLTPR, KATN*ATLSPR, KATXATLSPR, TSKLTR, FNDFTR, LSSMTR, PPSLTR and LSCGQR between amino acids 52 and 53 of the sequence SEQ ID No. 1,
mutation C consists of the insertion of a sequence chosen from DFLAEGLTPR, KATN*ATLSPR and KATXATLSPR, between amino acids 52 and 53 of the sequence SEQ ID No. 1, and of the deletion of amino acids 4 to 13 of the sequence SEQ ID No. 1,
mutation C' consists of the insertion of a sequence chosen from TSKLTR, FNDFTR, LSSMTR, PPSLTR and LSCGQR, between amino acids 52 and 53 of the sequence SEQ ID No. 1, and of the deletion of amino acids 4 to 9 of the sequence SEQ ID No. 1, where N* is an optionally glycosylated asparagine.

2. The protein as claimed in claim 1, **characterized in that** it comprises, preferably consists of, the sequence SEQ ID No. 7, with at least one mutation A, A', B, C or C'.

3. The protein as claimed in claim 1 or 2, **characterized in that** it comprises a sequence chosen from SEQ ID No. 9 to 16, 18 to 25, 27 to 34, 105 to 112, 114 to 121 and 123 to 130.

4. A protein complex comprising a protein as claimed in one of claims 1 to 3, and at least one protein of sequence SEQ ID No. 2, said proteins being linked to one another by a disulfide bridge.

5. A nucleic acid **characterized in that** it encodes the protein as claimed in one of claims 1 to 4.

6. The nucleic acid as claimed in claim 5, **characterized in that** it is chosen from the sequences SEQ ID No. 77 to 84, 86 to 93, 95 to 102, 133 to 140, 142 to 149 and 151 to 158.

7. An expression cassette comprising the nucleic acid as claimed in claim 5 or 6.

8. An expression vector **characterized in that** it comprises the expression cassette as claimed in claim 7.

9. A recombinant cell comprising the nucleic acid as claimed in claim 5 or 6, said nucleic acid preferentially being included in the vector as claimed in claim 8.

10. The protein as claimed in one of claims 1 to 4, for use as a medicament.

11. The protein as claimed in one of claims 1 to 4, for use in treating hemorrhagic disorders.
